# EUROPÄISCHE PATENTANMELDUNG

(11) **EP 2 333 550 A2**
(43) Veröffentlichungstag der Anmeldung: **15.06.2011**
(21) Anmeldenummer: 10183092.5
(22) Anmeldetag: 28.08.2006
(51) Int. Cl.: G01N 33/574, G01N 33/68, C07K 7/08

(54) **Polypeptidmarker zur Diagnose von Blasenkrebs**

(30) Priorität: 26.08.2005 EP 05107840
(62) Teilanmeldung aus: 06793031.3
(71) Anmelder: mosaiques diagnostics and therapeutics AG, 30625 Hannover (DE)
(72) Erfinder: Mischak, Harald, 31319 Sehnde (DE)
(74) Vertreter: von Kreisler Selting Werner

(57) **Zusammenfassung**

Verfahren zur Diagnose von Blasenkrebs (BC) und/oder zur Bestimmung eines Tumorstadiums von Blasenkrebs umfassend den Schritt
- der Bestimmung einer An- oder Abwesenheit oder Amplitude von mindestens sechs Polypeptidmarkern in einer Probe, wobei die Polypeptidmarker ausgewählt sind aus den Markern 1 bis 836

und die Polypeptidmarker durch Werte für die Molekularmassen und die Migrationszeit (CE-Zeit) charakterisiert sind.

## Beschreibung

Die vorliegende Erfindung betrifft die Verwendung der An- oder Abwesenheit oder Amplitude eines oder mehrerer Peptidmarker in einer Probe eines Individuums zur (Differential-) Diagnose von Blasenkrebs sowie ein Verfahren zur Diagnose von Blasenkrebs, wobei die An- oder Abwesenheit oder Amplitude des oder der Peptidmarker(s) indikativ für das Vorliegen sowie das Tumorstadium von Blasenkrebs ist.

Blasenkrebs ist ein bösartiger Tumor an der Schleimhaut der Harnblase. Blasenkrebs ist eine der häufigsten bösartigen Erkrankungen. Im urologischen Bereich ist sie nach Prostatakrebs die zweithäufigste Krebserkrankung. Im deutschen Sprachraum sind etwa 22 von 100.000 Menschen pro Jahr betroffen. Bei Männern tritt Blasenkrebs ungefähr zwei- bis dreimal häufiger auf als bei Frauen. Jährlich erkranken in der Bundesrepublik Deutschland schätzungsweise 13.000 Männer und 5.000 Frauen. Blasenkrebs ist eine Erkrankung des höheren Lebensalters. Das Erkrankungsrisiko steigt ab dem 40sten Lebensjahr mit zunehmendem Alter.

### Diagnose des Blasenkrebses:

Eine echte Früherkennung gibt es bei Blasenkrebs nicht. Bei Blut im Harn oder bei Problemen beim Wasserlassen wird dringend empfohlen, rasch zum Arzt zu gehen. Dadurch kann Blasenkrebs möglicherweise schon früher entdeckt werden. Besteht ein Verdacht auf eine Geschwulst in der Blase, z.B. wenn Blut im Urin bemerkt wurde oder wenn anhaltende Symptome einer Blasenirritation vorliegen, wird eine Blasenspiegelung, Zystoskopie, vorgenommen. Wenn der Untersucher einen Tumor in der Harnblasenwand sieht, kann er abschätzen, welche Wandschichten der Tumor durchdringt und auch Proben entnehmen, die dann mikroskopisch untersucht werden. Je nach Tumorwachstum unterscheidet man **oberflächliche** und **infiltrierende** (ins Gewebe eingedrungene) Karzinome. Letztere sind bereits in die Muskulatur der Harnblase eingewachsen und können sich in die Nachbarorgane (beim Mann z.B. Prostata, bei der Frau z.B. Gebärmutter) ausbreiten. Die histologisch-pathologische Klassifizierung der Schleimhauttumoren erfolgt nach dem TNM-System.

### Oberflächliche Karzinome:

pTa = nicht invasives papilläres Karzinom der Schleimhaut (Urothel)
pTcis = Carcinoma in situ
pT1 = Einwachsen (Infiltration) unter die Schleimhaut (subepitheliales Bindegewebe), Unterklassifikation pT1a-c

### Infiltrierende Karzinome

pT2 = Einwachsen in die Muskelschicht (Muscularis propria), Unterklassifikation pT2a-b
pT3 = Hinauswachsen über die Muskelschicht hinaus, Unterklassifikation pT3a-b
pT4 = Einwachsen in Nachbarorgane wie Prostata, Gebärmutter, Vagina, Beckenwand

Zusätzlich kann eine radiologische Untersuchung (Urographie) des gesamten Harntrakts durchgeführt werden. Ergänzend wird der Harn mikroskopisch auf bösartige Zellen untersucht. Zur Stadieneinteilung werden weitere diagnostische Verfahren eingesetzt wie Ultraschall, CT (Computer-Tomographie) oder MRT (Magnetresonanz-Tomographie). Durch eine Ultraschalluntersuchung des Bauches können Lage und Größe eines Tumors festgestellt werden. Außerdem werden mit ihrer Hilfe die Nieren auf einen Rückstau von Urin hin untersucht und die Lymphknoten sowie die Leber auf Metastasen geprüft.

Wie bereits beschrieben, gibt es keine funktionierende Früherkennung des Blasenkrebses. Eine klare Diagnose ist bisher mit invasiven Eingriffen wie der Zystoskopie und einer Gewebeentnahme verbunden. Es stellte sich also die Aufgabe, ein Verfahren und eine Methode zur möglichst wenig invasiven, schnellen und kostengünstigen Diagnose des Blasenkrebses sowie zur Bestimmung des Tumorstadiums zu finden.

Vlahou et al., American Journal of Pathology 158 (2001) 1491-1501 beschreiben die Analyse von Urinproben von Patienten mit "transitional cell carcinoma" (TCC) mittels SELDI. Aufgrund der geringen Auflösung der SELDI-Technologie können hiermit nur wenige Marker nebeneinander detektiert werden. Hieraus ergibt sich eine geringe Sensitivität und Spezifität der Diagnostik, welche in geblindeten Untersuchungen nicht validiert werden konnten

W. Liu et al., in European Urology 47 (2005) 456-462 beschreibt ebenfalls eine SELDI-Analyse von Urinproben. Die geringe Anzahl gefundener Marker ergibt nur eine schlechte Spezifität der Analyse.

Trotz der identischen Technologie welche von Vlahou et al. und Liu et al. verwendet wurde, werden in beiden Arbeiten unterschiedliche Marker gefunden, was die nicht vorhandene Validität der Vorgehensweise verdeutlicht. Auch sind die Massenangaben für die Marker so unpräzise, dass entsprechende Messungen nicht reproduzierbar, bzw. die Massen nicht eindeutig einzelnen Substanzen zuzuordnen sind.

Aufgabe der vorliegenden Erfindung war es, die genannten Nachteile des Standes der Technik zu überwinden, insbesondere Polypeptidmarker zu definieren, die eindeutig einzelnen Peptiden zuzuordnen sind, und zur Diagnose von Blasenkrebs und/oder zur Bestimmung des Tumorstadiums geeignet sind.

Überraschenderweise wurde nun gefunden, das bestimmte Peptidmarker in einer Probe eines Individuums sowohl zur Diagnose von Blasenkrebs als auch zur Abschätzung des Tumorstadiums eines Blasenkarzinoms verwendet werden können.

Folglich ist ein Gegenstand der vorliegenden Erfindung die Verwendung der An- oder Abwesenheit oder Amplitude mindestens eines Polypeptidmarkers in einer Probe eines Individuums zur Diagnose von Blasenkrebs, wobei der Polypeptidmarker ausgewählt ist aus den Polypeptidmarkern Nr. 1 bis Nr. 836, die durch die die in Tabelle 1 angegebenen Molekularmassen und ihre Migrationszeiten charakterisiert sind.

**Tabelle 1: Polypeptidmarker zur Diagnose von Blasenkrebs sowie ihre Molekularmassen und Migrationszeiten (CE-Zeit):**

| **Nummer** | **Masse** | **CE-Zeit** | **Nummer** | **Masse** | **CE-Zeit** | **Nummer** | **Masse** | **CE-Zeit** | **Nummer** | **Masse** | **CE-Zeit** | **Nummer** | **Masse** | **CE-Zeit** |
|---|---|---|---|---|---|---|---|---|---|---|---|---|---|---|
| 1 | 4933,97 | 17,2 | 60 | 1844,58 | 31,4 | 119 | 1935,96 | 13,6 | 178 | 1950,87 | 33,9 | 237 | 1073,36 | 35,39 |
| 2 | 4712,25 | 14,3 | 61 | 2130,97 | 29,6 | 120 | 2062,81 | 22,9 | 179 | 1649,76 | 17,9 | 238 | 1078,69 | 19,67 |
| 3 | 1071,54 | 16,3 | 62 | 2359,35 | 31,0 | 121 | 13372,32 | 25,9 | 180 | 1312,55 | 26,3 | 239 | 1082,55 | 24,37 |
| 4 | 3273,42 | 17,7 | 63 | 3343,58 | 28,5 | 122 | 1495,68 | 18,9 | 181 | 1438,47 | 35,2 | 240 | 1083,56 | 21,65 |
| 5 | 1130,37 | 33,3 | 64 | 3501,62 | 28,4 | 123 | 1513,49 | 35,4 | 182 | 3248,51 | 26,8 | 241 | 1096,41 | 35,91 |
| 6 | 1180,53 | 33,7 | 65 | 5675,31 | 19,0 | 124 | 1900,90 | 28,6 | 183 | 2742,26 | 25,3 | 242 | 1096,53 | 26,12 |
| 7 | 980,36 | 34,0 | 66 | 1312,64 | 17,6 | 125 | 3108,94 | 33,8 | 184 | 981,60 | 20,7 | 243 | 1098,56 | 21,42 |
| 8 | 11721,36 | 13,4 | 67 | 1867,68 | 30,4 | 126 | 1860,87 | 16,2 | 185 | 1679,97 | 19,3 | 244 | 1099,56 | 21,56 |
| 9 | 2077,12 | 16,8 | 68 | 2186,85 | 31,9 | 127 | 2194,68 | 14,3 | 186 | 801,43 | 22,7 | 245 | 1100,59 | 21,62 |
| 10 | 840,42 | 19,3 | 69 | 2414,51 | 33,6 | 128 | 3956,81 | 21,3 | 187 | 816,41 | 21,05 | 246 | 1101,54 | 22,21 |
| 11 | 2115,04 | 22,8 | 70 | 2739,24 | 24,8 | 129 | 1496,70 | 26,5 | 188 | 818,48 | 21,95 | 247 | 1106,52 | 26,02 |
| 12 | 3385,43 | 21,1 | 71 | 2907,36 | 33,9 | 130 | 3659,17 | 21,3 | 189 | 838,36 | 25,12 | 248 | 1109,68 | 21,06 |
| 13 | 4053,18 | 13,6 | 72 | 3031,42 | 34,0 | 131 | 13169,24 | 25,6 | 190 | 838,44 | 35,05 | 249 | 1114,54 | 25,52 |
| 14 | 4240,13 | 14,2 | 73 | 4345,89 | 30,6 | 132 | 1608,75 | 27,5 | 191 | 840,47 | 19,68 | 250 | 1114,57 | 21,27 |
| 15 | 1387,04 | 9,5 | 74 | 6211,67 | 14,6 | 133 | 1707,73 | 11,1 | 192 | 852,47 | 20,42 | 251 | 1124,58 | 21,04 |
| 16 | 2597,47 | 18,6 | 75 | 1080,52 | 22,9 | 134 | 1592,72 | 17,3 | 193 | 868,45 | 23,35 | 252 | 1125,55 | 24,9 |
| 17 | 2920,21 | 15,9 | 76 | 1142,80 | 10,9 | 135 | 2029,80 | 15,0 | 194 | 871,48 | 21,11 | 253 | 1125,58 | 21,77 |
| 18 | 4509,09 | 25,1 | 77 | 1321,74 | 10,6 | 136 | 13007,18 | 25,1 | 195 | 875,51 | 21,83 | 254 | 1126,52 | 21,2 |
| 19 | 8763,45 | 12,1 | 78 | 1750,77 | 19,5 | 137 | 2117,77 | 29,9 | 196 | 876,43 | 35,13 | 255 | 1126,57 | 25,61 |
| 20 | 1535,72 | 26,3 | 79 | 1989,90 | 29,3 | 138 | 2168,87 | 31,0 | 197 | 883,45 | 23,27 | 256 | 1134,63 | 23,68 |
| 21 | 911,28 | 31,9 | 80 | 2946,38 | 32,8 | 139 | 1916,86 | 31,3 | 198 | 884,36 | 24,91 | 257 | 1135,53 | 27,51 |
| 22 | 1191,54 | 34,5 | 81 | 4942,34 | 21,4 | 140 | 2802,78 | 34,7 | 199 | 892,32 | 35,22 | 258 | 1136,59 | 20,27 |
| 23 | 3759,90 | 12,8 | 82 | 1668,76 | 17,5 | 141 | 1111,77 | 10,8 | 200 | 906,34 | 34,93 | 259 | 1137,65 | 24,08 |
| 24 | 2973,43 | 20,0 | 83 | 1688,71 | 14,5 | 142 | 1806,84 | 18,5 | 201 | 906,51 | 21,89 | 260 | 1138,64 | 19,55 |
| 25 | 3325,67 | 15,4 | 84 | 1731,79 | 17,7 | 143 | 1594,71 | 26,1 | 202 | 912,55 | 20,04 | 261 | 1139,54 | 21,01 |
| 26 | 10042,81 | 12,2 | 85 | 1822,74 | 27,4 | 144 | 1778,76 | 27,0 | 203 | 914,49 | 23,75 | 262 | 1140,53 | 21,14 |
| 27 | 1380,67 | 19,3 | 86 | 3870,83 | 30,4 | 145 | 2582,95 | 19,0 | 204 | 915,28 | 35,24 | 263 | 1141,57 | 25,2 |
| 28 | 3502,70 | 12,5 | 87 | 4275,48 | 20,6 | 146 | 3605,63 | 21,22 | 205 | 931,51 | 19,99 | 264 | 1142,61 | 21,8 |
| 29 | 3632,63 | 15,9 | 88 | 1025,48 | 21,7 | 147 | 3765,31 | 42,77 | 206 | 935,49 | 23,76 | 265 | 1143,56 | 36,95 |
| 30 | 8559,22 | 13,2 | 89 | 1353,67 | 22,1 | 148 | 4067,63 | 31,22 | 207 | 936,49 | 21,43 | 266 | 1144,43 | 35,57 |
| 31 | 4960,86 | 15,6 | 90 | 1466,67 | 25,2 | 149 | 10640,4 | 19,68 | 208 | 939,51 | 23,79 | 267 | 1144,6 | 26,25 |
| 32 | 858,41 | 18,9 | 91 | 1588,73 | 26,7 | 150 | 2577,35 | 20,9 | 209 | 941,46 | 24,85 | 268 | 1150,61 | 22,42 |
| 33 | 847,40 | 20,0 | 92 | 1878,72 | 27,6 | 151 | 3292,55 | 37,5 | 210 | 944,55 | 21,35 | 269 | 1153,38 | 35,68 |
| 34 | 980,51 | 18,7 | 93 | 2933,31 | 24,1 | 152 | 2058,90 | 18,8 | 211 | 945,46 | 25,7 | 270 | 1153,57 | 26,34 |
| 35 | 1128,52 | 22,4 | 94 | 3338,45 | 19,3 | 153 | 3001,36 | 33,2 | 212 | 947,5 | 24,75 | 271 | 1154,57 | 36,72 |
| 36 | 1407,68 | 35,6 | 95 | 6055,63 | 15,6 | 154 | 2157,04 | 17,0 | 213 | 951,55 | 20,32 | 272 | 1155,55 | 20,77 |
| 37 | 2093,92 | 31,0 | 96 | 2266,00 | 17,2 | 155 | 2854,36 | 32,6 | 214 | 956,48 | 20,38 | 273 | 1157,58 | 37,41 |
| 38 | 2421,04 | 32,5 | 97 | 2939,05 | 31,1 | 156 | 1653,90 | 26,9 | 215 | 958,51 | 20,76 | 274 | 1159,63 | 20,75 |
| 39 | 1250,66 | 14,8 | 98 | 3021,39 | 18,9 | 157 | 2658,32 | 13,1 | 216 | 963,54 | 21,65 | 275 | 1159,64 | 26,09 |
| 40 | 3242,20 | 18,0 | 99 | 1299,60 | 17,6 | 158 | 3657,65 | 38,7 | 217 | 973,28 | 35,58 | 276 | 1160,6 | 26,14 |
| 41 | 1040,50 | 21,2 | 100 | 1405,68 | 14,3 | 159 | 1754,92 | 27,8 | 218 | 984,49 | 24,43 | 277 | 1162,59 | 20,26 |
| 42 | 3176,40 | 11,9 | 101 | 1526,72 | 19,3 | 160 | 1467,82 | 19,8 | 219 | 988,55 | 35,81 | 278 | 1165,55 | 37,15 |
| 43 | 2778,55 | 32,5 | 102 | 1630,78 | 15,4 | 161 | 1580,91 | 20,1 | 220 | 988,55 | 21,83 | 279 | 1166,62 | 22,8 |
| 44 | 1422,58 | 35,8 | 103 | 1865,83 | 30,0 | 162 | 2563,89 | 17,6 | 221 | 994,49 | 25,17 | 280 | 1168,58 | 26,23 |
| 45 | 3524,42 | 28,9 | 104 | 2687,24 | 25,4 | 163 | 2355,15 | 17,9 | 222 | 995,44 | 25,15 | 281 | 1169,61 | 23,85 |
| 46 | 3831,74 | 24,8 | 105 | 2756,27 | 32,8 | 164 | 3891,40 | 20,2 | 223 | 996,51 | 24,63 | 282 | 1170,64 | 21,61 |
| 47 | 2488,79 | 32,2 | 106 | 1200,84 | 10,7 | 165 | 1561,49 | 35,6 | 224 | 1009,47 | 20,96 | 283 | 1171,62 | 23,34 |
| 48 | 1099,51 | 24,9 | 107 | 1391,81 | 10,7 | 166 | 1576,63 | 23,1 | 225 | 1012,53 | 35,08 | 284 | 1173,58 | 37,51 |
| 49 | 1936,82 | 29,2 | 108 | 1487,68 | 26,1 | 167 | 1911,07 | 20,8 | 226 | 1016,31 | 35,67 | 285 | 1179,65 | 20,06 |
| 50 | 1265,61 | 23,4 | 109 | 1919,87 | 23,3 | 168 | 3265,47 | 19,8 | 227 | 1016,49 | 25,81 | 286 | 1181,62 | 26,06 |
| 51 | 3081,61 | 26,4 | 110 | 2063,48 | 14,1 | 169 | 2344,91 | 32,3 | 228 | 1027,55 | 24,6 | 287 | 1190,64 | 22,02 |
| 52 | 3266,48 | 26,5 | 111 | 2753,47 | 34,2 | 170 | 2743,98 | 32,8 | 229 | 1031,5 | 25,66 | 288 | 1196,37 | 36,14 |
| 53 | 3821,95 | 20,4 | 112 | 2864,16 | 28,5 | 171 | 3401,75 | 18,1 | 230 | 1037,57 | 21,98 | 289 | 1196,59 | 21,07 |
| 54 | 2407,10 | 24,0 | 113 | 3858,65 | 21,9 | 172 | 3425,87 | 26,1 | 231 | 1038,61 | 20,84 | 290 | 1198,6 | 26,05 |
| 55 | 1596,75 | 19,0 | 114 | 4015,96 | 24,4 | 173 | 5510,18 | 23,1 | 232 | 1042,54 | 25,13 | 291 | 1199,63 | 21,92 |
| 56 | 10341,75 | 18,1 | 115 | 1324,60 | 16,3 | 174 | 2356,70 | 33,5 | 233 | 1053,53 | 25,08 | 292 | 1200,59 | 24,21 |
| 57 | 4306,66 | 28,9 | 116 | 1793,60 | 15,4 | 175 | 3256,25 | 29,3 | 234 | 1056,57 | 25,49 | 293 | 1201,59 | 24,85 |
| 58 | 900,43 | 19,8 | 117 | 2215,79 | 30,7 | 176 | 12716,23 | 23,1 | 235 | 1066,52 | 25,96 | 294 | 1204,65 | 21,93 |
| 59 | 1645,74 | 15,9 | 118 | 1934,82 | 14,2 | 177 | 1050,49 | 23,3 | 236 | 1068,56 | 21,69 | 295 | 1209,58 | 26,26 |
| 296 | 1210,45 | 36,58 | 355 | 1396,68 | 28,05 | 414 | 1632,79 | 38,03 | 473 | 1863,95 | 44,05 | 532 | 2138,03 | 21,78 |
| 297 | 1210,61 | 20,92 | 356 | 1413,62 | 25,78 | 415 | 1633,83 | 19,58 | 474 | 1880,97 | 44,03 | 533 | 2146 | 52,45 |
| 298 | 1213,62 | 27,75 | 357 | 1417,69 | 20,01 | 416 | 1635,83 | 30,38 | 475 | 1885,96 | 21,26 | 534 | 2188,03 | 39,71 |
| 299 | 1215,49 | 27,61 | 358 | 1421,58 | 37,51 | 417 | 1636,79 | 20,1 | 476 | 1889,94 | 33,12 | 535 | 2192,08 | 22,16 |
| 300 | 1216,59 | 24,27 | 359 | 1423,68 | 21,47 | 418 | 1636,81 | 30,33 | 477 | 1891,65 | 54,28 | 536 | 2195,49 | 33,36 |
| 301 | 1217,58 | 35,91 | 360 | 1425,65 | 22,34 | 419 | 1638,8 | 20,24 | 478 | 1892,95 | 22,4 | 537 | 2197,94 | 34,23 |
| 302 | 1218,42 | 36,88 | 361 | 1436,79 | 22,82 | 420 | 1640,75 | 28,28 | 479 | 1893,06 | 24,64 | 538 | 2199,08 | 22,31 |
| 303 | 1219,62 | 26,92 | 362 | 1438,72 | 21,98 | 421 | 1658,68 | 21,55 | 480 | 1899,93 | 21,64 | 539 | 2207,16 | 43,61 |
| 304 | 1220,63 | 22,11 | 363 | 1439,72 | 29,7 | 422 | 1658,79 | 30,04 | 481 | 1911,05 | 30,74 | 540 | 2208 | 32,37 |
| 305 | 1221,63 | 26,86 | 364 | 1440,72 | 39,19 | 423 | 1660,76 | 37,7 | 482 | 1912,74 | 32,7 | 541 | 2208,97 | 31,75 |
| 306 | 1223,6 | 27,47 | 365 | 1440,75 | 21,63 | 424 | 1664,82 | 29,87 | 483 | 1916,85 | 20,35 | 542 | 2211,11 | 20,28 |
| 307 | 1226,58 | 21 | 366 | 1442,68 | 27,76 | 425 | 1668,87 | 40,47 | 484 | 1926,91 | 30,99 | 543 | 2212,06 | 33,53 |
| 308 | 1226,63 | 23,47 | 367 | 1444,49 | 36,69 | 426 | 1673,82 | 29,4 | 485 | 1927,99 | 19,53 | 544 | 2219,56 | 33,59 |
| 309 | 1230,6 | 21,74 | 368 | 1448,83 | 20,53 | 427 | 1674,83 | 20,71 | 486 | 1932,98 | 21,65 | 545 | 2227,05 | 33,42 |
| 310 | 1234,42 | 36,16 | 369 | 1449,7 | 21,84 | 428 | 1680,82 | 30,02 | 487 | 1933,95 | 21,63 | 546 | 2233,1 | 22,49 |
| 311 | 1234,61 | 27,41 | 370 | 1451,71 | 29,19 | 429 | 1684,74 | 31,42 | 488 | 1936,11 | 20,51 | 547 | 2237,06 | 27,1 |
| 312 | 1238,55 | 36,74 | 371 | 1452,72 | 23,55 | 430 | 1684,81 | 39,43 | 489 | 1938,96 | 21,33 | 548 | 2244,17 | 19,48 |
| 313 | 1238,63 | 21,86 | 372 | 1456,75 | 22,48 | 431 | 1689,86 | 27,62 | 490 | 1942,77 | 36,19 | 549 | 2248,75 | 22,52 |
| 314 | 1239,52 | 34,91 | 373 | 1457,68 | 22 | 432 | 1693,83 | 20,48 | 491 | 1945,07 | 33,71 | 550 | 2251,77 | 33,11 |
| 315 | 1241,6 | 26,68 | 374 | 1458,69 | 28,03 | 433 | 1693,84 | 23,48 | 492 | 1946,95 | 31,75 | 551 | 2264,12 | 22,67 |
| 316 | 1245,6 | 21,63 | 375 | 1460,71 | 20,31 | 434 | 1697,81 | 30,86 | 493 | 1949,97 | 21,66 | 552 | 2269,29 | 38,14 |
| 317 | 1247,58 | 22,02 | 376 | 1473,7 | 22,3 | 435 | 1698,67 | 37,53 | 494 | 1952,42 | 25,65 | 553 | 2279,57 | 23,77 |
| 318 | 1250,52 | 35,35 | 377 | 1474,75 | 19,94 | 436 | 1705,78 | 46,77 | 495 | 1955,04 | 25,29 | 554 | 2282,13 | 22,29 |
| 319 | 1257,63 | 26,86 | 378 | 1483,72 | 22,59 | 437 | 1708,85 | 31,08 | 496 | 1955,94 | 28,14 | 555 | 2283,12 | 26,51 |
| 320 | 1260,61 | 21,84 | 379 | 1487,71 | 29,58 | 438 | 1725,66 | 38,3 | 497 | 1969,43 | 32,31 | 556 | 2292,21 | 21,29 |
| 321 | 1263,6 | 22,72 | 380 | 1491,8 | 39,86 | 439 | 1728,84 | 36,74 | 498 | 1976,96 | 32,36 | 557 | 2302,38 | 26,16 |
| 322 | 1267,64 | 21,55 | 381 | 1493,74 | 22,11 | 440 | 1729,6 | 53,46 | 499 | 1977,99 | 32,21 | 558 | 2305,7 | 34,8 |
| 323 | 1281,64 | 27,16 | 382 | 1507,8 | 39,98 | 441 | 1737,85 | 31,04 | 500 | 1989,02 | 24,75 | 559 | 2310,15 | 41,24 |
| 324 | 1284,62 | 28,22 | 383 | 1508,77 | 23,17 | 442 | 1746,95 | 19,75 | 501 | 1992,02 | 22,05 | 560 | 2315,22 | 19,45 |
| 325 | 1284,69 | 21,88 | 384 | 1509,59 | 37,07 | 443 | 1749,88 | 30,61 | 502 | 1993,02 | 27,07 | 561 | 2318,29 | 26,28 |
| 326 | 1287,65 | 21,85 | 385 | 1510,72 | 28,39 | 444 | 1761,91 | 21,61 | 503 | 1993,96 | 32,2 | 562 | 2319,19 | 33,87 |
| 327 | 1296,65 | 19,37 | 386 | 1510,75 | 20,11 | 445 | 1764,86 | 29,97 | 504 | 1997,99 | 25,13 | 563 | 2320,79 | 22,48 |
| 328 | 1300,65 | 23,07 | 387 | 1522,78 | 22,81 | 446 | 1767,07 | 24,12 | 505 | 2004,02 | 24,63 | 564 | 2322,17 | 22,28 |
| 329 | 1304,66 | 21,85 | 388 | 1523,89 | 29,75 | 447 | 1768,9 | 20,78 | 506 | 2006,93 | 33 | 565 | 2323,14 | 22,39 |
| 330 | 1309,74 | 21,97 | 389 | 1524,72 | 20,02 | 448 | 1769,78 | 28,25 | 507 | 2008,03 | 22,13 | 566 | 2328,06 | 20,83 |
| 331 | 1310,64 | 27,14 | 390 | 1535,75 | 22,03 | 449 | 1773,87 | 34,89 | 508 | 2021,06 | 21,98 | 567 | 2332,08 | 50,2 |
| 332 | 1321,63 | 37,98 | 391 | 1540,81 | 39,97 | 450 | 1782,91 | 25,93 | 509 | 2022,97 | 33,39 | 568 | 2335,75 | 26,61 |
| 333 | 1321,65 | 28,4 | 392 | 1549,76 | 39,5 | 451 | 1783,86 | 39,94 | 510 | 2023,99 | 21,47 | 569 | 2339,08 | 34,01 |
| 334 | 1328,64 | 26,87 | 393 | 1552,57 | 37,35 | 452 | 1784,88 | 40,04 | 511 | 2026,1 | 25,66 | 570 | 2339,17 | 22,63 |
| 335 | 1338,66 | 23,99 | 394 | 1556,8 | 40,03 | 453 | 1786,66 | 38,34 | 512 | 2031 | 21,8 | 571 | 2343,49 | 20,08 |
| 336 | 1340,65 | 28,33 | 395 | 1560,69 | 22,08 | 454 | 1788,89 | 30,26 | 513 | 2035,07 | 40,19 | 572 | 2361,19 | 20,78 |
| 337 | 1349,48 | 36,47 | 396 | 1562,76 | 22,48 | 455 | 1794,84 | 30,49 | 514 | 2040,02 | 32,87 | 573 | 2367,48 | 34,22 |
| 338 | 1349,77 | 20,22 | 397 | 1563,77 | 29,56 | 456 | 1796,85 | 30,86 | 515 | 2042,14 | 25,21 | 574 | 2371,18 | 22,77 |
| 339 | 1350,65 | 21,56 | 398 | 1564,78 | 28,53 | 457 | 1798,79 | 37,33 | 516 | 2057,04 | 24,85 | 575 | 2377,19 | 20,79 |
| 340 | 1352,62 | 29,68 | 399 | 1567,77 | 20,2 | 458 | 1807,91 | 20,7 | 517 | 2065 | 24,48 | 576 | 2380,16 | 36,49 |
| 341 | 1352,83 | 24,47 | 400 | 1576,74 | 46,02 | 459 | 1813,78 | 31,79 | 518 | 2076,61 | 32,44 | 577 | 2389,33 | 22,4 |
| 342 | 1353,65 | 21,48 | 401 | 1577,75 | 40,06 | 460 | 1818,9 | 31,1 | 519 | 2078,05 | 22,47 | 578 | 2392,8 | 35,28 |
| 343 | 1358,44 | 36,46 | 402 | 1579,75 | 20,05 | 461 | 1821,88 | 30,43 | 520 | 2081,03 | 20,17 | 579 | 2394 | 23,93 |
| 344 | 1361,69 | 21,95 | 403 | 1579,78 | 29,83 | 462 | 1823,99 | 24,61 | 521 | 2087,96 | 19,46 | 580 | 2405,6 | 22,19 |
| 345 | 1362,72 | 22,13 | 404 | 1609,81 | 30,25 | 463 | 1825,87 | 31,81 | 522 | 2088,04 | 32,92 | 581 | 2410,16 | 23,45 |
| 346 | 1366,68 | 27,48 | 405 | 1610,91 | 30,12 | 464 | 1829,03 | 21,24 | 523 | 2088,95 | 23,68 | 582 | 2414,26 | 19,58 |
| 347 | 1368,68 | 21,7 | 406 | 1611,83 | 40,12 | 465 | 1834,9 | 31,15 | 524 | 2090,04 | 39,48 | 583 | 2430,19 | 28,28 |
| 348 | 1375,67 | 23,28 | 407 | 1611,89 | 23,27 | 466 | 1835,92 | 24,04 | 525 | 2095,53 | 24,83 | 584 | 2430,72 | 35,39 |
| 349 | 1378,67 | 28,85 | 408 | 1613,89 | 23,98 | 467 | 1838,89 | 27,03 | 526 | 2100,12 | 19,6 | 585 | 2436,15 | 22,8 |
| 350 | 1383,69 | 39,02 | 409 | 1619,78 | 22,6 | 468 | 1846,93 | 32,08 | 527 | 2110 | 24,11 | 586 | 2437,22 | 22,86 |
| 351 | 1390,5 | 37,05 | 410 | 1620,74 | 29,58 | 469 | 1852,96 | 25,14 | 528 | 2114,07 | 31,61 | 587 | 2445,8 | 28,15 |
| 352 | 1392,68 | 21,75 | 411 | 1620,79 | 22,71 | 470 | 1858,91 | 24,21 | 529 | 2118,04 | 27,72 | 588 | 2451,59 | 24,02 |
| 353 | 1395,71 | 22,59 | 412 | 1624,61 | 37,73 | 471 | 1859,9 | 24,38 | 530 | 2129,06 | 26,97 | 589 | 2471,25 | 34,76 |
| 354 | 1396,5 | 37,63 | 413 | 1631,78 | 47,02 | 472 | 1860,42 | 33,82 | 531 | 2134,04 | 27,78 | 590 | 2478,22 | 22,45 |
| 591 | 2479,15 | 48,79 | 650 | 2989,4 | 24,42 | 709 | 3333,28 | 19,42 | 768 | 4083,75 | 31,32 | 827 | 8853,9 | 21,1 |
| 592 | 2485,16 | 34,39 | 651 | 2999,23 | 22,22 | 710 | 3333,8 | 23,85 | 769 | 4097,98 | 24,6 | 828 | 8917,52 | 22,55 |
| 593 | 2501,21 | 34,37 | 652 | 3002,47 | 23,95 | 711 | 3334,27 | 31 | 770 | 4113,77 | 24,46 | 829 | 10199,94 | 21,11 |
| 594 | 2502,73 | 22,84 | 653 | 3011,46 | 29,72 | 712 | 3336,48 | 39,43 | 771 | 4122,1 | 24,06 | 830 | 10753,72 | 19,71 |
| 595 | 2507,21 | 22,78 | 654 | 3013,27 | 22,28 | 713 | 3341,91 | 21,05 | 772 | 4124,05 | 21,49 | 831 | 10770,19 | 19,64 |
| 596 | 2518,4 | 22,8 | 655 | 3017,95 | 49,05 | 714 | 3356,38 | 25,56 | 773 | 4143,81 | 26,55 | 832 | 11041,95 | 21,98 |
| 597 | 2525,21 | 27,72 | 656 | 3023,33 | 24,55 | 715 | 3359,67 | 31,88 | 774 | 4196,47 | 20,96 | 833 | 11738,36 | 19,6 |
| 598 | 2534,56 | 22,81 | 657 | 3025,69 | 29,85 | 716 | 3363,69 | 30,21 | 775 | 4215,52 | 26,95 | 834 | 11967,98 | 20,48 |
| 599 | 2548,38 | 35,1 | 658 | 3035 | 42,23 | 717 | 3375,69 | 31,9 | 776 | 4217,98 | 26 | 835 | 14111,34 | 21,97 |
| 600 | 2551,32 | 34,75 | 659 | 3035,17 | 47,18 | 718 | 3386,16 | 22,7 | 777 | 4229,11 | 29,06 | 836 | 3266,45 | 22,73 |
| 601 | 2553,52 | 34,27 | 660 | 3038,11 | 22,42 | 719 | 3400,07 | 42,03 | 778 | 4278,77 | 23,34 | | | |
| 602 | 2557,15 | 28,17 | 661 | 3041,47 | 29,95 | 720 | 3416,82 | 36,78 | 779 | 4306 | 25,13 | | | |
| 603 | 2559,25 | 19,44 | 662 | 3044,98 | 34,34 | 721 | 3435,94 | 22,7 | 780 | 4322,14 | 25,18 | | | |
| 604 | 2565,18 | 23,74 | 663 | 3047,64 | 26,68 | 722 | 3457,46 | 35,83 | 781 | 4339 | 25,4 | | | |
| 605 | 2567,28 | 28,17 | 664 | 3056,94 | 45,28 | 723 | 3457,73 | 31,51 | 782 | 4352,98 | 20,17 | | | |
| 606 | 2570,28 | 42,66 | 665 | 3064,36 | 20,55 | 724 | 3472,36 | 21,75 | 783 | 4363,14 | 23,05 | | | |
| 607 | 2574,08 | 32,92 | 666 | 3076,33 | 19,64 | 725 | 3510,65 | 40,23 | 784 | 4369,07 | 20,21 | | | |
| 608 | 2580,19 | 23 | 667 | 3080,99 | 29,92 | 726 | 3521,07 | 30,74 | 785 | 4378,88 | 23,14 | | | |
| 609 | 2587,39 | 21,17 | 668 | 3091,41 | 28,33 | 727 | 3530,63 | 26,11 | 786 | 4381,08 | 34,13 | | | |
| 610 | 2599,26 | 28,18 | 669 | 3092,45 | 30,97 | 728 | 3531,46 | 30,16 | 787 | 4395,06 | 23,09 | | | |
| 611 | 2607,6 | 34,4 | 670 | 3092,51 | 36,23 | 729 | 3546,82 | 26,7 | 788 | 4415,72 | 22,53 | | | |
| 612 | 2616 | 28,34 | 671 | 3098,49 | 30,07 | 730 | 3559,71 | 24,89 | 789 | 4426,27 | 20,09 | | | |
| 613 | 2627,08 | 23,44 | 672 | 3112,42 | 22,44 | 731 | 3571,61 | 31,03 | 790 | 4436,04 | 26,25 | | | |
| 614 | 2632,18 | 24,96 | 673 | 3113,81 | 20,39 | 732 | 3582,7 | 19,47 | 791 | 4441,22 | 22,43 | | | |
| 615 | 2639,41 | 20,98 | 674 | 3114,66 | 30,3 | 733 | 3583,5 | 41,45 | 792 | 4483,33 | 25,4 | | | |
| 616 | 2668,46 | 42,02 | 675 | 3121,31 | 30,23 | 734 | 3590,72 | 29,01 | 793 | 4491,91 | 26,23 | | | |
| 617 | 2682,22 | 22,49 | 676 | 3129,86 | 35,93 | 735 | 3596,51 | 21,57 | 794 | 4538,61 | 26,35 | | | |
| 618 | 2702,1 | 38,01 | 677 | 3136,55 | 24,5 | 736 | 3603,92 | 20,21 | 795 | 4548,93 | 26,45 | | | |
| 619 | 2710,58 | 35,15 | 678 | 3139,44 | 29,48 | 737 | 3633,02 | 33,15 | 796 | 4550,95 | 23,31 | | | |
| 620 | 2723,92 | 23,81 | 679 | 3143,51 | 32,87 | 738 | 3633,71 | 27,02 | 797 | 4567,27 | 23,29 | | | |
| 621 | 2726,32 | 28,87 | 680 | 3145,49 | 38,8 | 739 | 3635,47 | 31,87 | 798 | 4583,7 | 35,76 | | | |
| 622 | 2726,37 | 42,94 | 681 | 3152,23 | 24,54 | 740 | 3669,71 | 24,13 | 799 | 4619,43 | 25,07 | | | |
| 623 | 2733,84 | 34,18 | 682 | 3158,6 | 29,69 | 741 | 3686,04 | 22,21 | 800 | 4637,97 | 25,75 | | | |
| 624 | 2742,36 | 42,12 | 683 | 3168,38 | 24,69 | 742 | 3696,88 | 26,94 | 801 | 4805,62 | 26,47 | | | |
| 625 | 2748,87 | 36,35 | 684 | 3178,26 | 30,25 | 743 | 3702,45 | 32,45 | 802 | 4817,33 | 23,86 | | | |
| 626 | 2754,36 | 29,64 | 685 | 3184,67 | 24,91 | 744 | 3703,85 | 22,51 | 803 | 4833,29 | 23,89 | | | |
| 627 | 2761,4 | 21,49 | 686 | 3193,27 | 22,63 | 745 | 3706,68 | 22 | 804 | 4849,07 | 23,86 | | | |
| 628 | 2777,61 | 21,54 | 687 | 3195,2 | 20,64 | 746 | 3710,86 | 19,6 | 805 | 4863,23 | 26,67 | | | |
| 629 | 2796,02 | 21 | 688 | 3202,2 | 30,52 | 747 | 3737,71 | 37,15 | 806 | 4864,81 | 23,88 | | | |
| 630 | 2810,46 | 36,66 | 689 | 3204,32 | 35,31 | 748 | 3738,58 | 24,74 | 807 | 4933,46 | 20,58 | | | |
| 631 | 2816,5 | 28,41 | 690 | 3219,43 | 35,05 | 749 | 3765,51 | 20,21 | 808 | 4976,46 | 20,62 | | | |
| 632 | 2821,19 | 23,73 | 691 | 3223,42 | 24,74 | 750 | 3774,84 | 22,9 | 809 | 5027,18 | 26,53 | | | |
| 633 | 2837,91 | 23,99 | 692 | 3240,47 | 25,2 | 751 | 3775,25 | 27 | 810 | 5039,24 | 25,56 | | | |
| 634 | 2839,42 | 24,17 | 693 | 3249,16 | 22,61 | 752 | 3775,9 | 25,28 | 811 | 5043,12 | 26,54 | | | |
| 635 | 2853,32 | 23,8 | 694 | 3258,51 | 22,89 | 753 | 3790,98 | 32,88 | 812 | 5090,45 | 20,1 | | | |
| 636 | 2861,32 | 50,73 | 695 | 3260,4 | 41,55 | 754 | 3816,45 | 21,94 | 813 | 5160,13 | 21,74 | | | |
| 637 | 2887,42 | 35,66 | 696 | 3261,35 | 22,17 | 755 | 3839,91 | 19,7 | 814 | 5229,59 | 25,1 | | | |
| 638 | 2889,19 | 24,09 | 697 | 3265,33 | 36,02 | 756 | 3864,66 | 33,71 | 815 | 5276,59 | 20,04 | | | |
| 639 | 2889,65 | 28,73 | 698 | 3271,39 | 30,75 | 757 | 3906,75 | 24,13 | 816 | 5495,5 | 19,52 | | | |
| 640 | 2903,72 | 35,61 | 699 | 3276,25 | 33,59 | 758 | 3935,56 | 34,14 | 817 | 5574,44 | 23,22 | | | |
| 641 | 2911 | 21,68 | 700 | 3282,79 | 30,09 | 759 | 3948,83 | 45,72 | 818 | 6186,07 | 24,91 | | | |
| 642 | 2914,57 | 24,28 | 701 | 3287,39 | 30,84 | 760 | 3968,68 | 21,11 | 819 | 6237,1 | 21,07 | | | |
| 643 | 2917,41 | 29,03 | 702 | 3290,39 | 24,1 | 761 | 4002,73 | 20,66 | 820 | 6491,14 | 19,44 | | | |
| 644 | 2924,58 | 22,97 | 703 | 3295,51 | 25,38 | 762 | 4008,95 | 23,57 | 821 | 6650,84 | 25,52 | | | |
| 645 | 2926,2 | 22,16 | 704 | 3298,48 | 36,06 | 763 | 4025,43 | 33,23 | 822 | 7907,23 | 19,59 | | | |
| 646 | 2932,36 | 34,11 | 705 | 3310,26 | 36,5 | 764 | 4042,93 | 33,13 | 823 | 7958,72 | 34,38 | | | |
| 647 | 2942,4 | 22,22 | 706 | 3314,41 | 20,12 | 765 | 4044,98 | 26,39 | 824 | 8289,54 | 19,54 | | | |
| 648 | 2977,61 | 29,14 | 707 | 3318,68 | 30,98 | 766 | 4069,65 | 25,2 | 825 | 8445,41 | 19,52 | | | |
| 649 | 2982,9 | 22,24 | 708 | 3325,9 | 54,11 | 767 | 4081,58 | 24,53 | 826 | 8837,64 | 21,06 | | | |

Mit der vorliegenden Erfindung ist es möglich, Blasenkrebs sehr frühzeitig zu diagnostizieren. Dadurch kann die Krankheit in einem frühen Stadium durch bekannte Verfahren therapiert werden. Die Erfindung ermöglicht weiterhin eine kostengünstige, schnelle und zuverlässige Abschätzung des Tumorstadiums sowie eine Diagnose bei zum Teil nicht oder nur minimal invasiven Eingriffen.

Die Migrationszeit wird mittels Kapillarelektrophorese (capillary electrophoresis, CE) -wie z.B. in Beispiel unter Punkt 2 ausgeführt- bestimmt. In diesem Beispiel wird eine 90 cm lange Glaskapillare mit einem inneren Durchmesser (ID) von 50 µm und einem äußeren Durchmesser (OD) von 360 µm bei einer angelegten Spannung von 30 kV betrieben. Als Laufmittel wird beispielsweise 30% Methanol, 0,5% Ameisensäure in Wasser verwendet.

Es ist bekannt, das die CE-Migrationszeit variieren kann. Dennoch ist die Reihenfolge, mit der die Polypeptidmarker eluieren, für jedes verwendete CE System unter den angegebenen Bedingungen typischerweise gleich. Um dennoch auftretende Unterschiede in der Migrationszeit auszugleichen, kann das System unter Verwendung von Standards, für die die Migrationszeiten genau bekannt sind, normiert werden. Diese Standards können z.B. die in den Beispielen angegebenen Polypeptide sein (siehe Beispiel Punkt 3).

Die Charakterisierung der Polypeptide, die in den Tabellen 1 bis 8 gezeigt sind, erfolgte mittels Kapillarelektrophorese-Massenspektrometrie (CE-MS), einem Verfahren, das z.B. ausführlich von Neuhoff et al., Rapid Comm. in mass spectrometry 2004 (20) 149-156 beschrieben wurde. Die Variation der Molekülmassen zwischen einzelnen Messungen oder zwischen verschiedenen Massenspektrometern ist bei exakter Kalibrierung relativ klein, typischerweise im Bereich von ± 0,1%, vorzugsweise im Bereich von ± 0,05%, mehr bevorzugt ± 0,03%.

Die erfindungsgemäßen Polypeptidmarker sind Proteine oder Peptide oder Abbauprodukte von Proteinen oder Peptiden. Sie können chemisch modifiziert sein, z.B. durch posttranslationale Modifikationen wie Glykolisierung, Phosphorylierung, Alkylierung oder Disulfidverbrückung, oder durch andere Reaktionen, z.B. im Rahmen des Abbaus, verändert sein. Darüber hinaus können die Polypeptidmarker auch im Rahmen der Aufreinigung der Proben chemisch verändert, z.B. oxidiert, sein.

Ausgehend von den Parametern, die die Polypeptidmarker bestimmen (Molekularmasse und Migrationszeit), ist es möglich, durch im Stand der Technik bekannte Verfahren die Sequenz der entsprechenden Polypeptide zu identifizieren.

Die erfindungsgemäßen Polypeptide (siehe Tabelle 1 bis 8) werden verwendet, um einerseits Blasenkrebs zu diagnostizieren als auch andererseits eine Unterscheidung zwischen verschiedenen Tumorstadien zu ermöglichen. Unter Diagnose versteht man den Vorgang der Erkenntnisgewinnung durch die Zuordnung von Symptomen oder Phänomenen zu einer Krankheit oder Verletzung. Im vorliegenden Fall wird von der An- oder Abwesenheit oder Amplitude bestimmter Polypeptidmarker auf das Vorliegen von Blasenkrebs sowie das Tumorstadium des Blasenkarzinoms geschlossen. Hierzu werden die erfindungsgemäßen Polypeptidmarker in einer Probe eines Individuums bestimmt, wobei, im Falle von Frequenzmarkern, ihre An- oder Abwesenheit bzw. im Falle von Amplitudenmarkern der Unterschied in der Signalintensität auf das Vorliegen von Blasenkrebs schließen lässt. Die An- oder Abwesenheit oder Amplitude eines Polypeptidmarkers kann durch jedes im Stand der Technik bekannte Verfahren gemessen werden. Verfahren, die verwendet werden können, sind weiter unten beispielhaft aufgeführt.

Ein Polypeptidmarker ist anwesend, wenn sein Messwert mindestens so hoch ist wie der Schwellenwert. Liegt sein Messwert darunter, ist der Polypeptidmarker abwesend. Der Schwellenwert kann entweder durch die Sensitivität des Messverfahrens (Nachweisgrenze) bestimmt werden oder anhand von Erfahrungen definiert werden.

Im Zusammenhang mit der vorliegenden Erfindung wird der Schwellenwert vorzugsweise überschritten, wenn der Messwert der Probe für eine bestimmte Molekularmasse mindestens doppelt so hoch ist, wie der einer Leerprobe (z.B. nur Puffer oder Lösungsmittel).

Der oder die Polypeptidmarker wird/werden in der Weise verwendet, dass seine/ihre An- oder Abwesenheit gemessen wird, wobei die An- oder Abwesenheit indikativ für Blasenkrebs ist (Frequenzmarker). So gibt es Polypeptidmarker, die typischerweise bei Patienten mit Blasenkrebs (krank) vorhanden sind, wie z.B. Polypeptidmarker Nr. 1 bis 44, jedoch bei Probanden ohne Blasenkrebs (Kontrolle) nicht oder nur selten vorhanden sind. Weiterhin gibt es Polypeptidmarker, die bei Individuen ohne Blasenkrebs vorhanden sind, jedoch bei Individuen mit Blasenkrebs seltener oder gar nicht auftreten, z.B. Nr. 45 bis 149 (Tabelle 2)

Ein Frequenzmarker ist eine Variante des Amplitudenmarkers, bei dem in einigen Proben die Amplitude gering ist. Es ist möglich, solche Frequenzmarker in Amplitudenmarker umzurechnen, in dem in die Berechnung der Amplitude die entsprechenden Proben, bei denen der Marker nicht gefunden wird, mit einer sehr kleinen Amplitude - im Bereich der Nachweisgrenze - in die Berechnung eingeht.

**Tabelle 2: Polypeptidmarker (Frequenzmarker) zur Diagnose von Blasenkrebs, ihre Molekularmassen und Migrationszeiten sowie ihre An- und Abwesenheit (Vorkommen) bei an Blasenkrebs (BC) erkrankten Patientengruppen sowie Kontrollgruppen als Faktor (1=100%, 0=0%; Probenaufarbeitung und Messung wie im Beispiel beschrieben).**

| Nummer | Vorkommen Gruppe BC | Vorkommen Kontrolle | Nummer | Vorkommen Gruppe BC | Vorkommen Kontrolle | Nummer | Vorkommen Gruppe BC | Vorkommen Kontrolle |
|---|---|---|---|---|---|---|---|---|
| 1 | 0,86 | 0,13 | 60 | 0 | 0,5 | 119 | 0,29 | 0,88 |
| 2 | 0,71 | 0 | 61 | 0 | 0,5 | 120 | 0,29 | 0,88 |
| 3 | 0,73 | 0,03 | 62 | 0 | 0,5 | 121 | 0,36 | 0,95 |
| 4 | 0,71 | 0,07 | 63 | 0 | 0,5 | 122 | 0 | 0,6 |
| 5 | 0,64 | 0,03 | 64 | 0 | 0,5 | 123 | 0 | 0,6 |
| 6 | 0,64 | 0,03 | 65 | 0 | 0,5 | 124 | 0 | 0,6 |
| 7 | 0,71 | 0,13 | 66 | 0,29 | 0,8 | 125 | 0 | 0,6 |
| 8 | 0,71 | 0,13 | 67 | 0,14 | 0,65 | 126 | 0,29 | 0,9 |
| 9 | 0,73 | 0,16 | 68 | 0,14 | 0,65 | 127 | 0,14 | 0,75 |
| 10 | 0,67 | 0,09 | 69 | 0,14 | 0,65 | 128 | 0,29 | 0,9 |
| 11 | 0,57 | 0 | 70 | 0,29 | 0,8 | 129 | 0,18 | 0,8 |
| 12 | 0,92 | 0,35 | 71 | 0,29 | 0,8 | 130 | 0,13 | 0,75 |
| 13 | 0,57 | 0 | 72 | 0,14 | 0,65 | 131 | 0,31 | 0,93 |
| 14 | 0,57 | 0 | 73 | 0,14 | 0,65 | 132 | 0,14 | 0,78 |
| 15 | 0,57 | 0,03 | 74 | 0,29 | 0,8 | 133 | 0 | 0,63 |
| 16 | 0,57 | 0,03 | 75 | 0,18 | 0,7 | 134 | 0,28 | 0,93 |
| 17 | 0,57 | 0,03 | 76 | 0,33 | 0,85 | 135 | 0 | 0,65 |
| 18 | 0,57 | 0,03 | 77 | 0,08 | 0,6 | 136 | 0,18 | 0,82 |
| 19 | 0,57 | 0,03 | 78 | 0,43 | 0,95 | 137 | 0,14 | 0,8 |
| 20 | 0,67 | 0,13 | 79 | 0,43 | 0,95 | 138 | 0,14 | 0,8 |
| 21 | 0,78 | 0,25 | 80 | 0,31 | 0,82 | 139 | 0 | 0,68 |
| 22 | 0,69 | 0,16 | 81 | 0,2 | 0,72 | 140 | 0 | 0,68 |
| 23 | 0,78 | 0,25 | 82 | 0,14 | 0,68 | 141 | 0 | 0,7 |
| 24 | 0,69 | 0,17 | 83 | 0,14 | 0,68 | 142 | 0 | 0,7 |
| 25 | 0,56 | 0,03 | 84 | 0,14 | 0,68 | 143 | 0 | 0,75 |
| 26 | 0,57 | 0,05 | 85 | 0,14 | 0,68 | 144 | 0 | 0,75 |
| 27 | 0,67 | 0,16 | 86 | 0 | 0,53 | 145 | 0,14 | 0,95 |
| 28 | 0,6 | 0,09 | 87 | 0 | 0,53 | 146 | 0,15 | 0,55 |
| 29 | 0,51 | 0 | 88 | 0,29 | 0,82 | 147 | 0,15 | 0,6 |
| 30 | 0,86 | 0,35 | 89 | 0,29 | 0,82 | 148 | 0,15 | 0,57 |
| 31 | 0,91 | 0,41 | 90 | 0,29 | 0,82 | 149 | 0,15 | 0,56 |
| 32 | 0,71 | 0,22 | 91 | 0,29 | 0,82 | | | |
| 33 | 0,51 | 0,03 | 92 | 0,29 | 0,82 | | | |
| 34 | 0,58 | 0,09 | 93 | 0,29 | 0,82 | | | |
| 35 | 0,89 | 0,41 | 94 | 0,29 | 0,82 | | | |
| 36 | 0,51 | 0,03 | 95 | 0,26 | 0,8 | | | |
| 37 | 0,73 | 0,25 | 96 | 0,43 | 0,97 | | | |
| 38 | 0,51 | 0,03 | 97 | 0,43 | 0,97 | | | |
| 39 | 0,62 | 0,16 | 98 | 0,43 | 0,97 | | | |
| 40 | 0,69 | 0,22 | 99 | 0,29 | 0,85 | | | |
| 41 | 0,58 | 0,13 | 100 | 0,14 | 0,7 | | | |
| 42 | 0,58 | 0,13 | 101 | 0,29 | 0,85 | | | |
| 43 | 0,73 | 0,38 | 102 | 0,14 | 0,7 | | | |
| 44 | 0,8 | 0,5 | 103 | 0,29 | 0,85 | | | |
| 45 | 0,42 | 0,72 | 104 | 0,29 | 0,85 | | | |
| 46 | 0,51 | 0,81 | 105 | 0,29 | 0,85 | | | |
| 47 | 0,44 | 0,75 | 106 | 0,26 | 0,82 | | | |
| 48 | 0,62 | 0,95 | 107 | 0 | 0,57 | | | |
| 49 | 0,57 | 0,9 | 108 | 0,26 | 0,82 | | | |
| 50 | 0,57 | 0,93 | 109 | 0 | 0,57 | | | |
| 51 | 0,29 | 0,65 | 110 | 0 | 0,57 | | | |
| 52 | 0,29 | 0,68 | 111 | 0 | 0,57 | | | |
| 53 | 0,42 | 0,81 | 112 | 0 | 0,57 | | | |
| 54 | 0,57 | 0,97 | 113 | 0,43 | 1 | | | |
| 55 | 0,14 | 0,55 | 114 | 0,24 | 0,81 | | | |
| 56 | 0,31 | 0,78 | 115 | 0,14 | 0,72 | | | |
| 57 | 0,33 | 0,81 | 116 | 0,14 | 0,72 | | | |
| 58 | 0 | 0,5 | 117 | 0,14 | 0,72 | | | |
| 59 | 0 | 0,5 | 118 | 0,29 | 0,88 | | | |

Die Verwendung der Marker 1-146 ist besonders bevorzugt.

Zusätzlich oder auch alternativ zu den Frequenzmarkern (Bestimmung der An- oder Abwesenheit) können auch die in Tabelle 3 und 4 angegebenen Amplitudenmarker zur Diagnose von Blasenkrebs verwendet werden (Nummer 150-836). Amplitudenmarker werden in der Weise verwendet, das nicht die An- oder Abwesenheit entscheidend ist, sondern die Höhe des Signals (die Amplitude) bei Anwesenheit des Signals in beiden Gruppen entscheidet. In Tabelle 3 und 4 sind die mittleren normalisierten Amplituden der entsprechenden Signale (charakterisiert über Masse and Migrationszeit) über alle gemessenen Proben angegeben. Dabei sind zwei Normierungsverfahren möglich, um eine Vergleichbarkeit zwischen unterschiedlich konzentrierten Proben oder unterschiedlichen Messmethoden zu erreichen:

Im ersten Ansatz werden alle Peptidsignale einer Probe auf eine Gesamtamplitude von 1 Million Counts normiert. Die jeweiligen mittleren Amplituden der Einzelmarker sind daher als parts per million (ppm) angegeben. Die sich nach diesem Verfahren ergebenden Amplitudenmarker sind in Tabelle 3 gezeigt (Nummer 150-185).

Zusätzlich besteht die Möglichkeit über ein alternatives Normierungsverfahren weitere Amplitudenmarker zu definieren: In diesem Fall werden alle Peptidsignale einer Probe mit einem gemeinsamen Normierungsfaktor skaliert. Dazu wird eine lineare Regression zwischen den Peptid-Amplituden der einzelnen Proben und den Referenzwerten aller bekannten Polypeptide gebildet. Die Steigung der Regressionsgeraden entspricht gerade der relativen Konzentration und wird als Normierungsfaktor für diese Probe verwandt. Die sich nach diesem Normierungsverfahren ergebenden Biomarker sind in Tabelle 4 gezeigt (Nummer 186-836).

Die Verwendung der Marker 150-185 ist besonders bevorzugt.

Alle verwendeten Gruppen bestehen aus mindestens 40 einzelnen Patienten- oder Kontrollproben, um eine verlässliche mittlere Amplitude zu erhalten. Die Entscheidung zu einer Diagnose (Blasenkrebs oder nicht) fällt dabei je nachdem, wie hoch die Amplitude der jeweiligen Polypeptidmarker in der Patientenprobe im Vergleich zu den mittleren Amplituden in der Kontrollgruppe bzw. der Blasenkrebs-Gruppe ist. Entspricht die Amplitude eher den mittleren Amplituden der Blasenkrebs-Gruppe, ist von Blasenkrebs auszugehen, entspricht sie eher den mittleren Amplituden der KontrollGruppe, ist nicht von Blasenkrebs auszugehen. Der Abstand zwischen dem Messwert und der mittleren Amplitude kann als eine Wahrscheinlichkeit für die Zugehörigkeit zu einer Gruppe betrachtet werden.

Beispielhaft soll anhand von Marker Nr. 162 (Tabelle 3) eine genauere Erläuterung für die Vorgehensweise nach Ansatz 1 gegeben werden. Die mittlere Amplitude des Markers ist bei Blasenkrebs deutlich erhöht (6370 ppm gegen 1431 ppm in der Kontrollgruppe). Liegt nun in einer Patientenprobe der Wert für diesen Marker bei 0 bis 1431 ppm, bzw. maximal 20% darüber, also 0 bis 1717 ppm, gehört diese Probe zur Kontrollgruppe. Liegt der Wert bei 6370 ppm, bzw. 20% darunter, oder höher, also zwischen 5096 ppm und sehr hohen Werten, ist von Blasenkrebs auszugehen.

Für Ansatz 2 soll anhand von Marker Nr. 192 (Tabelle 4) eine beispielhafte Erläuterung für die Vorgehensweise gegeben werden. Die mittlere Amplitude des Markers ist bei Blasenkrebs deutlich erhöht (374,91 counts gegen 44,29 counts in der Kontrollgruppe). Liegt nun in einer Patientenprobe der Wert für diesen Marker bei 0 bis 44,29 counts, bzw. maximal 20% darüber, also 0 bis 53,15 counts, gehört diese Probe zur Kontrollgruppe. Liegt der Wert bei 374,91 counts, bzw. 20% darunter, oder höher, also zwischen 300 counts und sehr hohen Werten, ist von Blasenkrebs auszugehen. Alternativ kann der Abstand zwischen dem Messwert und der mittleren Amplitude als eine Wahrscheinlichkeit für die Zugehörigkeit zu einer Gruppe betrachtet werden.

**Tabelle 3: Amplitudenmarker aus der ppm-Normierung (Ansatz 1)**

| Nummer | Mittlere Amplitude Gruppe BC [ppm] | Mittlere Amplitude Kontrollen [ppm] | Nummer | Mittlere Amplitude Nummer Gruppe BC [ppm] | Mittlere Amplitude Kontrollen [ppm] |
|---|---|---|---|---|---|
| 150 | 512 | 234 | 168 | 2602 | 981 |
| 151 | 4341 | 11833 | 169 | 321 | 745 |
| 152 | 320 | 107 | 170 | 106 | 308 |
| 153 | 4943 | 19582 | 171 | 596 | 1337 |
| 154 | 831 | 404 | 172 | 4128 | 10960 |
| 155 | 1615 | 4183 | 173 | 761 | 1961 |
| 156 | 882 | 2816 | 174 | 200 | 428 |
| 157 | 532 | 573 | 175 | 2150 | 4372 |
| 158 | 922 | 2703 | 176 | 995 | 2026 |
| 159 | 3070 | 7231 | 177 | 215 | 654 |
| 160 | 1863 | 3755 | 178 | 215 | 449 |
| 161 | 2322 | 5187 | 179 | 281 | 574 |
| 162 | 6370 | 1431 | 180 | 691 | 1578 |
| 163 | 1394 | 580 | 181 | 1860 | 4502 |
| 164 | 1039 | 428 | 182 | 1078 | 6650 |
| 165 | 225 | 542 | 183 | 406 | 846 |
| 166 | 769 | 1578 | 184 | 342 | 1360 |
| 167 | 42329 | 112162 | 185 | 1425 | 10166 |

**Tabelle 4: Amplitudenmarker aus der Normierung mit Normierungsfaktor (Ansatz 2)**

| Nummer | Mittlere Amplitude Gruppe BC | Mittlere Amplitude Kontrollen | Nummer | Mittlere Amplitude Gruppe BC | Mittlere Amplitude Kontrollen | Nummer | Mittlere Amplitude Gruppe BC | Mittlere Amplitude Kontrollen | Nummer | Mittlere Amplitude Gruppe BC | Mittlere Amplitude Kontrollen |
|---|---|---|---|---|---|---|---|---|---|---|---|
| 186 | 119,63 | 80,79 | 245 | 224,51 | 141,51 | 304 | 173,39 | 55,41 | 363 | 430 | 232,16 |
| 187 | 77,4 | 56,61 | 246 | 183,13 | 124,78 | 305 | 148,48 | 73,15 | 364 | 550,02 | 691,79 |
| 188 | 44,83 | 19,49 | 247 | 106,82 | 88,51 | 306 | 156,84 | 179,86 | 365 | 1968,51 | 197,72 |
| 189 | 75,44 | 45,2 | 248 | 3078,26 | 140,75 | 307 | 469,03 | 595,19 | 366 | 350,92 | 381,91 |
| 190 | 118,21 | 235,42 | 249 | 3190,15 | 3178,22 | 308 | 133,11 | 102,43 | 367 | 706,87 | 835,85 |
| 191 | 74,62 | 33,56 | 250 | 368,81 | 72,76 | 309 | 792,94 | 497,12 | 368 | 2384,33 | 175,16 |
| 192 | 374,91 | 44,29 | 251 | 256,09 | 245,38 | 310 | 1537,92 | 2939,46 | 369 | 3854,98 | 5258,01 |
| 193 | 157,08 | 123,78 | 252 | 130,05 | 134,05 | 311 | 1580,14 | 1795,44 | 370 | 14948,23 | 21376,25 |
| 194 | 453,57 | 10,31 | 253 | 448,49 | 239,5 | 312 | 193,48 | 190,55 | 371 | 728,16 | 250,02 |
| 195 | 88 | 45,2 | 254 | 232,48 | 315,01 | 313 | 794,93 | 201,72 | 372 | 552,5 | 144,36 |
| 196 | 90,46 | 720,98 | 255 | 205,39 | 158,14 | 314 | 928,42 | 699,24 | 373 | 210,05 | 246,6 |
| 197 | 111,95 | 85,68 | 256 | 404,85 | 342,62 | 315 | 143,26 | 115,01 | 374 | 347,41 | 249,22 |
| 198 | 410,43 | 469,76 | 257 | 249,07 | 176,66 | 316 | 303,03 | 129,16 | 375 | 207,87 | 315,21 |
| 199 | 427,05 | 319,41 | 258 | 94,05 | 45,1 | 317 | 1342,16 | 932,91 | 376 | 357,91 | 878,46 |
| 200 | 83,12 | 93,94 | 259 | 175,28 | 119,61 | 318 | 1790,3 | 3379,16 | 377 | 274,83 | 200,69 |
| 201 | 102,07 | 57,89 | 260 | 202,45 | 124,75 | 319 | 746,14 | 204,66 | 378 | 852,25 | 552,92 |
| 202 | 238,89 | 119,03 | 261 | 853,59 | 1140,19 | 320 | 706,29 | 396,08 | 379 | 427,78 | 628,41 |
| 203 | 111,31 | 90,37 | 262 | 1327,35 | 1993,54 | 321 | 609,39 | 342,54 | 380 | 344,12 | 480,68 |
| 204 | 195,66 | 197,97 | 263 | 356,82 | 284,59 | 322 | 201,07 | 279,53 | 381 | 10371,52 | 253,26 |
| 205 | 223,5 | 223,37 | 264 | 265,05 | 150,92 | 323 | 440,59 | 252,99 | 382 | 1974,82 | 2562,08 |
| 206 | 149,65 | 149,75 | 265 | 558,75 | 451,29 | 324 | 1500,12 | 166,99 | 383 | 869,39 | 408,55 |
| 207 | 159,86 | 56,01 | 266 | 221,77 | 143,72 | 325 | 231,13 | 194,08 | 384 | 72,76 | 94,63 |
| 208 | 353,83 | 70,68 | 267 | 1128,53 | 72,92 | 326 | 690,08 | 362,46 | 385 | 441,96 | 297,83 |
| 209 | 79,65 | 40,47 | 268 | 331,84 | 200,42 | 327 | 239,27 | 204,76 | 386 | 401,89 | 356,62 |
| 210 | 669,1 | 617,85 | 269 | 505,65 | 421,74 | 328 | 312,93 | 156,8 | 387 | 1259,94 | 1584,04 |
| 211 | 153,61 | 135,8 | 270 | 261,75 | 225,98 | 329 | 325,22 | 238 | 388 | 1178,53 | 2616,54 |
| 212 | 163,45 | 287,56 | 271 | 169,57 | 130,21 | 330 | 8160,78 | 334,35 | 389 | 358,67 | 507,39 |
| 213 | 1141,91 | 27,72 | 272 | 266,62 | 223,68 | 331 | 271 | 198,75 | 390 | 482,45 | 537,75 |
| 214 | 216,39 | 127,03 | 273 | 1362,12 | 1707,78 | 332 | 543,53 | 962,52 | 391 | 1542,53 | 2790,5 |
| 215 | 833,58 | 39,12 | 274 | 294,36 | 402,59 | 333 | 10101,83 | 12695,31 | 392 | 503,57 | 330,15 |
| 216 | 139 | 88,24 | 275 | 221,55 | 166,84 | 334 | 459,58 | 129,16 | 393 | 2430,83 | 3353,77 |
| 217 | 397,21 | 292,45 | 276 | 335,58 | 44,14 | 335 | 648,02 | 415,43 | 394 | 523,9 | 843,44 |
| 218 | 126,14 | 93,76 | 277 | 259,95 | 223,47 | 336 | 163,79 | 121,63 | 395 | 478,99 | 596,56 |
| 219 | 421,86 | 309,18 | 278 | 768,74 | 684,34 | 337 | 205,35 | 258,72 | 396 | 1207,17 | 1548,95 |
| 220 | 136,07 | 221,65 | 279 | 251,7 | 214,42 | 338 | 4607,6 | 550,93 | 397 | 248,65 | 483,71 |
| 221 | 152,19 | 120,89 | 280 | 169,01 | 89,8 | 339 | 112,46 | 181,81 | 398 | 166,63 | 140,29 |
| 222 | 219,55 | 145,69 | 281 | 206,44 | 145,14 | 340 | 1168,83 | 221,78 | 399 | 1049,78 | 696,02 |
| 223 | 125,21 | 31,02 | 282 | 3791,23 | 83,4 | 341 | 407,83 | 425,82 | 400 | 3862,62 | 4198,48 |
| 224 | 84,53 | 308,11 | 283 | 147,66 | 91,52 | 342 | 429,65 | 329,69 | 401 | 733,5 | 999,31 |
| 225 | 83,05 | 172,95 | 284 | 257,97 | 474,54 | 343 | 1760,83 | 2575,03 | 402 | 5193,79 | 7951,13 |
| 226 | 1842,39 | 1141,14 | 285 | 2312,84 | 72,28 | 344 | 514,01 | 309,71 | 403 | 2139,76 | 2730,17 |
| 227 | 1324,02 | 1031,48 | 286 | 2355,55 | 150,4 | 345 | 2515,14 | 115,68 | 404 | 916,36 | 816,75 |
| 228 | 350,65 | 89,47 | 287 | 1856,18 | 0 | 346 | 565,18 | 631,08 | 405 | 1684,49 | 1580,08 |
| 229 | 170,43 | 64,39 | 288 | 22744,05 | 27845,62 | 347 | 348,79 | 312,62 | 406 | 237,78 | 343,53 |
| 230 | 190,08 | 78,16 | 289 | 579,96 | 1024,9 | 348 | 184,84 | 126,88 | 407 | 2026,66 | 464,44 |
| 231 | 489,23 | 26,8 | 290 | 181 | 79,4 | 349 | 1992,63 | 2743,26 | 408 | 196,63 | 264,47 |
| 232 | 1996,47 | 72,63 | 291 | 449,65 | 287,08 | 350 | 4620,61 | 341,71 | 409 | 304,49 | 439,85 |
| 233 | 188,24 | 95,18 | 292 | 8039,6 | 6153,2 | 351 | 21183,33 | 33275,83 | 410 | 337,51 | 477,51 |
| 234 | 161,17 | 119,99 | 293 | 400,81 | 327,89 | 352 | 3541,13 | 4832,9 | 411 | 255,82 | 416,12 |
| 235 | 103,9 | 97,75 | 294 | 310,29 | 151 | 353 | 267,96 | 178,61 | 412 | 1221,53 | 1619,64 |
| 236 | 480,36 | 237,07 | 295 | 1031,54 | 860,46 | 354 | 260,72 | 600,85 | 413 | 9331,13 | 14100,68 |
| 237 | 5707,2 | 7336,84 | 296 | 633,16 | 564,48 | 355 | 598,13 | 660,69 | 414 | 197,33 | 300,63 |
| 238 | 379,06 | 56,43 | 297 | 218,71 | 298,42 | 356 | 318,88 | 381,49 | 415 | 212 | 165,73 |
| 239 | 308,98 | 301,08 | 298 | 687,69 | 58,53 | 357 | 784,39 | 610,28 | 416 | 1346,36 | 1460,35 |
| 240 | 462,23 | 78,2 | 299 | 462,1 | 597,81 | 358 | 394,52 | 233,81 | 417 | 820,34 | 1192,4 |
| 241 | 194,93 | 151,59 | 300 | 1581,02 | 1215,02 | 359 | 2575,84 | 1071,44 | 418 | 1548,36 | 1967,46 |
| 242 | 5055,93 | 4175,12 | 301 | 2630,29 | 3679,53 | 360 | 2460,15 | 3145,77 | 419 | 1218,82 | 1006,41 |
| 243 | 366,14 | 189,83 | 302 | 979,29 | 1913,27 | 361 | 1463,89 | 405,51 | 420 | 218,44 | 251,93 |
| 244 | 367,44 | 170,6 | 303 | 243,92 | 96,75 | 362 | 330,7 | 296,81 | 421 | 667,59 | 467,84 |
| 422 | 630,62 | 817,36 | 481 | 16250,01 | 1560,5 | 540 | 116,27 | 110,6 | 599 | 411,12 | 893,15 |
| 423 | 1128,79 | 1580,15 | 482 | 945,07 | 559,31 | 541 | 286,2 | 368,78 | 600 | 297,54 | 266,17 |
| 424 | 584,6 | 641,55 | 483 | 1339,21 | 1514,58 | 542 | 355,35 | 522,74 | 601 | 1122,23 | 1242,63 |
| 425 | 923,66 | 1154,89 | 484 | 358,02 | 192,21 | 543 | 3635,8 | 755,11 | 602 | 140,12 | 176,5 |
| 426 | 245,2 | 209,04 | 485 | 232,09 | 187,49 | 544 | 282,24 | 163,79 | 603 | 2821 | 1369,9 |
| 427 | 208,86 | 122,78 | 486 | 684,31 | 479,07 | 545 | 258,35 | 676,03 | 604 | 894,97 | 169,59 |
| 428 | 2141,81 | 2794,73 | 487 | 728,45 | 407,72 | 546 | 336,29 | 179,19 | 605 | 342,43 | 207,05 |
| 429 | 2673,32 | 3482,39 | 488 | 196,89 | 476,3 | 547 | 411,08 | 341,22 | 606 | 5208,16 | 8436,04 |
| 430 | 194,01 | 349,03 | 489 | 633,98 | 149,83 | 548 | 288,6 | 126,04 | 607 | 109,36 | 126,02 |
| 431 | 244,32 | 190,7 | 490 | 91,61 | 147,08 | 549 | 433,38 | 207,91 | 608 | 5005,47 | 886,26 |
| 432 | 171,13 | 269,09 | 491 | 502,32 | 282,04 | 550 | 468,04 | 764,16 | 609 | 385,8 | 404,35 |
| 433 | 389,06 | 220,01 | 492 | 441,16 | 507,47 | 551 | 514,82 | 290,72 | 610 | 384,35 | 2017,29 |
| 434 | 950,01 | 1103,05 | 493 | 714,01 | 210,4 | 552 | 309,49 | 252,04 | 611 | 1447,69 | 90,16 |
| 435 | 737,26 | 1145,11 | 494 | 349,59 | 153,45 | 553 | 352,9 | 63,73 | 612 | 189,43 | 158,12 |
| 436 | 698,31 | 1006,16 | 495 | 269,05 | 450,84 | 554 | 832,83 | 559,35 | 613 | 886,48 | 201,67 |
| 437 | 1034,44 | 1328,66 | 496 | 403,2 | 515,62 | 555 | 213,46 | 131,69 | 614 | 171,69 | 342,56 |
| 438 | 1549,89 | 2565,32 | 497 | 513,8 | 133,24 | 556 | 2691,55 | 211,72 | 615 | 712,07 | 723,54 |
| 439 | 242,37 | 346,97 | 498 | 295,46 | 431,46 | 557 | 5649,48 | 1095,48 | 616 | 604,67 | 979,18 |
| 440 | 4225,33 | 7163,11 | 499 | 790,03 | 915,09 | 558 | 267,74 | 274,93 | 617 | 645,45 | 1001,2 |
| 441 | 2026,98 | 2749,89 | 500 | 411,21 | 61,58 | 559 | 197,06 | 351,22 | 618 | 367,72 | 477,68 |
| 442 | 1669,33 | 64,04 | 501 | 1600,21 | 707,59 | 560 | 322,71 | 181,52 | 619 | 188,76 | 457,6 |
| 443 | 453,3 | 412,54 | 502 | 185,12 | 169,93 | 561 | 1774,17 | 965,1 | 620 | 612,89 | 94,01 |
| 444 | 284,09 | 110,58 | 503 | 211,53 | 252,44 | 562 | 674,15 | 1019,16 | 621 | 225,78 | 223,31 |
| 445 | 452,02 | 464,12 | 504 | 202,48 | 131,9 | 563 | 514,89 | 202,92 | 622 | 3910,57 | 6523,87 |
| 446 | 10928,25 | 6009,48 | 505 | 106,45 | 139,9 | 564 | 280,12 | 231,27 | 623 | 258,4 | 256,33 |
| 447 | 248,66 | 244,14 | 506 | 172,8 | 123,01 | 565 | 612,61 | 484,55 | 624 | 693,23 | 913,29 |
| 448 | 544,89 | 495,75 | 507 | 2238,19 | 1347,32 | 566 | 305,8 | 281,04 | 625 | 92,22 | 168,82 |
| 449 | 91,29 | 220,69 | 508 | 4881,51 | 210,22 | 567 | 6126,15 | 6394,03 | 626 | 338,07 | 497,04 |
| 450 | 690,87 | 378,52 | 509 | 386,33 | 241,64 | 568 | 564,34 | 127,81 | 627 | 1743,21 | 858,44 |
| 451 | 372,26 | 469,99 | 510 | 389,85 | 197,63 | 569 | 895,57 | 671,47 | 628 | 1934,06 | 274,04 |
| 452 | 695,43 | 437,27 | 511 | 1044,25 | 1860,09 | 570 | 234,83 | 74,14 | 629 | 3254,92 | 22,36 |
| 453 | 197,46 | 344,87 | 512 | 911,8 | 213,78 | 571 | 918,01 | 306,21 | 630 | 173,04 | 158,48 |
| 454 | 636,99 | 851,23 | 513 | 165,39 | 235,62 | 572 | 490,15 | 215,39 | 631 | 375,54 | 200,28 |
| 455 | 109,04 | 132,39 | 514 | 366,71 | 78,89 | 573 | 340,36 | 561,18 | 632 | 317,73 | 93 |
| 456 | 114,34 | 159,23 | 515 | 3952,23 | 943,15 | 574 | 293,37 | 165,07 | 633 | 2739,3 | 1120,66 |
| 457 | 247,87 | 278,24 | 516 | 394,1 | 154,31 | 575 | 1457,87 | 1267 | 634 | 563,71 | 786,49 |
| 458 | 1565,61 | 2420,43 | 517 | 721,07 | 344,83 | 576 | 186,86 | 193,04 | 635 | 1199,43 | 277,21 |
| 459 | 2149,51 | 2722,27 | 518 | 866,53 | 120,51 | 577 | 15200,82 | 840,95 | 636 | 744,12 | 919,2 |
| 460 | 1761,85 | 1313,47 | 519 | 384,56 | 159,41 | 578 | 445 | 510,51 | 637 | 346,57 | 197,87 |
| 461 | 458,34 | 487,39 | 520 | 1491,26 | 589,3 | 579 | 503,8 | 219,86 | 638 | 1090,93 | 283,03 |
| 462 | 1518,75 | 1405,34 | 521 | 969,57 | 410,04 | 580 | 3363,04 | 542,25 | 639 | 290,46 | 140,43 |
| 463 | 339,47 | 310,42 | 522 | 1522,66 | 1334,42 | 581 | 399,54 | 283,96 | 640 | 111,85 | 66,25 |
| 464 | 9070,76 | 1838,11 | 523 | 145,86 | 141,48 | 582 | 1073,6 | 416,48 | 641 | 455,21 | 420,21 |
| 465 | 3861,14 | 2831,77 | 524 | 189,68 | 287,18 | 583 | 344,17 | 359,53 | 642 | 345,83 | 199,62 |
| 466 | 423,42 | 203,85 | 525 | 1514,05 | 111,26 | 584 | 679,11 | 618,79 | 643 | 1651,16 | 1011,11 |
| 467 | 283,34 | 170,89 | 526 | 270,67 | 55,86 | 585 | 657,73 | 217,1 | 644 | 4741,83 | 614,65 |
| 468 | 367,72 | 315,21 | 527 | 223,41 | 313,99 | 586 | 3920,85 | 58,99 | 645 | 697,29 | 334,7 |
| 469 | 1424,84 | 339,86 | 528 | 220,43 | 162,9 | 587 | 192,77 | 218,19 | 646 | 277,24 | 288,32 |
| 470 | 1243,61 | 366,74 | 529 | 775,4 | 654,44 | 588 | 417,28 | 103,5 | 647 | 4690,58 | 3358,31 |
| 471 | 519,88 | 332,05 | 530 | 115,96 | 136,71 | 589 | 331,42 | 405,5 | 648 | 240,79 | 294,91 |
| 472 | 457,11 | 528,19 | 531 | 2682,19 | 2753,18 | 590 | 2854 | 126,77 | 649 | 641,99 | 200,78 |
| 473 | 1906,66 | 2592,95 | 532 | 1004,59 | 725,68 | 591 | 809,25 | 1010,64 | 650 | 371,35 | 238,75 |
| 474 | 840,95 | 1804,57 | 533 | 24215,6 | 23635,89 | 592 | 262,72 | 539,84 | 651 | 2349,83 | 1291,14 |
| 475 | 285,04 | 113,11 | 534 | 1380,23 | 1722,54 | 593 | 146,88 | 411,88 | 652 | 193,38 | 143,66 |
| 476 | 232,38 | 117,03 | 535 | 635,5 | 466,98 | 594 | 8079,35 | 1105,07 | 653 | 1226,05 | 1935,03 |
| 477 | 4444,41 | 11160,44 | 536 | 590,85 | 530,1 | 595 | 488,35 | 132,06 | 654 | 5138,39 | 3725,55 |
| 478 | 698,61 | 390,57 | 537 | 205,17 | 252,33 | 596 | 3335 | 859,68 | 655 | 3230,3 | 7444,98 |
| 479 | 648,4 | 507,64 | 538 | 262,07 | 133,39 | 597 | 516,5 | 679,53 | 656 | 746,95 | 386,71 |
| 480 | 442,16 | 240,49 | 539 | 236,94 | 716,14 | 598 | 1181,54 | 168,71 | 657 | 239,61 | 201,28 |
| 658 | 464,77 | 869,33 | 717 | 869,93 | 987,76 | 776 | 5071,3 | 7395,44 | 835 | 900,1 | 189,35 |
| 659 | 210,32 | 980,59 | 718 | 35304,93 | 419,08 | 777 | 271,15 | 292,43 | 836 | 4098,86 | 2173,05 |
| 660 | 3851,92 | 329,08 | 719 | 295,28 | 457,92 | 778 | 502,9 | 356,61 | | | |
| 661 | 2485,76 | 2716,97 | 720 | 97,18 | 91,86 | 779 | 4550,74 | 733,37 | | | |
| 662 | 347,83 | 623,37 | 721 | 5238,18 | 742,94 | 780 | 811,84 | 153,69 | | | |
| 663 | 145,28 | 189,73 | 722 | 1105,15 | 2446,05 | 781 | 5268,58 | 6177,03 | | | |
| 664 | 479,37 | 1413,92 | 723 | 15994,58 | 18395,94 | 782 | 964,7 | 441,9 | | | |
| 665 | 690,96 | 407,33 | 724 | 25574,15 | 56,87 | 783 | 1288,41 | 1241,77 | | | |
| 666 | 440,32 | 770,9 | 725 | 223,22 | 334,29 | 784 | 761,9 | 106,33 | | | |
| 667 | 348,99 | 416,59 | 726 | 390,33 | 591,38 | 785 | 302,57 | 220,29 | | | |
| 668 | 321,33 | 612,28 | 727 | 874,08 | 1016,73 | 786 | 441,39 | 34,69 | | | |
| 669 | 330,69 | 333,64 | 728 | 129,08 | 109,04 | 787 | 4805,19 | 408,09 | | | |
| 670 | 465,29 | 768,38 | 729 | 287,17 | 386,91 | 788 | 724,85 | 750,37 | | | |
| 671 | 804,23 | 897,03 | 730 | 938,84 | 1689,36 | 789 | 2797,11 | 4381,9 | | | |
| 672 | 331,26 | 117,39 | 731 | 249,13 | 373,29 | 790 | 1660,28 | 627,22 | | | |
| 673 | 18864,79 | 34,43 | 732 | 2681,59 | 934,95 | 791 | 82,16 | 51,86 | | | |
| 674 | 222,74 | 256,29 | 733 | 518,6 | 570,92 | 792 | 1500,15 | 777,08 | | | |
| 675 | 777,58 | 927,95 | 734 | 304,49 | 410,25 | 793 | 131,83 | 184,79 | | | |
| 676 | 218,75 | 161,04 | 735 | 515,18 | 321,95 | 794 | 1950,66 | 1314,25 | | | |
| 677 | 675,28 | 307,55 | 736 | 719,9 | 389,6 | 795 | 445,08 | 140,52 | | | |
| 678 | 1427,9 | 1826,69 | 737 | 162,63 | 213,43 | 796 | 421,99 | 182,5 | | | |
| 679 | 1500,77 | 1728,96 | 738 | 202,87 | 327,64 | 797 | 426,27 | 390,57 | | | |
| 680 | 1179,77 | 1493,41 | 739 | 310,7 | 366,38 | 798 | 498,29 | 564,91 | | | |
| 681 | 1035,25 | 621,15 | 740 | 1057,06 | 967,69 | 799 | 483,58 | 288,48 | | | |
| 682 | 827,45 | 539,76 | 741 | 1305,72 | 1525,5 | 800 | 177,85 | 148,57 | | | |
| 683 | 928,3 | 504,81 | 742 | 406,27 | 251,43 | 801 | 1927,19 | 290,82 | | | |
| 684 | 379,72 | 200,01 | 743 | 209,98 | 332,04 | 802 | 1908,71 | 378,98 | | | |
| 685 | 454,16 | 274,58 | 744 | 779,02 | 507,03 | 803 | 1356,99 | 309,29 | | | |
| 686 | 1247,87 | 797,98 | 745 | 509,21 | 780,22 | 804 | 590,4 | 454,65 | | | |
| 687 | 21125,88 | 178,01 | 746 | 550,52 | 205,81 | 805 | 479,33 | 263,09 | | | |
| 688 | 131 | 156,14 | 747 | 303,67 | 178,71 | 806 | 1688,17 | 334,94 | | | |
| 689 | 86,47 | 120,23 | 748 | 391,6 | 345,85 | 807 | 2524,15 | 1193,98 | | | |
| 690 | 184,62 | 196,27 | 749 | 161,04 | 486,26 | 808 | 613,82 | 295,85 | | | |
| 691 | 326,21 | 74,96 | 750 | 1633,21 | 1883,97 | 809 | 1847,5 | 1489 | | | |
| 692 | 467,89 | 307,96 | 751 | 1948,88 | 819,34 | 810 | 551,98 | 311,34 | | | |
| 693 | 796,96 | 485,58 | 752 | 947,01 | 1410,71 | 811 | 908,48 | 388,74 | | | |
| 694 | 2248,11 | 1305,99 | 753 | 88,29 | 103,94 | 812 | 124,16 | 66,69 | | | |
| 695 | 492,82 | 227,88 | 754 | 748,98 | 398,24 | 813 | 1567,12 | 859,71 | | | |
| 696 | 443,67 | 725,02 | 755 | 5654,78 | 2358,14 | 814 | 537,13 | 331,57 | | | |
| 697 | 810,12 | 535,52 | 756 | 176,88 | 284,44 | 815 | 1081,92 | 743,86 | | | |
| 698 | 998,77 | 1170,01 | 757 | 641,35 | 134,74 | 816 | 1288,32 | 753,52 | | | |
| 699 | 192,96 | 140,39 | 758 | 193,83 | 250,67 | 817 | 1522,33 | 1216,08 | | | |
| 700 | 115 | 132,51 | 759 | 265,87 | 443,61 | 818 | 3743,12 | 844,55 | | | |
| 701 | 2241,77 | 2710,35 | 760 | 1611,2 | 2134,8 | 819 | 211,47 | 155,57 | | | |
| 702 | 770,19 | 988,21 | 761 | 895 | 953,08 | 820 | 232,71 | 324,57 | | | |
| 703 | 401,99 | 247,99 | 762 | 4307,33 | 1318,11 | 821 | 311,5 | 454,92 | | | |
| 704 | 360,05 | 552,79 | 763 | 425,51 | 320,2 | 822 | 433,81 | 689,88 | | | |
| 705 | 107,75 | 98,14 | 764 | 157,27 | 248,54 | 823 | 2674,9 | 1470,8 | | | |
| 706 | 998,83 | 1032,67 | 765 | 2159,53 | 4308,89 | 824 | 2022,24 | 1507,34 | | | |
| 707 | 164,07 | 209,94 | 766 | 601,24 | 540,73 | 825 | 4346,69 | 2618,94 | | | |
| 708 | 1627,4 | 1118,73 | 767 | 259,57 | 190,8 | 826 | 2344,92 | 1552,78 | | | |
| 709 | 763,49 | 250,07 | 768 | 268,04 | 788,11 | 827 | 898,51 | 575,57 | | | |
| 710 | 773,85 | 675,53 | 769 | 1124,91 | 947,92 | 828 | 901,65 | 505,99 | | | |
| 711 | 155,22 | 286,79 | 770 | 345,81 | 258,56 | 829 | 1946,95 | 9898,6 | | | |
| 712 | 1272,34 | 2512,12 | 771 | 4076,84 | 585,19 | 830 | 961,96 | 4337,06 | | | |
| 713 | 27791,32 | 1191,08 | 772 | 1545,75 | 627,71 | 831 | 1561,13 | 2095,65 | | | |
| 714 | 459,25 | 281,17 | 773 | 635,06 | 927,49 | 832 | 5309,3 | 1583,61 | | | |
| 715 | 941,28 | 1159 | 774 | 1015,92 | 550,41 | 833 | 532,67 | 313,23 | | | |
| 716 | 341,85 | 213,77 | 775 | 475,86 | 490,03 | 834 | 855,38 | 600,48 | | | |

Die Polypeptidmarker sind auch geeignet, ein Tumorstadium zu bestimmen. Zur Differentialdiagnostik zwischen Patienten ohne BC und Patienten mit oberflächlichem BC eignen sich die Frequenzen gemäß Tabelle 5 und die Amplituden gemäß Tabelle 6.

**Tabelle 5: Frequenzmarker zur Unterscheidung von oberflächlichem BC und Patienten ohne BC**

| Nummer | Vorkommen Gruppe BC oberflächlich | Vorkommen Kontrolle |
|---|---|---|
| 146 | 0,13 | 0,55 |
| 147 | 0,15 | 0,6 |
| 148 | 0,17 | 0,57 |

**Tabelle 6: Amplitudenmarker zur Unterscheidung von oberflächlichem BC und Patienten ohne BC**

| Nummer | Mittlere Amplitude Gruppe BC oberflächlich | Mittlere Amplitude Kontrollen | Nummer | Mittlere Amplitude Gruppe BC oberflächlich | Mittlere Amplitude Kontrollen | Nummer | Mittlere Amplitude Gruppe BC oberflächlich | Mittlere Amplitude Kontrollen | Nummer | Mittlere Amplitude Gruppe BC oberflächlich | Mittlere Amplituden Kontrollen | Nummer | Mittlere Amplitude Gruppe BC oberflächlich | Mittlere Amplituden Kontrollen |
|---|---|---|---|---|---|---|---|---|---|---|---|---|---|---|
| 7 | 116,31 | 171,88 | 306 | 114,63 | 179,86 | 477 | 5333,72 | 11160,44 | 650 | 445,12 | 238,75 | 826 | 2857,72 | 2618,94 |
| 8 | 3346,12 | 1718,08 | 307 | 423,6 | 595,19 | 478 | 665 | 390,57 | 655 | 3310,08 | 7444,98 | 830 | 1733,28 | 9898,6 |
| 20 | 1414,75 | 204,11 | 311 | 1632,87 | 1795,44 | 480 | 182,45 | 240,49 | 656 | 582,62 | 386,71 | 831 | 451,53 | 4337,06 |
| 23 | 1152,72 | 758,3 | 312 | 244,52 | 190,55 | 481 | 30800,29 | 1560,5 | 657 | 293,05 | 201,28 | 836 | 2928,46 | 2173,05 |
| 31 | 13833,1 | 1997,34 | 313 | 147,27 | 201,72 | 482 | 1149,59 | 559,31 | 658 | 369,66 | 869,33 | | | |
| 37 | 404,73 | 159,1 | 318 | 1130,23 | 3379,16 | 483 | 1217,27 | 1514,58 | 659 | 288,42 | 980,59 | | | |
| 40 | 451,06 | 159,59 | 332 | 668,14 | 962,52 | 487 | 659,74 | 407,72 | 664 | 803,94 | 1413,92 | | | |
| 43 | 203,42 | 135,13 | 334 | 124,59 | 129,16 | 488 | 184,56 | 476,3 | 665 | 587,16 | 407,33 | | | |
| 44 | 619,98 | 943,28 | 340 | 135,75 | 221,78 | 491 | 780,25 | 282,04 | 666 | 469,6 | 770,9 | | | |
| 58 | 1631,78 | 2420,43 | 341 | 213,78 | 425,82 | 499 | 549,66 | 915,09 | 668 | 454,53 | 612,28 | | | |
| 61 | 109,9 | 106,43 | 343 | 1942,45 | 2575,03 | 502 | 230,68 | 169,93 | 670 | 421,48 | 333,64 | | | |
| 63 | 399,18 | 318,87 | 347 | 403,35 | 312,62 | 507 | 2145,46 | 1347,32 | 676 | 284,62 | 161,04 | | | |
| 70 | 255,14 | 168,72 | 350 | 406,28 | 341,71 | 510 | 329,78 | 197,63 | 681 | 891,35 | 621,15 | | | |
| 71 | 505,84 | 220,23 | 351 | 24046,12 | 33275,83 | 511 | 1149,81 | 1860,09 | 682 | 1299,73 | 539,76 | | | |
| 81 | 1457,02 | 1083,72 | 354 | 288,33 | 600,85 | 517 | 597,1 | 344,83 | 684 | 540,83 | 200,01 | | | |
| 89 | 287,82 | 410,5 | 359 | 1679,91 | 1071,44 | 519 | 214,5 | 159,41 | 693 | 695,98 | 485,58 | | | |
| 90 | 387,02 | 395,23 | 360 | 2442,99 | 3145,77 | 522 | 2040,96 | 1334,42 | 695 | 449,35 | 725,02 | | | |
| 92 | 971,64 | 1464,36 | 366 | 428,94 | 381,91 | 523 | 188,34 | 141,48 | 696 | 335,66 | 227,88 | | | |
| 97 | 147,12 | 266,31 | 367 | 1048,83 | 835,85 | 528 | 283,36 | 162,9 | 697 | 1024,07 | 535,52 | | | |
| 101 | 272,02 | 206,42 | 371 | 737,87 | 250,02 | 531 | 3574,56 | 2753,18 | 699 | 141,11 | 140,39 | | | |
| 115 | 203,74 | 303,62 | 373 | 158,25 | 246,6 | 535 | 704,86 | 466,98 | 704 | 195,53 | 552,79 | | | |
| 126 | 707,92 | 764,74 374 | | 416,91 | 249,22 | 536 | 800,65 | 530,1 | 705 | 132,97 | 98,14 | | | |
| 129 | 114,31 | 208,29 | 375 | 200,86 | 315,21 | 539 | 123,56 | 716,14 | 706 | 659,99 | 1032,67 | | | |
| 132 | 397,6 | 591,14 | 376 | 431,93 | 878,46 | 540 | 119,18 | 110,6 | 710 | 933,34 | 675,53 | | | |
| 135 | 449,32 | 298,56 | 381 | 3618,42 | 253,26 | 543 | 536,82 | 522,74 | 712 | 1576,36 | 2512,12 | | | |
| 137 | 216,96 | 149,67 | 384 | 70,76 | 94,63 | 544 | 228,52 | 163,79 | 716 | 418,32 | 213,77 | | | |
| 152 | 325,5 | 272,96 | 385 | 502,72 | 297,83 | 545 | 304,3 | 676,03 | 719 | 179,81 | 457,92 | | | |
| 156 | 3756 | 2810,21 | 386 | 241,72 | 356,62 | 547 | 510,33 | 341,22 | 726 | 379,78 | 591,38 | | | |
| 158 | 1109,21 | 1996,73 | 387 | 1388,15 | 1584,04 | 551 | 518,82 | 290,72 | 728 | 206,19 | 109,04 | | | |
| 170 | 110,86 | 224,3 | 389 | 324,34 | 507,39 | 552 | 322,61 | 252,04 | 729 | 207,95 | 386,91 | | | |
| 173 | 521,41 | 717,77 | 390 | 284,78 | 537,75 | 564 | 316,34 | 231,27 | 733 | 466,68 | 570,92 | | | |
| 176 | 712,45 | 869,56 | 391 | 1787,19 | 2790,5 | 566 | 256,99 | 281,04 | 738 | 227,37 | 327,64 | | | |
| 180 | 1377,28 | 2012,33 | 393 | 2600,66 | 3353,77 | 568 | 213,06 | 127,81 | 740 | 1593,31 | 967,69 | | | |
| 184 | 453,72 | 946,92 | 395 | 398,9 | 596,56 | 569 | 981 | 671,47 | 741 | 1317,94 | 1525,5 | | | |
| 185 | 10671,27 | 12125,03 | 398 | 187,41 | 140,29 | 574 | 256,67 | 165,07 | 742 | 396,52 | 251,43 | | | |
| 190 | 118,18 | 235,42 | 404 | 631,32 | 816,75 | 591 | 883,27 | 1010,64 | 745 | 366,95 | 780,22 | | | |
| 198 | 250,7 | 469,76 | 409 | 255,99 | 439,85 | 595 | 204,98 | 132,06 | 749 | 170,59 | 486,26 | | | |
| 210 | 446,64 | 617,85 | 412 | 1364,55 | 1619,64 | 599 | 349 | 893,15 | 752 | 852,35 | 1410,71 | | | |
| 212 | 112,84 | 287,56 | 414 | 216,1 | 300,63 | 600 | 410,22 | 266,17 | 753 | 69,09 | 103,94 | | | |
| 219 | 144 | 221,65 | 419 | 537,72 | 1006,41 | 601 | 1685,83 | 1242,63 | 759 | 198,1 | 443,61 | | | |
| 225 | 95,25 | 172,95 | 423 | 1441,12 | 1580,15 | 602 | 130,05 | 176,5 | 761 | 430,84 | 953,08 | | | |
| 226 | 785,46 | 1141,14 | 430 | 171,47 | 349,03 | 605 | 458,37 | 207,05 | 765 | 2889,47 | 4308,89 | | | |
| 237 | 4373,54 | 7336,84 | 432 | 194,68 | 269,09 | 609 | 409,71 | 404,35 | 766 | 883,73 | 540,73 | | | |
| 239 | 273,08 | 301,08 | 435 | 713,74 | 1145,11 | 610 | 621,93 | 2017,29 | 768 | 232,2 | 788,11 | | | |
| 249 | 2176,11 | 3178,22 | 438 | 1960,48 | 2565,32 | 614 | 192,26 | 342,56 | 769 | 728,06 | 947,92 | | | |
| 261 | 1093,03 | 1140,19 | 440 | 5360,16 | 7163,11 | 615 | 316,08 | 723,54 | 770 | 166,59 | 258,56 | | | |
| 262 | 1453,44 | 1993,54 | 445 | 594,8 | 464,12 | 618 | 342,45 | 477,68 | 778 | 783,21 | 356,61 | | | |
| 266 | 82,68 | 143,72 | 446 | 10467,64 | 6009,48 | 619 | 197,75 | 457,6 | 780 | 2863,23 | 733,37 | | | |
| 269 | 272,05 | 421,74 | 448 | 660,96 | 495,75 | 621 | 4385,63 | 6523,87 | 781 | 370,95 | 153,69 | | | |
| 271 | 115,76 | 130,21 | 449 | 105,44 | 220,69 | 625 | 129,34 | 168,82 | 798 | 465,94 | 390,57 | | | |
| 272 | 133,52 | 223,68 | 450 | 585,27 | 378,52 | 630 | 254,47 | 158,48 | 802 | 1483,13 | 290,82 | | | |
| 275 | 195,93 | 402,59 | 452 | 586,56 | 437,27 | 631 | 326,82 | 200,28 | 803 | 1420,86 | 378,98 | | | |
| 284 | 155,65 | 474,54 | 453 | 246,69 | 344,87 | 633 | 2257,95 | 1120,66 | 809 | 490,19 | 295,85 | | | |
| 288 | 18118,22 | 27845,62 | 460 | 2511,6 | 1313,47 | 636 | 730,41 | 919,2 | 810 | 1868,52 | 1489 | | | |
| 289 | 705,44 | 1024,9 | 461 | 753,53 | 487,39 | 638 | 373,94 | 283,03 | 811 | 421,44 | 311,34 | | | |
| 296 | 369,58 | 564,48 464 | | 20601,57 | 1838,11 639 | | 305,66 | 140,43 819 | | 1952,07 | 844,55 | | | |
| 299 | 418,65 | 597,81 | 470 | 590,47 | 366,74 | 643 | 1759,51 | 1011,11 | 821 | 232,74 | 324,57 | | | |
| 301 | 2355,61 | 3679,53 | 471 | 544,61 | 332,05 | 645 | 584,26 | 334,7 | 822 | 291,21 | 454,92 | | | |
| 303 | 255,32 | 96,75 | 474 | 879,35 | 1804,57 | 646 | 351,46 | 288,32 | 824 | 1711,53 | 1470,8 | | | |

Weithin ist es möglich, differentialdiagnostisch infiltrierende BC zu erkennen. Zur Differentialdiagnostik zwischen Patienten ohne BC und Patienten mit infiltrierendem BC eignen sich die Frequenzen gemäß Tabelle 7 und die Amplituden gemäß Tabelle 8.

**Tabelle 7: Frequenzmarker zur Unterscheidung von infiltrierendem BC und Patienten ohne BC**

| Nummer | Vorkommen Gruppe BC invasiv | Vorkommen Kontrolle | Nummer | Vorkommen Gruppe BC invasiv | Vorkommen Kontrolle | Nummer | Vorkommen Gruppe BC invasiv | Vorkommen Kontrolle |
|---|---|---|---|---|---|---|---|---|
| 1 | 0,44 | 0,04 | 330 | 0,51 | 0,02 | 708 | 0,75 | 0,07 |
| 3 | 0,89 | 0,34 | 338 | 0,44 | 0,02 | 709 | 0,69 | 0,28 |
| 4 | 0,76 | 0,14 | 345 | 0,55 | 0,06 | 712 | 0,33 | 0,76 |
| 7 | 0,95 | 0,37 | 361 | 0,47 | 0,02 | 713 | 0,58 | 0,02 |
| 11 | 0,58 | 0,17 | 365 | 0,58 | 0,07 | 718 | 0,47 | 0,02 |
| 16 | 0,44 | 0,01 | 368 | 0,51 | 0,01 | 721 | 0,42 | 0,02 |
| 23 | 0,84 | 0,14 | 372 | 0,53 | 0,02 | 724 | 0,49 | 0,07 |
| 27 | 0,89 | 0,3 | 377 | 0,62 | 0,13 | 736 | 0,53 | 0,06 |
| 28 | 0,56 | 0,02 | 383 | 0,71 | 0,12 | 746 | 0,78 | 0,22 |
| 29 | 0,53 | 0,07 | 390 | 0,69 | 0,2 | 750 | 0,6 | 0,14 |
| 30 | 0,76 | 0,16 | 394 | 0,2 | 0,61 | 757 | 0,56 | 0,16 |
| 31 | 0,93 | 0,46 | 400 | 0,27 | 0,75 | 759 | 0,16 | 0,6 |
| 34 | 0,6 | 0,13 | 407 | 0,55 | 0,12 | 762 | 0,6 | 0,2 |
| 39 | 0,76 | 0,24 | 413 | 0,33 | 0,88 | 771 | 0,73 | 0,22 |
| 91 | 0,4 | 0,88 | 415 | 0,6 | 0,19 | 779 | 0,47 | 0,04 |
| 112 | 0,75 | 0,34 | 425 | 0,24 | 0,69 | 780 | 0,78 | 0,33 |
| 132 | 0,25 | 0,86 | 426 | 0,04 | 0,47 | 788 | 0,53 | 0,04 |
| 134 | 0,27 | 0,8 | 433 | 0,67 | 0,19 | 791 | 0,49 | 0,04 |
| 179 | 0,71 | 0,24 | 436 | 0,07 | 0,48 | 802 | 0,67 | 0,27 |
| 180 | 0,64 | 0,18 | 440 | 0,2 | 0,68 | 803 | 0,71 | 0,28 |
| 186 | 0,62 | 0,16 | 442 | 0,53 | 0,02 | 804 | 0,71 | 0,14 |
| 187 | 0,73 | 0,19 | 451 | 0,13 | 0,62 | 806 | 0,51 | 0,07 |
| 189 | 0,53 | 0,06 | 466 | 0,71 | 0,27 | 807 | 0,53 | 0,06 |
| 192 | 0,42 | 0,01 | 469 | 0,56 | 0,04 | 808 | 0,73 | 0,18 |
| 194 | 0,44 | 0,02 | 474 | 0,2 | 0,66 | 825 | 0,55 | 0,07 |
| 200 | 0,62 | 0,14 | 477 | 0,24 | 0,71 | | | |
| 204 | 0,71 | 0,2 | 485 | 0,58 | 0,15 | | | |
| 205 | 0,76 | 0,3 | 494 | 0,56 | 0,07 | | | |
| 206 | 0,73 | 0,3 | 496 | 0,24 | 0,67 | | | |
| 207 | 0,6 | 0,11 | 497 | 0,67 | 0,16 | | | |
| 209 | 0,53 | 0,04 | 508 | 0,56 | 0,08 | | | |
| 211 | 0,84 | 0,28 | 525 | 0,47 | 0,06 | | | |
| 213 | 0,51 | 0,02 | 526 | 0,49 | 0,03 | | | |
| 215 | 0,58 | 0,07 | 533 | 0,16 | 0,59 | | | |
| 216 | 0,71 | 0,21 | 539 | 0,05 | 0,46 | | | |
| 228 | 0,49 | 0,06 | 542 | 0,45 | 0,06 | | | |
| 229 | 0,55 | 0,07 | 557 | 0,58 | 0,15 | | | |
| 230 | 0,51 | 0,08 | 560 | 0,51 | 0,06 | | | |
| 231 | 0,45 | 0,02 | 561 | 0,53 | 0,07 | | | |
| 232 | 0,78 | 0,24 | 567 | 0,29 | 0,73 | | | |
| 233 | 0,73 | 0,23 | 576 | 0,15 | 0,57 | | | |
| 234 | 0,45 | 0,05 | 580 | 0,78 | 0,24 | | | |
| 235 | 0,69 | 0,25 | 581 | 0,55 | 0,14 | | | |
| 240 | 0,71 | 0,1 | 582 | 0,64 | 0,2 | | | |
| 248 | 0,49 | 0,09 | 583 | 0,47 | 0,92 | | | |
| 255 | 0,85 | 0,38 | 591 | 0,25 | 0,7 | | | |
| 259 | 0,6 | 0,19 | 594 | 0,67 | 0,22 | | | |
| 260 | 0,71 | 0,21 | 596 | 0,55 | 0,07 | | | |
| 264 | 0,73 | 0,24 | 613 | 0,45 | 0,06 | | | |
| 267 | 0,53 | 0,02 | 620 | 0,64 | 0,07 | | | |
| 270 | 0,56 | 0,11 | 629 | 0,49 | 0,01 | | | |
| 277 | 0,84 | 0,39 | 635 | 0,73 | 0,22 | | | |
| 279 | 0,71 | 0,18 | 644 | 0,44 | 0,04 | | | |
| 280 | 0,69 | 0,17 | 655 | 0,27 | 0,73 | | | |
| 282 | 0,8 | 0,12 | 685 | 0,87 | 0,34 | | | |
| 287 | 0,4 | 0 | 687 | 0,55 | 0,07 | | | |
| 316 | 0,58 | 0,13 | 691 | 0,67 | 0,16 | | | |
| 325 | 0,47 | 0,04 | 692 | 0,75 | 0,2 | | | |
| 327 | 0,62 | 0,2 | 695 | 0,4 | 0,81 | | | |

**Tabelle 8: Amlitudenmarker zur Unterscheidung von infiltrierendem BC und Patienten ohne BC**

| Nummer | Mittlere Amplitude Gruppe BC_inf | Mittlere Amplitude Kontrollen | Nummer | Mittlere Amplitude Gruppe BC_inf | Mittlere Amplitude Kontrollen | Nummer | Mittlere Amplitude Gruppe BC_inf | Mittlere Amplitude Kontrollen | Nummer | Mittlere Amplitude Gruppe BC_inf | Mittlere Amplitude Kontrollen | | | |
|---|---|---|---|---|---|---|---|---|---|---|---|---|---|---|
| 5 | 681,6 | 915,74 | 173 | 355,05 | 717,77 | 281 | 243,62 | 145,14 | 363 | 632,9 | 232,16 | | | |
| 6 | 815,21 | 734,8 | 174 | 336,82 | 673,1 | 283 | 160,68 | 91,52 | 364 | 639,09 | 691,79 | | | |
| 9 | 1243,87 | 782,89 | 175 | 1222,79 | 2369,24 | 284 | 337,03 | 474,54 | 369 | 3411,18 | 5258,01 | | | |
| 10 | 141,18 | 124,95 | 176 | 845,59 | 869,56 | 285 | 2505,38 | 72,28 | 370 | 11667,49 | 21376,25 | | | |
| 21 | 1635,84 | 1958,08 | 178 | 324,58 | 744,72 | 286 | 2549,36 | 150,4 | 371 | 725,25 | 250,02 | | | |
| 32 | 397,54 | 254,21 | 179 | 439,74 | 900,41 | 289 | 467,03 | 1024,9 | 376 | 283,9 | 878,46 | | | |
| 37 | 446,42 | 159,1 | 180 | 1096,91 | 2012,33 | 290 | 186,3 | 79,4 | 378 | 728,83 | 552,92 | | | |
| 40 | 1989,31 | 159,59 | 182 | 832,52 | 1599,5 | 291 | 558,3 | 287,08 | 379 | 411,32 | 628,41 | | | |
| 41 | 392 | 216,08 | 183 | 968,19 | 1591,47 | 292 | 9841,21 | 6153,2 | 380 | 276,41 | 480,68 | | | |
| 43 | 222,66 | 135,13 | 188 | 47,58 | 19,49 | 293 | 414,98 | 327,89 | 381 | 13594,59 | 253,26 | | | |
| 44 | 580,55 | 943,28 | 190 | 118,25 | 235,42 | 294 | 348,42 | 151 | 382 | 1797,04 | 2562,08 | | | |
| 46 | 512,02 | 1158,64 | 191 | 79,98 | 33,56 | 295 | 1234,05 | 860,46 | 384 | 74,02 | 94,63 | | | |
| 48 | 425,9 | 664,52 | 193 | 192,29 | 123,78 | 296 | 783,79 | 564,48 | 386 | 459,1 | 356,62 | | | |
| 52 | 4960,1 | 2173,05 | 195 | 95,67 | 45,2 | 297 | 213,75 | 298,42 | 388 | 649,43 | 2616,54 | | | |
| 53 | 433,44 | 537,27 | 196 | 90,6 | 720,98 | 298 | 840,15 | 58,53 | 391 | 1297,86 | 2790,5 | | | |
| 56 | 1116,75 | 2641,97 | 197 | 127,3 | 85,68 | 300 | 1990,34 | 1215,02 | 392 | 538,4 | 330,15 | | | |
| 57 | 366,09 | 577,01 | 199 | 583,69 | 319,41 | 302 | 671,08 | 1913,27 | 396 | 1118,64 | 1548,95 | | | |
| 63 | 189,75 | 318,87 | 201 | 107,79 | 57,89 | 304 | 194,42 | 55,41 | 397 | 255,21 | 483,71 | | | |
| 66 | 1298,04 | 761,77 | 202 | 277,13 | 119,03 | 305 | 172,63 | 73,15 | 398 | 122,48 | 140,29 | | | |
| 69 | 413,77 | 604,54 | 203 | 135,81 | 90,37 | 308 | 147,74 | 102,43 | 399 | 1368,03 | 696,02 | | | |
| 72 | 275,44 | 190,05 | 208 | 374,07 | 70,68 | 309 | 867,46 | 497,12 | 401 | 368,33 | 999,31 | | | |
| 78 | 1447,52 | 957,12 | 210 | 815,93 | 617,85 | 310 | 1133,99 | 2939,46 | 402 | 4724,92 | 7951,13 | | | |
| 80 | 117,88 | 139,63 | 212 | 197,69 | 287,56 | 313 | 1096,17 | 201,72 | 403 | 1526,84 | 2730,17 | | | |
| 81 | 716,39 | 1083,72 | 214 | 286,24 | 127,03 | 314 | 1191,03 | 699,24 | 405 | 702,99 | 1580,08 | | | |
| 85 | 327,7 | 495,82 | 217 | 495,39 | 292,45 | 315 | 162,8 | 115,01 | 406 | 167,28 | 343,53 | | | |
| 88 | 429,34 | 216,08 | 218 | 149,46 | 93,76 | 317 | 1710,2 | 932,91 | 408 | 142,98 | 264,47 | | | |
| 92 | 373,18 | 1464,36 | 220 | 542,41 | 309,18 | 319 | 818,29 | 204,66 | 410 | 250,12 | 477,51 | | | |
| 93 | 380,46 | 315,1 | 221 | 175,55 | 120,89 | 320 | 874,9 | 396,08 | 411 | 241,86 | 416,12 | | | |
| 101 | 239,84 | 206,42 | 222 | 178,86 | 145,69 | 321 | 699,24 | 342,54 | 412 | 1103,38 | 1619,64 | | | |
| 111 | 99,21 | 241,12 | 223 | 134,7 | 31,02 | 322 | 135,37 | 279,53 | 414 | 141,03 | 300,63 | | | |
| 113 | 695,63 | 1862,59 | 224 | 90,56 | 308,11 | 323 | 487,82 | 252,99 | 416 | 999,69 | 1460,35 | | | |
| 114 | 377,09 | 503,56 | 226 | 2547,02 | 1141,14 | 324 | 2230,59 | 166,99 | 417 | 800,56 | 1192,4 | | | |
| 115 | 179,69 | 303,62 | 227 | 1599,83 | 1031,48 | 326 | 857,23 | 362,46 | 418 | 1088,84 | 1967,46 | | | |
| 117 | 192,12 | 324,63 | 236 | 619,61 | 237,07 | 328 | 377,72 | 156,8 | 419 | 1559,36 | 1006,41 | | | |
| 118 | 480,66 | 608,18 | 238 | 409,42 | 56,43 | 329 | 387,05 | 238 | 420 | 153,22 | 251,93 | | | |
| 126 | 1277,47 | 764,74 | 241 | 226,47 | 151,59 | 331 | 343,69 | 198,75 | 421 | 686,69 | 467,84 | | | |
| 130 | 126,13 | 352,84 | 242 | 5691,93 | 4175,12 | 333 | 8726,36 | 12695,31 | 422 | 400,49 | 817,36 | | | |
| 140 | 188,41 | 276,45 | 243 | 436,57 | 189,83 | 335 | 798,51 | 415,43 | 423 | 878,93 | 1580,15 | | | |
| 145 | 353,26 | 183,44 | 244 | 415,21 | 170,6 | 336 | 205,47 | 121,63 | 424 | 443,24 | 641,55 | | | |
| 148 | 118,89 | 351,18 | 245 | 260,16 | 141,51 | 337 | 221,04 | 258,72 | 427 | 230,97 | 122,78 | | | |
| 149 | 1146,83 | 1740,05 | 246 | 200,04 | 124,78 | 339 | 97,95 | 181,81 | 428 | 1576,37 | 2794,73 | | | |
| 151 | 5196,35 | 10498,07 | 247 | 96,98 | 88,51 | 342 | 432,51 | 329,69 | 429 | 2145,42 | 3482,39 | | | |
| 152 | 433,72 | 272,96 | 250 | 477,46 | 72,76 | 343 | 1621,95 | 2575,03 | 431 | 252,94 | 190,7 | | | |
| 153 | 9705,5 | 20785,64 | 251 | 330,58 | 245,38 | 344 | 614,12 | 309,71 | 432 | 151,34 | 269,09 | | | |
| 154 | 1117,92 | 807,4 | 252 | 141,41 | 134,05 | 346 | 441,7 | 631,08 | 434 | 773,88 | 1103,05 | | | |
| 155 | 2216,16 | 4792,75 | 253 | 608,95 | 239,5 | 348 | 194,86 | 126,88 | 437 | 788,23 | 1328,66 | | | |
| 156 | 1782,53 | 2810,21 | 254 | 242,32 | 315,01 | 349 | 1762,38 | 2743,26 | 438 | 1217,92 | 2565,32 | | | |
| 157 | 13518,15 | 5970,9 | 256 | 510,44 | 342,62 | 350 | 7179,32 | 341,71 | 439 | 200,2 | 346,97 | | | |
| 158 | 931,68 | 1996,73 | 257 | 283 | 176,66 | 351 | 18950,35 | 33275,83 | 441 | 1957,36 | 2749,89 | | | |
| 159 | 4992,07 | 13360,51 | 258 | 103,68 | 45,1 | 352 | 3233,23 | 4832,9 | 443 | 465,87 | 412,54 | | | |
| 160 | 3493,09 | 2426,25 | 262 | 1237,68 | 1993,54 | 353 | 280,53 | 178,61 | 444 | 272,39 | 110,58 | | | |
| 161 | 4055,4 | 3023,97 | 263 | 437,43 | 284,59 | 354 | 205,51 | 600,85 | 445 | 245,78 | 464,12 | | | |
| 162 | 8398,05 | 5176,62 | 265 | 682 | 451,29 | 355 | 489,54 | 660,69 | 447 | 281,31 | 244,14 | | | |
| 163 | 2593,57 | 863,5 | 268 | 374,36 | 200,42 | 356 | 212,11 | 381,49 | 449 | 76,2 | 220,69 | | | |
| 164 | 2038,97 | 484,44 | 269 | 675,02 | 421,74 | 357 | 945,98 | 610,28 | 450 | 750,55 | 378,52 | | | |
| 165 | 357,58 | 558,57 | 273 | 1603,17 | 1707,78 | 358 | 294,04 | 233,81 | 453 | 139,55 | 344,87 | | | |
| 166 | 1145,96 | 2233,02 | 274 | 253,2 | 166,84 | 359 | 3084,9 | 1071,44 | 454 | 369,2 | 851,23 | | | |
| 169 | 352,85 | 86,39 | 276 | 371,26 | 44,14 | 360 | 2474,74 | 3145,77 | 455 | 85,25 | 132,39 | | | |
| 170 | 105,05 | 224,3 | 278 | 1001,65 | 684,34 | 362 | 138,38 | 296,81 | 456 | 106,58 | 159,23 | | | |
| 457 | 212,57 | 278,24 | 548 | 296,88 | 126,04 | 633 | 3020,85 | 1120,66 | 722 | 447,59 | 2446,05 | 805 | 634,21 | 454,65 |
| 458 | 1516,3 | 2420,43 | 549 | 550,37 | 207,91 | 634 | 329,03 | 786,49 | 723 | 13940,67 | 18395,94 | 809 | 679,06 | 295,85 |
| 459 | 1588,11 | 2722,27 | 550 | 228,52 | 764,16 | 637 | 364,89 | 197,87 | 725 | 187,68 | 334,29 | 811 | 618,77 | 311,34 |
| 461 | 287,43 | 487,39 | 551 | 512,6 | 290,72 | 639 | 275,25 | 140,43 | 726 | 397,81 | 591,38 | 812 | 1119,36 | 388,74 |
| 462 | 1790,75 | 1405,34 | 552 | 297,73 | 252,04 | 640 | 115,65 | 66,25 | 727 | 637,1 | 1016,73 | 813 | 145,77 | 66,69 |
| 463 | 432,2 | 310,42 | 553 | 107,65 | 63,73 | 641 | 498,77 | 420,21 | 728 | 65,58 | 109,04 | 814 | 864,31 | 859,71 |
| 465 | 2273,09 | 2831,77 | 554 | 925,07 | 559,35 | 642 | 414,68 | 199,62 | 730 | 671,33 | 1689,36 | 815 | 638,46 | 331,57 |
| 467 | 300,32 | 170,89 | 555 | 226,74 | 131,69 | 645 | 779,48 | 334,7 | 731 | 110,46 | 373,29 | 816 | 1143,81 | 743,86 |
| 468 | 277,28 | 315,21 | 556 | 3423,44 | 211,72 | 647 | 5050,96 | 3358,31 | 732 | 3464,04 | 934,95 | 817 | 1557,62 | 753,52 |
| 470 | 1748,3 | 366,74 | 558 | 281,61 | 274,93 | 648 | 175,97 | 294,91 | 733 | 556,67 | 570,92 | 818 | 1908,62 | 1216,08 |
| 472 | 234,2 | 528,19 | 559 | 145,28 | 351,22 | 649 | 699,27 | 200,78 | 734 | 215,66 | 410,25 | 819 | 4979,79 | 844,55 |
| 473 | 1735,46 | 2592,95 | 562 | 648,04 | 1019,16 | 651 | 2586,25 | 1291,14 | 735 | 622,74 | 321,95 | 820 | 217,82 | 155,57 |
| 475 | 345,34 | 113,11 | 563 | 670,49 | 202,92 | 652 | 231,51 | 143,66 | 737 | 130 | 213,43 | 823 | 336,34 | 689,88 |
| 476 | 250,43 | 117,03 | 565 | 758,68 | 484,55 | 653 | 915,81 | 1935,03 | 738 | 190,04 | 327,64 | 824 | 3252,92 | 1470,8 |
| 478 | 719,07 | 390,57 | 568 | 779,64 | 127,81 | 654 | 5467,07 | 3725,55 | 739 | 293,28 | 366,38 | 826 | 5471,02 | 2618,94 |
| 479 | 849,79 | 507,64 | 569 | 836,94 | 671,47 | 656 | 819,99 | 386,71 | 743 | 185,63 | 332,04 | 827 | 2845,32 | 1552,78 |
| 480 | 568,31 | 240,49 | 570 | 258,18 | 74,14 | 659 | 141,99 | 980,59 | 744 | 819,18 | 507,03 | 828 | 1179,08 | 575,57 |
| 484 | 488,92 | 192,21 | 571 | 944,81 | 306,21 | 660 | 3289,37 | 329,08 | 745 | 602,42 | 780,22 | 829 | 1035,75 | 505,99 |
| 486 | 714,64 | 479,07 | 572 | 526,09 | 215,39 | 661 | 1919,62 | 2716,97 | 747 | 347,49 | 178,71 | 830 | 2085,83 | 9898,6 |
| 487 | 779,62 | 407,72 | 573 | 265,12 | 561,18 | 662 | 250,8 | 623,37 | 748 | 410,63 | 345,85 | 832 | 1003,08 | 2095,65 |
| 489 | 862,45 | 149,83 | 574 | 318,19 | 165,07 | 663 | 81,8 | 189,73 | 749 | 137,85 | 486,26 | 833 | 6617,56 | 1583,61 |
| 490 | 69,53 | 147,08 | 575 | 1531,87 | 1267 | 664 | 122,34 | 1413,92 | 751 | 1303,86 | 1883,97 | 834 | 670,25 | 313,23 |
| 491 | 254,43 | 282,04 | 577 | 18967,25 | 840,95 | 665 | 737,4 | 407,33 | 754 | 822,75 | 398,24 | 835 | 1014,66 | 600,48 |
| 492 | 402,39 | 507,47 | 578 | 210,08 | 510,51 | 666 | 426,8 | 770,9 | 755 | 6713,94 | 2358,14 | | | |
| 493 | 941,73 | 210,4 | 579 | 509,75 | 219,86 | 667 | 299,35 | 416,59 | 756 | 166,21 | 284,44 | | | |
| 495 | 257,49 | 450,84 | 584 | 181,19 | 618,79 | 668 | 188,12 | 612,28 | 758 | 200,24 | 250,67 | | | |
| 498 | 257,46 | 431,46 | 585 | 759,09 | 217,1 | 669 | 387,84 | 768,38 | 760 | 1216,67 | 2134,8 | | | |
| 500 | 502,69 | 61,58 | 586 | 4565,75 | 58,99 | 671 | 587,68 | 897,03 | 763 | 399,33 | 320,2 | | | |
| 501 | 1854,99 | 707,59 | 587 | 191,97 | 218,19 | 672 | 368,29 | 117,39 | 764 | 128,33 | 248,54 | | | |
| 502 | 137,49 | 169,93 | 588 | 192,37 | 103,5 | 673 | 21397,52 | 34,43 | 765 | 1514,46 | 4308,89 | | | |
| 503 | 140,4 | 252,44 | 589 | 263 | 405,5 | 674 | 187,19 | 256,29 | 767 | 293,5 | 190,8 | | | |
| 504 | 203,04 | 131,9 | 590 | 3057,26 | 126,77 | 675 | 665,34 | 927,95 | 768 | 283,27 | 788,11 | | | |
| 505 | 84,63 | 139,9 | 592 | 184,87 | 539,84 | 677 | 892,27 | 307,55 | 769 | 1403,56 | 947,92 | | | |
| 506 | 184,34 | 123,01 | 593 | 135,07 | 411,88 | 678 | 989,87 | 1826,69 | 770 | 418,46 | 258,56 | | | |
| 507 | 2296,38 | 1347,32 | 595 | 674,57 | 132,06 | 679 | 1128,17 | 1728,96 | 772 | 1313,89 | 627,71 | | | |
| 509 | 477,71 | 241,64 | 597 | 309,64 | 679,53 | 680 | 851,56 | 1493,41 | 773 | 211,83 | 927,49 | | | |
| 510 | 425,28 | 197,63 | 598 | 1239,18 | 168,71 | 681 | 1136,29 | 621,15 | 774 | 1337,79 | 550,41 | | | |
| 511 | 995,99 | 1860,09 | 603 | 3414,12 | 1369,9 | 683 | 1148,54 | 504,81 | 775 | 244,98 | 490,03 | | | |
| 512 | 1078,39 | 213,78 | 604 | 962,06 | 169,59 | 686 | 1363,26 | 797,98 | 776 | 3574,99 | 7395,44 | | | |
| 513 | 66,83 | 235,62 | 606 | 3896,53 | 8436,04 | 688 | 98,69 | 156,14 | 777 | 164,47 | 292,43 | | | |
| 514 | 358,26 | 78,89 | 607 | 111,01 | 126,02 | 689 | 53,97 | 120,23 | 778 | 387,48 | 356,61 | | | |
| 515 | 4934,49 | 943,15 | 608 | 7801,67 | 886,26 | 690 | 132,93 | 196,27 | 781 | 1038,97 | 153,69 | | | |
| 516 | 510,22 | 154,31 | 610 | 200,22 | 2017,29 | 693 | 843,36 | 485,58 | 782 | 4315,89 | 6177,03 | | | |
| 517 | 790,5 | 344,83 | 611 | 1670,86 | 90,16 | 694 | 2694,55 | 1305,99 | 783 | 916,46 | 441,9 | | | |
| 518 | 862,14 | 120,51 | 612 | 207,94 | 158,12 | 696 | 586,14 | 227,88 | 784 | 1412,71 | 1241,77 | | | |
| 520 | 976,83 | 589,3 | 614 | 152,15 | 342,56 | 698 | 960,67 | 1170,01 | 785 | 634,19 | 106,33 | | | |
| 521 | 1091,43 | 410,04 | 616 | 627,87 | 979,18 | 699 | 218,88 | 140,39 | 786 | 78,13 | 220,29 | | | |
| 524 | 124,24 | 287,18 | 617 | 549,7 | 1001,2 | 700 | 68,12 | 132,51 | 787 | 413,79 | 34,69 | | | |
| 527 | 127,3 | 313,99 | 619 | 183,49 | 457,6 | 701 | 1882,74 | 2710,35 | 789 | 814,07 | 750,37 | | | |
| 528 | 122,54 | 162,9 | 621 | 191,66 | 223,31 | 702 | 709,66 | 988,21 | 790 | 1584 | 4381,9 | | | |
| 529 | 543,24 | 654,44 | 622 | 3449,9 | 6523,87 | 703 | 465,93 | 247,99 | 792 | 92,04 | 51,86 | | | |
| 530 | 87,72 | 136,71 | 623 | 182 | 256,33 | 704 | 452,19 | 552,79 | 793 | 1306,38 | 777,08 | | | |
| 532 | 1066,35 | 725,68 | 624 | 577,49 | 913,29 | 707 | 157,49 | 209,94 | 794 | 125,55 | 184,79 | | | |
| 534 | 1202,07 | 1722,54 | 625 | 62,18 | 168,82 | 708 | 157,54 | 1118,73 | 795 | 825,81 | 1314,25 | | | |
| 537 | 258,42 | 252,33 | 626 | 244,08 | 497,04 | 711 | 132,71 | 286,79 | 796 | 456,95 | 140,52 | | | |
| 538 | 305,95 | 133,39 | 627 | 2052,37 | 858,44 | 714 | 543,08 | 281,17 | 797 | 461,61 | 182,5 | | | |
| 541 | 294,45 | 368,78 | 628 | 2563,18 | 274,04 | 715 | 644,42 | 1159 | 799 | 437,29 | 564,91 | | | |
| 543 | 139,85 | 522,74 | 630 | 103,25 | 158,48 | 717 | 612,86 | 987,76 | 800 | 448,25 | 288,48 | | | |
| 546 | 410,38 | 179,19 | 632 | 317,32 | 93 | 720 | 116,14 | 91,86 | 801 | 165,93 | 148,57 | | | |

Die erfindungsgemäßen Marker lassen sich auch zur Differentialdiagnostik der einzelnen Stadien pTa bis pT4 einsetzen.

Das Individuum, von dem die Probe stammt, in der die An- oder Abwesenheit oder die Amplitude eines oder mehrerer Polypeptidmarker bestimmt wird, kann jedes Individuum sein, das an Blasenkrebs leiden kann, z.B. ein Tier oder ein Mensch. Vorzugsweise handelt es sich bei dem Individuum um ein Säugetier, am meisten bevorzugt handelt es sich um einen Menschen.

In einer bevorzugten Ausführungsform der Erfindung wird nicht nur ein Polypeptidmarker, sondern eine Kombination von Markern verwendet, um Blasenkrebs zu diagnostizieren. Dabei wird durch ihre An- oder Abwesenheit und/oder die Höhe der Amplitude auf das Vorliegen von Blasenkrebs geschlossen. Durch Vergleich einer Mehrzahl von Polypeptidmarkern kann die Verfälschung des Gesamtergebnisses durch einzelne individuelle Abweichungen von der typischen Anwesenheitswahrscheinlichkeit im Kranken oder Kontrollindividuum reduziert oder vermieden werden.

Bei der Probe, in der die An- oder Abwesenheit oder die Amplitude des oder der erfindungsgemäßen Polypeptidmarker gemessen werden, kann es sich um jede Probe handeln, die aus dem Körper des Individuums gewonnen wird. Bei der Probe handelt es sich um eine Probe, die über eine Polypeptidzusammensetzung verfügt, die geeignet ist, Aussagen über den Zustand des Individuums (Blasenkrebs oder nicht) zu treffen. Beispielsweise kann es sich um Blut, Urin, eine Gelenkflüssigkeit, eine Gewebeflüssigkeit, ein Körpersekret, Schweiß, Liquor, Lymphe, Darm-, Magen-, Pankreassaft, Galle, Tränenflüssigkeit, eine Gewebeprobe, Sperma, Vaginalflüssigkeit oder eine Stuhlprobe handeln. Vorzugsweise handelt es sich um eine Flüssigprobe.

In einer bevorzugten Ausführungsform handelt es sich bei der Probe um eine Urinprobe oder eine Blutprobe, wobei es sich bei einer Blutprobe um eine (Blut)serum- oder (Blut)plasmaprobe handeln kann.

Urinproben können wie im Stand der Technik bekannt genommen werden. Vorzugsweise wird im Zusammenhang mit der vorliegenden Erfindung eine Mittelstrahlurinprobe verwendet. Die Urinprobe kann z.B. mittels eines Katheters oder auch mit Hilfe eines Urinierungsapparates, wie in WO 01/74275 beschrieben, entnommen werden.

Blutproben können durch im Stand der Technik bekannte Verfahren beispielsweise aus einer Vene, Arterie oder Kapillare entnommen werden. Für gewöhnlich wird eine Blutprobe erhalten, indem einem Individuum venöses Blut mittels einer Spritze z.B. aus dem Arm entnommen wird. Der Begriff Blutprobe bezieht auch Proben ein, die aus Blut durch weitere, aus dem Stand der Technik bekannte Aufreinigungs- und Trennverfahren gewonnen wurden, wie z.B. Blutplasma oder Blutserum.

Die An- oder Abwesenheit oder Amplitude eines Polypeptidmarkers in der Probe kann durch jedes im Stand der Technik bekannte Verfahren, das zur Messung von Polypeptidmarkern geeignet ist, bestimmt werden. Dem Fachmann sind solche Verfahren bekannt. Grundsätzlich kann die An- oder Abwesenheit oder Amplitude eines Polypeptidmarkers durch direkte Verfahren, wie z.B. Massenspektrometrie, oder indirekte Verfahren, wie z.B. mittels Liganden, bestimmt werden.

Falls erforderlich oder wünschenswert kann die Probe des Individuums, z.B. die Urin- oder Blutprobe, vor der Messung der An- oder Abwesenheit oder Amplitude des oder der Polypeptidmarker durch jedes geeignete Mittel vorbehandelt und z.B. aufgereinigt oder aufgetrennt werden. Die Behandlung kann z.B. eine Aufreinigung, Trennung, Verdünnung oder Konzentrierung umfassen. Die Verfahren können beispielsweise eine Zentrifugation, Filtration, Ultrafiltration, Dialyse, eine Fällung oder chromatographische Verfahren wie Affinitätstrennung oder Trennung mittels Ionenaustauscher-chromatographie, oder eine elektrophoretische Trennung sein. Besondere Beispiele hierfür sind Gelelektrophorese, zweidimensionale Polyacrylamidgelelektrophorese (2D-PAGE), Kapillarelektrophorese, Metallaffinitätschromatographie, immobilisierte Metallaffinitätschromatographie (IMAC), Affinitätschromatographie auf der Basis von Lektinen, Flüssigchromatographie, Hochleistungsflüssigchromatographie (HPLC), Normal- und Umkehrphasen-HPLC, Kationenaustauscherchromatographie und selektive Bindung an Oberflächen. Alle diese Verfahren sind dem Fachmann gut bekannt und der Fachmann wird das Verfahren in Abhängigkeit von der verwendeten Probe und dem Verfahren zur Bestimmung der An- oder Abwesenheit oder Amplitude des oder der Polypeptidmarker auswählen können.

In einer Ausführungsform der Erfindung wird die Probe vor ihrer Messung mittels Kapillarelektrophorese aufgetrennt, mittels Ultrazentrifugation gereinigt und/oder mittels Ultrafiltration in Fraktionen, die Polypeptidmarker bestimmter molekularer Größe enthalten, aufgetrennt.

Vorzugsweise wird ein massenspektrometrisches Verfahren verwendet, um die An- oder Abwesenheit oder Amplitude eines Polypeptidmarkers zu bestimmen, wobei diesem Verfahren eine Aufreinigung oder Auftrennung der Probe vorgeschaltet werden kann. Die massenspektrometrische Analyse besitzt gegenüber den derzeit gängigen Verfahren den Vorteil, dass die Konzentration vieler (>500) Polypeptide einer Probe mittels einer einzigen Analyse bestimmt werden kann. Jeder Typ eines Massenspektrometers kann verwendet werden. Mit der Massenspektrometrie ist es möglich, routinemäßig 10 fmol eines Polypeptidmarkers, also 0,1 ng eines 10 kDa Proteins mit einer Messgenauigkeit von ca. ±0,01% aus einem komplexen Gemisch zu vermessen. Bei Massenspektrometern ist eine Ionen-bildende Einheit mit einem geeigneten Analysegerät gekoppelt. Zum Beispiel werden meistens Elektrospray-Ionisations (ESI) Interfaces verwendet, um Ionen aus Flüssigproben zu vermessen, wohingegen die Matrix-assisted-laser-desorption/ionisation (MALDI) Technik verwendet wird, um Ionen aus mit einer Matrix kristallisierten Probe zu vermessen. Zur Analyse der entstandenen Ionen können z.B. Quadrupole, Ionenfallen, FT-ICR oder Time-of-flight (TOF) Analysatoren verwendet werden.

Bei der Elektrosprayionisation (ESI) werden die in Lösung vorliegenden Moleküle u.a. unter dem Einfluss von Hochspannung (z.B. 1-8 kV) versprüht, wobei sich geladenen Tröpfchen bilden, die durch Verdampfen des Lösungsmittels kleiner werden. Schließlich kommt es durch sog. Coulomb-Explosionen zur Bildung freier Ionen, die dann analysiert und detektiert werden können.

Bei der Analyse der Ionen mittels TOF wird eine bestimmte Beschleunigungsspannung angelegt, die den Ionen eine gleich große kinetische Energie verleiht. Dann wird sehr genau die Zeit gemessen, die die jeweiligen Ionen benötigen, um eine Driftstrecke durch das Flugrohr zurückzulegen. Da bei gleicher kinetische Energie die Geschwindigkeit der Ionen von Ihrer Masse abhängt, kann diese somit bestimmt werden. TOF-Analysatoren haben eine sehr hohe Scan-Geschwindigkeit und erreichen eine hohe Auflösung.

Bevorzugte Verfahren zur Bestimmung der An- oder Abwesenheit oder Amplitude von Polypeptidmarkern schließen Gasphasenionenspektrometrie, wie Laserdesorptions /Ionisations-Massenspektrometrie, MALDI-TOF-MS, SELDI-TOF-MS (Surface enhanced laser desorption ionisation), LC-MS (Liquid chromatography- mass spectrometry), 2D-PAGE-MS und Kapillarelektrophorese-Massenspektrometrie (CE-MS) ein. Alle genannten Verfahren sind dem Fachmann bekannt.

Ein besonders bevorzugtes Verfahren ist CE-MS, in welchem die Kapillarelektrophorese mit Massenspektrometrie gekoppelt wird. Dieses Verfahren ist ausführlich z.B. in der deutschen Patentanmeldung DE 10021737, bei Kaiser et al. (J. Chromatogr. A, 2003, Bd. 1013:157-171, sowie Electrophoresis, 2004, 25:2044-2055) und bei Wittke et al. (J. Chromatogr. A, 2003, 1013:173-181) beschrieben. Die CE-MS Technik erlaubt, das Vorhandensein einiger Hunderter Polypeptidmarker einer Probe gleichzeitig in kurzer Zeit, einem geringen Volumen und hoher Sensitivität zu bestimmen. Nachdem eine Probe vermessen wurde, wird ein Muster der gemessenen Polypeptidmarker hergestellt. Dieses kann mit Referenzmustern von kranken bzw. gesunden Individuen verglichen werden. In den meisten Fällen ist es ausreichend, eine begrenzte Anzahl von Polypeptidmarkern für die Diagnose von Blasenkrebs und die Differentialdiagnose zwischen verschiedenen Stadien des Blasenkrebses zu verwenden, beispielsweise mindestens 6, 8, 10, 20, 50 oder 100 Marker.

Weiter bevorzugt ist ein CE-MS Verfahren, das CE online an ein ESI-TOF-MS gekoppelt, einschließt.

Für CE-MS ist die Verwendung von flüchtigen Lösungsmitteln bevorzugt, außerdem arbeitet man am besten unter im Wesentlichen salzfreien Bedingungen. Beispiele geeigneter Lösungsmittel umfassen Acetonitril, Methanol und ähnliche. Die Lösungsmittel können mit Wasser verdünnt und mit einer schwachen Säure (z.B. 0,1% bis 1% Ameisensäure) versetzt sein, um den Analyten, vorzugsweise die Polypeptide, zu protonieren.

Mit der Kapillarelektrophorese ist es möglich, Moleküle nach ihrer Ladung und Größe zu trennen. Neutrale Teilchen wandern beim Anlegen eines Stromes mit der Geschwindigkeit des elektroosmotischen Flusses, Kationen werden zur Kathode beschleunigt und Anionen verzögert. Der Vorteil von Kapillaren in der Elektrophorese besteht im günstigen Verhältnis von Oberfläche zu Volumen, was einen guten Abtransport der beim Stromfluss entstehenden Jouleschen Wärme ermöglicht. Dies wiederum erlaubt das Anlegen hoher Spannungen (üblicherweise bis 30 kV) und damit eine hohe Trennleistung und kurze Analysezeiten.

Bei der Kapillarelektrophorese werden normalerweise Quarzglaskapillaren mit Innendurchmessern von typischerweise 50 bis 75 µm eingesetzt. Die verwendeten Längen betragen 30-100 cm. Darüber hinaus bestehen die Kapillaren in der Regel aus kunststoffumhüllten Quarzglas. Die Kapillaren können sowohl unbehandelt sein, d.h. auf der Innenseite ihre hydrophilen Gruppen zeigen, als auch auf der Innenseite beschichtet sein. Eine hydrophobe Beschichtung (Coating: ein Verfahren, welches z.B. die negative polarisierte Oberfläche des Quarzes maskiert) kann verwendet werden, um die Auflösung zu verbessern. Zusätzlich zur Spannung kann auch ein Druck angelegt werden, der typischerweise im Bereich von 0-1 psi liegt. Der Druck kann dabei auch erst während der Trennung angelegt oder währenddessen verändert werden.

In einem bevorzugten Verfahren zur Messung von Polypeptidmarkern werden die Marker der Probe mittels Kapillarelektrophorese getrennt, anschließend direkt ionisiert und online in ein daran gekoppeltes Massenspektrometer zur Detektion überführt.

In dem erfindungsgemäßen Verfahren können vorteilhafter Weise mehrere Polypeptidmarker zur Diagnose von Blasenkrebs verwendet werden. Insbesondere können mindestens drei Polypeptidmarker verwendet werden, beispielsweise die Marker 1, 2 und 3; 1, 2 und 4; usw.

Mehr bevorzugt ist die Verwendung von mindestens 4, 5, oder 6 Markern.

Noch mehr bevorzugt ist die Verwendung von mindestens 15 Markern, beispielsweise die Marker 1 bis 15.

Am meisten bevorzugt ist die Verwendung aller in den Tabellen 1 bzw. 4 aufgeführten Marker.

Um die Wahrscheinlichkeit für das Vorliegen von Blasenkrebs bei Verwendung mehrerer Marker zu bestimmen, können dem Fachmann bekannte statistische Verfahren verwendet werden. Beispielsweise kann das von Weissinger et al. (Kidney Int., 2004, 65:2426-2434) beschriebene Random-Forests-Verfahren unter Verwendung eines Computerprogramms wie z.B. S-Plus oder die in derselben Veröffentlichung beschriebenen support-vector-machines verwendet werden.

### Beispiel:

### 1. Probenvorbereitung:

Zur Detektion der Polypeptidmarker für Blasenkrebs wurde Urin verwendet. Urin wurde von gesunden Spendern (Vergleichsgruppe) sowie Patienten, die an Blasenkrebs leiden, abgenommen.

Für die nachfolgende CE-MS Messung mussten die auch in Urin von Patienten in höherer Konzentration vorkommenden Proteine wie Albumin und Immunoglobuline durch Ultrafiltration abgetrennt werden. Dazu wurden 700 µl Urin entnommen und mit 700 µl Filtrationspuffer (2M Harnstoff, 10 mM Ammoniak, 0,02% SDS) versetzt. Diese 1,4 ml Probenvolumen wurden ultrafiltriert (20 kDa, Sartorius, Göttingen, Deutschland). Die UF wurde bei 3000 U/min in einer Zentrifuge durchgeführt bis 1,1 ml Ultrafiltrat erhalten wurden.

Die erhaltenen 1,1 ml Filtrat wurden dann auf eine PD 10 Säule aufgetragen (Amersham Bioscience, Uppsala, Schweden) und mit 2,5 ml 0,01% NH₄OH eluiert und lyophillisiert. Zur CE-MS Messung wurden die Polypeptide dann mit 20 µl Wasser (HPLC-Reinheit, Merck) resuspendiert.

### 2. CE-MS Messung:

Die CE-MS Messungen wurden mit einem Kapillarelektrophoresesystem von Beckman Coulter (P/ACE MDQ System; Beckman Coulter Inc, Fullerton, USA) und einem ESI-TOF Massenspektrometer von Bruker (micro-TOF MS, Bruker Daltonik, Bremen, D) durchgeführt.

Die CE Kapillaren wurden von Beckman Coulter bezogen, sie hatten einen ID/OD von 50/360 µm und eine Länge von 90 cm. Die mobile Phase für die CE Trennung bestand aus 20% Acetonitril und 0,25% M Ameisensäure in Wasser. Für den "Sheath-Flow" am MS wurde 30% Isopropanol mit 0,5% Ameisensäure verwendet, hier mit einer Flussrate von 2 µl/min. Die Kopplung von CE und MS wurde durch ein CE-ESI-MS Sprayer Kit (Agilent Technologies, Waldbronn, DE) realisiert.

Um die Probe zu injizieren, wurde 1 bis max. 6 psi Druck angelegt, die Dauer der Injektion betrug 99 Sekunden. Mit diesen Parametern wurden ca. 150 bis 900 nl der Probe in die Kapillare injiziert, dieses entspricht ca. 10% bis 50% des Kapillarvolumens. Um die Probe in der Kapillare aufzukonzentrieren wurde eine "Stacking"-Technik verwendet. Dabei wird vor der Probeninjektion für 7 Sek. (bei 1 psi) eine 1M NH₃ Lösung injiziert, nach der Probeninjektion für 5 Sek. eine 2M Ameisensäurelösung. Nach Anlegen der Trennspannung (30 kV) werden die Analyten zwischen diesen Lösungen automatisch aufkonzentriert.

Die folgende CE-Trennung wurde mit einer Druckmethode durchgeführt: 40 Minuten mit 0 psi, dann für 2 min 0,1 psi, für 2 min 0,2 psi, für 2 min 0,3 psi, für 2 min 0,4 psi, abschließend 32 min bei 0,5 psi. Die Gesamtdauer eines Trennlaufes betrug damit 80 Minuten.

Um auf der Seite des MS eine möglichst gute Signalintensität zu erhalten, wurde das "Nebulizer Gas" auf den niedrigsten möglichen Wert eingestellt. Die angelegte Spannung zur Erzeugung des Elektrosprays betrug 3700 - 4100 V. Die übrigen Einstellungen am Massenspektrometer wurden gemäß Anweisung des Herstellers für Peptiddetektion optimiert. Die Spektren wurden über einen Massenbereich von m/z 400 bis m/z 3000 aufgenommen und alle 3 Sek. akkumuliert.

### 3. Standards für die CE-Messunq

Zur Kontrolle und Kalibrierung der CE-Messung wurden die folgenden Proteine bzw. Polypeptide eingesetzt, welche unter den gewählten Bedingungen durch die unten aufgeführten CE-Migrationszeiten charakterisiert sind:

| Protein/Polypeptid | Migrationszeit |
|---|---|
| Aprotinin, (SIGMA, Taufkirchen, DE; Kat.Nr. A1153) | 9,2 min |
| Ribonuclease, SIGMA, Taufkirchen, DE; Kat.Nr.; R4875 | 10,9 min |
| Lysozym, SIGMA, Taufkirchen, DE; Kat.Nr.; L7651 | 8,9 min |
| "REV", Sequenz: REVQSKIGYGRQIIS | 15,6 min |
| "ELM", Sequenz: ELMTGELPYSHINNRDQIIFMVGR | 23,4 min |
| "KINCON", Sequenz: TGSLPYSHIGSRDQIIFMVGR | 20,0 min |
| "GIVLY" Sequenz: GIVLYELMTGELPYSHIN | 36,8 min |

Die Proteine/Polypeptide werden jeweils in einer Konzentration von 10 pmol/µl in Wasser eingesetzt. "REV", "ELM", "KINCON" und "GIVLY" stellen synthetische Peptide dar.

Die Molekularmassen der Peptide sowie die in der MS sichtbaren m/z Verhältnisse der einzelnen Ladungszustände sind in der folgenden Tabelle angegeben:

| **H (mono)** | 1,0079 | 1,0079 | 1,0079 | 1,0079 | 1,0079 | 1,0079 | 1,0079 |
|---|---|---|---|---|---|---|---|
| m/z | **Aprotinin** | **Ribonuclease** | **Lysozym** | **REV** | **KINCON** | **ELM** | **GIVLY** |
| | Mono Mass | Mono Mass | Mono Mass | Mono Mass | Mono Mass | Mono Mass | Mono Mass |
| 0 | 6513,09 | 13681,32 | 14303,88 | 1732,96 | 2333,19 | 2832,41 | 2048,03 |
| 1 | 6514,0979 | 13682,328 | 14304,888 | 1733,9679 | 2334,1979 | 2833,4179 | 2049,0379 |
| 2 | 3257,5529 | 6841,6679 | 7152,9479 | 867,4879 | 1167,6029 | 1417,2129 | 1025,0229 |
| 3 | 2172,0379 | 4561,4479 | 4768,9679 | 578,6612 | 778,7379 | 945,1446 | 683,6846 |
| 4 | 1629,2804 | 3421,3379 | 3576,9779 | 434,2479 | 584,3054 | 709,1104 | 513,0154 |
| 5 | 1303,6259 | 2737,2719 | 2861,7839 | 347,5999 | 467,6459 | 567,4899 | 410,6139 |
| 6 | 1086,5229 | 2281,2279 | 2384,9879 | 289,8346 | 389,8729 | 473,0762 | 342,3462 |
| 7 | 931,4494 | 1955,4822 | 2044,4193 | 248,5736 | 334,3208 | 405,6379 | 293,5836 |
| 8 | 815,1442 | 1711,1729 | 1788,9929 | 217,6279 | 292,6567 | 355,0592 | 257,0117 |
| 9 | 724,6846 | 1521,1546 | 1590,3279 | 193,559 | 260,2512 | 315,7201 | 228,5668 |
| 10 | 652,3169 | 1369,1399 | 1431,3959 | 174,3039 | 234,3269 | 284,2489 | 205,8109 |
| 11 | 593,107 | 1244,7643 | 1301,3606 | 158,5497 | 213,1161 | 258,4997 | 187,1924 |
| 12 | 543,7654 | 1141,1179 | 1192,9979 | 145,4212 | 195,4404 | 237,0421 | 171,6771 |
| 13 | 502,0148 | 1053,4171 | 1101,3063 | 134,3125 | 180,4841 | 218,8856 | 158,5486 |

## Patentansprüche

1. Verfahren zur Diagnose von Blasenkrebs (BC) und/oder zur Bestimmung eines Tumorstadiums von Blasenkrebs umfassend den Schritt der Bestimmung einer An- oder Abwesenheit oder Amplitude von mindestens sechs Polypeptidmarkern in einer Probe, wobei die Polypeptidmarker ausgewählt sind aus den Markern 1 bis 836
und die Polypeptidmarker durch folgende Werte für die Molekularmassen und die Migrationszeit (CE-Zeit) charakterisiert sind:
| **Nummer** | **Masse** | **CE-Zeit** | **Nummer** | **Masse** | **CE-Zeit** | **Nummer** | **Masse** | **CE-Zeit** | **Nummer** | **Masse** | **CE-Zeit** | **Nummer** | **Masse** | **CE-Zeit** |
|---|---|---|---|---|---|---|---|---|---|---|---|---|---|---|
| 1 | 4933,97 | 17,2 | 60 | 1844,58 | 31,4 | 119 | 1935,96 | 13,6 | 178 | 1950,87 | 33,9 | 237 | 1073,36 | 35,39 |
| 2 | 4712,25 | 14,3 | 61 | 2130,97 | 29,6 | 120 | 2062,81 | 22,9 | 179 | 1649,76 | 17,9 | 238 | 1078,69 | 19,67 |
| 3 | 1071,54 | 16,3 | 62 | 2359,35 | 31,0 | 121 | 13372,32 | 25,9 | 180 | 1312,55 | 26,3 | 239 | 1082,55 | 24,37 |
| 4 | 3273,42 | 17,7 | 63 | 3343,58 | 28,5 | 122 | 1495,68 | 18,9 | 181 | 1438,47 | 35,2 | 240 | 1083,56 | 21,65 |
| 5 | 1130,37 | 33,3 | 64 | 3501,62 | 28,4 | 123 | 1513,49 | 35,4 | 182 | 3248,51 | 26,8 | 241 | 1096,41 | 35,91 |
| 6 | 1180,53 | 33,7 | 65 | 5675,31 | 19,0 | 124 | 1900,90 | 28,6 | 183 | 2742,26 | 25,3 | 242 | 1096,53 | 26,12 |
| 7 | 980,36 | 34,0 | 66 | 1312,64 | 17,6 | 125 | 3108,94 | 33,8 | 184 | 981,60 | 20,7 | 243 | 1098,56 | 21,42 |
| 8 | 11721,36 | 13,4 | 67 | 1867,68 | 30,4 | 126 | 1860,87 | 16,2 | 185 | 1679,97 | 19,3 | 244 | 1099,56 | 21,56 |
| 9 | 2077,12 | 16,8 | 68 | 2186,85 | 31,9 | 127 | 2194,68 | 14,3 | 186 | 801,43 | 22,7 | 245 | 1100,59 | 21,62 |
| 10 | 840,42 | 19,3 | 69 | 2414,51 | 33,6 | 128 | 3956,81 | 21,3 | 187 | 816,41 | 21,05 | 246 | 1101,54 | 22,21 |
| 11 | 2115,04 | 22,8 | 70 | 2739,24 | 24,8 | 129 | 1496,70 | 26,5 | 188 | 818,48 | 21,95 | 247 | 1106,52 | 26,02 |
| 12 | 3385,43 | 21,1 | 71 | 2907,36 | 33,9 | 130 | 3659,17 | 21,3 | 189 | 838,36 | 25,12 | 248 | 1109,68 | 21,06 |
| 13 | 4053,18 | 13,6 | 72 | 3031,42 | 34,0 | 131 | 13169,24 | 25,6 | 190 | 838,44 | 35,05 | 249 | 1114,54 | 25,52 |
| 14 | 4240,13 | 14,2 | 73 | 4345,89 | 30,6 | 132 | 1608,75 | 27,5 | 191 | 840,47 | 19,68 | 250 | 1114,57 | 21,27 |
| 15 | 1387,04 | 9,5 | 74 | 6211,67 | 14,6 | 133 | 1707,73 | 11,1 | 192 | 852,47 | 20,42 | 251 | 1124,58 | 21,04 |
| 16 | 2597,47 | 18,6 | 75 | 1080,52 | 22,9 | 134 | 1592,72 | 17,3 | 193 | 868,45 | 23,35 | 252 | 1125,55 | 24,9 |
| 17 | 2920,21 | 15,9 | 76 | 1142,80 | 10,9 | 135 | 2029,80 | 15,0 | 194 | 871,48 | 21,11 | 253 | 1125,58 | 21,77 |
| 18 | 4509,09 | 25,1 | 77 | 1321,74 | 10,6 | 136 | 13007,18 | 25,1 | 195 | 875,51 | 21,83 | 254 | 1126,52 | 21,2 |
| 19 | 8763,45 | 12,1 | 78 | 1750,77 | 19,5 | 137 | 2117,77 | 29,9 | 196 | 876,43 | 35,13 | 255 | 1126,57 | 25,61 |
| 20 | 1535,72 | 26,3 | 79 | 1989,90 | 29,3 | 138 | 2168,87 | 31,0 | 197 | 883,45 | 23,27 | 256 | 1134,63 | 23,68 |
| 21 | 911,28 | 31,9 | 80 | 2946,38 | 32,8 | 139 | 1916,86 | 31,3 | 198 | 884,36 | 24,91 | 257 | 1135,53 | 27,51 |
| 22 | 1191,54 | 34,5 | 81 | 4942,34 | 21,4 | 140 | 2802,78 | 34,7 | 199 | 892,32 | 35,22 | 258 | 1136,59 | 20,27 |
| 23 | 3759,90 | 12,8 | 82 | 1668,76 | 17,5 | 141 | 1111,77 | 10,8 | 200 | 906,34 | 34,93 | 259 | 1137,65 | 24,08 |
| 24 | 2973,43 | 20,0 | 83 | 1688,71 | 14,5 | 142 | 1806,84 | 18,5 | 201 | 906,51 | 21,89 | 260 | 1138,64 | 19,55 |
| 25 | 3325,67 | 15,4 | 84 | 1731,79 | 17,7 | 143 | 1594,71 | 26,1 | 202 | 912,55 | 20,04 | 261 | 1139,54 | 21,01 |
| 26 | 10042,81 | 12,2 | 85 | 1822,74 | 27,4 | 144 | 1778,76 | 27,0 | 203 | 914,49 | 23,75 | 262 | 1140,53 | 21,14 |
| 27 | 1380,67 | 19,3 | 86 | 3870,83 | 30,4 | 145 | 2582,95 | 19,0 | 204 | 915,28 | 35,24 | 263 | 1141,57 | 25,2 |
| 28 | 3502,70 | 12,5 | 87 | 4275,48 | 20,6 | 146 | 3605,63 | 21,22 | 205 | 931,51 | 19,99 | 264 | 1142,61 | 21,8 |
| 29 | 3632,63 | 15,9 | 88 | 1025,48 | 21,7 | 147 | 3765,31 | 42,77 | 206 | 935,49 | 23,76 | 265 | 1143,56 | 36,95 |
| 30 | 8559,22 | 13,2 | 89 | 1353,67 | 22,1 | 148 | 4067,63 | 31,22 | 207 | 936,49 | 21,43 | 266 | 1144,43 | 35,57 |
| 31 | 4960,86 | 15,6 | 90 | 1466,67 | 25,2 | 149 | 10640,4 | 19,68 | 208 | 939,51 | 23,79 | 267 | 1144,6 | 26,25 |
| 32 | 858,41 | 18,9 | 91 | 1588,73 | 26,7 | 150 | 2577,35 | 20,9 | 209 | 941,46 | 24,85 | 268 | 1150,61 | 22,42 |
| 33 | 847,40 | 20,0 | 92 | 1878,72 | 27,6 | 151 | 3292,55 | 37,5 | 210 | 944,55 | 21,35 | 269 | 1153,38 | 35,68 |
| 34 | 980,51 | 18,7 | 93 | 2933,31 | 24,1 | 152 | 2058,90 | 18,8 | 211 | 945,46 | 25,7 | 270 | 1153,57 | 26,34 |
| 35 | 1128,52 | 22,4 | 94 | 3338,45 | 19,3 | 153 | 3001,36 | 33,2 | 212 | 947,5 | 24,75 | 271 | 1154,57 | 36,72 |
| 36 | 1407,68 | 35,6 | 95 | 6055,63 | 15,6 | 154 | 2157,04 | 17,0 | 213 | 951,55 | 20,32 | 272 | 1155,55 | 20,77 |
| 37 | 2093,92 | 31,0 | 96 | 2266,00 | 17,2 | 155 | 2854,36 | 32,6 | 214 | 956,48 | 20,38 | 273 | 1157,58 | 37,41 |
| 38 | 2421,04 | 32,5 | 97 | 2939,05 | 31,1 | 156 | 1653,90 | 26,9 | 215 | 958,51 | 20,76 | 274 | 1159,63 | 20,75 |
| 39 | 1250,66 | 14,8 | 98 | 3021,39 | 18,9 | 157 | 2658,32 | 13,1 | 216 | 963,54 | 21,65 | 275 | 1159,64 | 26,09 |
| 40 | 3242,20 | 18,0 | 99 | 1299,60 | 17,6 | 158 | 3657,65 | 38,7 | 217 | 973,28 | 35,58 | 276 | 1160,6 | 26,14 |
| 41 | 1040,50 | 21,2 | 100 | 1405,68 | 14,3 | 159 | 1754,92 | 27,8 | 218 | 984,49 | 24,43 | 277 | 1162,59 | 20,26 |
| 42 | 3176,40 | 11,9 | 101 | 1526,72 | 19,3 | 160 | 1467,82 | 19,8 | 219 | 988,55 | 35,81 | 278 | 1165,55 | 37,15 |
| 43 | 2778,55 | 32,5 | 102 | 1630,78 | 15,4 | 161 | 1580,91 | 20,1 | 220 | 988,55 | 21,83 | 279 | 1166,62 | 22,8 |
| 44 | 1422,58 | 35,8 | 103 | 1865,83 | 30,0 | 162 | 2563,89 | 17,6 | 221 | 994,49 | 25,17 | 280 | 1168,58 | 26,23 |
| 45 | 3524,42 | 28,9 | 104 | 2687,24 | 25,4 | 163 | 2355,15 | 17,9 | 222 | 995,44 | 25,15 | 281 | 1169,61 | 23,85 |
| 46 | 3831,74 | 24,8 | 105 | 2756,27 | 32,8 | 164 | 3891,40 | 20,2 | 223 | 996,51 | 24,63 | 282 | 1170,64 | 21,61 |
| 47 | 2488,79 | 32,2 | 106 | 1200,84 | 10,7 | 165 | 1561,49 | 35,6 | 224 | 1009,47 | 20,96 | 283 | 1171,62 | 23,34 |
| 48 | 1099,51 | 24,9 | 107 | 1391,81 | 10,7 | 166 | 1576,63 | 23,1 | 225 | 1012,53 | 35,08 | 284 | 1173,58 | 37,51 |
| 49 | 1936,82 | 29,2 | 108 | 1487,68 | 26,1 | 167 | 1911,07 | 20,8 | 226 | 1016,31 | 35,67 | 285 | 1179,65 | 20,06 |
| 50 | 1265,61 | 23,4 | 109 | 1919,87 | 23,3 | 168 | 3265,47 | 19,8 | 227 | 1016,49 | 25,81 | 286 | 1181,62 | 26,06 |
| 51 | 3081,61 | 26,4 | 110 | 2063,48 | 14,1 | 169 | 2344,91 | 32,3 | 228 | 1027,55 | 24,6 | 287 | 1190,64 | 22,02 |
| 52 | 3266,48 | 26,5 | 111 | 2753,47 | 34,2 | 170 | 2743,98 | 32,8 | 229 | 1031,5 | 25,66 | 288 | 1196,37 | 36,14 |
| 53 | 3821,95 | 20,4 | 112 | 2864,16 | 28,5 | 171 | 3401,75 | 18,1 | 230 | 1037,57 | 21,98 | 289 | 1196,59 | 21,07 |
| 54 | 2407,10 | 24,0 | 113 | 3858,65 | 21,9 | 172 | 3425,87 | 26,1 | 231 | 1038,61 | 20,84 | 290 | 1198,6 | 26,05 |
| 55 | 1596,75 | 19,0 | 114 | 4015,96 | 24,4 | 173 | 5510,18 | 23,1 | 232 | 1042,54 | 25,13 | 291 | 1199,63 | 21,92 |
| 56 | 10341,75 | 18,1 | 115 | 1324,60 | 16,3 | 174 | 2356,70 | 33,5 | 233 | 1053,53 | 25,08 | 292 | 1200,59 | 24,21 |
| 57 | 4306,66 | 28,9 | 116 | 1793,60 | 15,4 | 175 | 3256,25 | 29,3 | 234 | 1056,57 | 25,49 | 293 | 1201,59 | 24,85 |
| 58 | 900,43 | 19,8 | 117 | 2215,79 | 30,7 | 176 | 12716,23 | 23,1 | 235 | 1066,52 | 25,96 | 294 | 1204,65 | 21,93 |
| 59 | 1645,74 | 15,9 | 118 | 1934,82 | 14,2 | 177 | 1050,49 | 23,3 | 236 | 1068,56 | 21,69 | 295 | 1209,58 | 26,26 |
| 296 | 1210,45 | 36,58 | 355 | 1396,68 | 28,05 | 414 | 1632,79 | 38,03 | 473 | 1863,95 | 44,05 | 532 | 2138,03 | 21,78 |
| 297 | 1210,61 | 20,92 | 356 | 1413,62 | 25,78 | 415 | 1633,83 | 19,58 | 474 | 1880,97 | 44,03 | 533 | 2146 | 52,45 |
| 298 | 1213,62 | 27,75 | 357 | 1417,69 | 20,01 | 416 | 1635,83 | 30,38 | 475 | 1885,96 | 21,26 | 534 | 2188,03 | 39,71 |
| 299 | 1215,49 | 27,61 | 358 | 1421,58 | 37,51 | 417 | 1636,79 | 20,1 | 476 | 1889,94 | 33,12 | 535 | 2192,08 | 22,16 |
| 300 | 1216,59 | 24,27 | 359 | 1423,68 | 21,47 | 418 | 1636,81 | 30,33 | 477 | 1891,65 | 54,28 | 536 | 2195,49 | 33,36 |
| 301 | 1217,58 | 35,91 | 360 | 1425,65 | 22,34 | 419 | 1638,8 | 20,24 | 478 | 1892,95 | 22,4 | 537 | 2197,94 | 34,23 |
| 302 | 1218,42 | 36,88 | 361 | 1436,79 | 22,82 | 420 | 1640,75 | 28,28 | 479 | 1893,06 | 24,64 | 538 | 2199,08 | 22,31 |
| 303 | 1219,62 | 26,92 | 362 | 1438,72 | 21,98 | 421 | 1658,68 | 21,55 | 480 | 1899,93 | 21,64 | 539 | 2207,16 | 43,61 |
| 304 | 1220,63 | 22,11 | 363 | 1439,72 | 29,7 | 422 | 1658,79 | 30,04 | 481 | 1911,05 | 30,74 | 540 | 2208 | 32,37 |
| 305 | 1221,63 | 26,86 | 364 | 1440,72 | 39,19 | 423 | 1660,76 | 37,7 | 482 | 1912,74 | 32,7 | 541 | 2208,97 | 31,75 |
| 306 | 1223,6 | 27,47 | 365 | 1440,75 | 21,63 | 424 | 1664,82 | 29,87 | 483 | 1916,85 | 20,35 | 542 | 2211,11 | 20,28 |
| 307 | 1226,58 | 21 | 366 | 1442,68 | 27,76 | 425 | 1668,87 | 40,47 | 484 | 1926,91 | 30,99 | 543 | 2212,06 | 33,53 |
| 308 | 1226,63 | 23,47 | 367 | 1444,49 | 36,69 | 426 | 1673,82 | 29,4 | 485 | 1927,99 | 19,53 | 544 | 2219,56 | 33,59 |
| 309 | 1230,6 | 21,74 | 368 | 1448,83 | 20,53 | 427 | 1674,83 | 20,71 | 486 | 1932,98 | 21,65 | 545 | 2227,05 | 33,42 |
| 310 | 1234,42 | 36,16 | 369 | 1449,7 | 21,84 | 428 | 1680,82 | 30,02 | 487 | 1933,95 | 21,63 | 546 | 2233,1 | 22,49 |
| 311 | 1234,61 | 27,41 | 370 | 1451,71 | 29,19 | 429 | 1684,74 | 31,42 | 488 | 1936,11 | 20,51 | 547 | 2237,06 | 27,1 |
| 312 | 1238,55 | 36,74 | 371 | 1452,72 | 23,55 | 430 | 1684,81 | 39,43 | 489 | 1938,96 | 21,33 | 548 | 2244,17 | 19,48 |
| 313 | 1238,63 | 21,86 | 372 | 1456,75 | 22,48 | 431 | 1689,86 | 27,62 | 490 | 1942,77 | 36,19 | 549 | 2248,75 | 22,52 |
| 314 | 1239,52 | 34,91 | 373 | 1457,68 | 22 | 432 | 1693,83 | 20,48 | 491 | 1945,07 | 33,71 | 550 | 2251,77 | 33,11 |
| 315 | 1241,6 | 26,68 | 374 | 1458,69 | 28,03 | 433 | 1693,84 | 23,48 | 492 | 1946,95 | 31,75 | 551 | 2264,12 | 22,67 |
| 316 | 1245,6 | 21,63 | 375 | 1460,71 | 20,31 | 434 | 1697,81 | 30,86 | 493 | 1949,97 | 21,66 | 552 | 2269,29 | 38,14 |
| 317 | 1247,58 | 22,02 | 376 | 1473,7 | 22,3 | 435 | 1698,67 | 37,53 | 494 | 1952,42 | 25,65 | 553 | 2279,57 | 23,77 |
| 318 | 1250,52 | 35,35 | 377 | 1474,75 | 19,94 | 436 | 1705,78 | 46,77 | 495 | 1955,04 | 25,29 | 554 | 2282,13 | 22,29 |
| 319 | 1257,63 | 26,86 | 378 | 1483,72 | 22,59 | 437 | 1708,85 | 31,08 | 496 | 1955,94 | 28,14 | 555 | 2283,12 | 26,51 |
| 320 | 1260,61 | 21,84 | 379 | 1487,71 | 29,58 | 438 | 1725,66 | 38,3 | 497 | 1969,43 | 32,31 | 556 | 2292,21 | 21,29 |
| 321 | 1263,6 | 22,72 | 380 | 1491,8 | 39,86 | 439 | 1728,84 | 36,74 | 498 | 1976,96 | 32,36 | 557 | 2302,38 | 26,16 |
| 322 | 1267,64 | 21,55 | 381 | 1493,74 | 22,11 | 440 | 1729,6 | 53,46 | 499 | 1977,99 | 32,21 | 558 | 2305,7 | 34,8 |
| 323 | 1281,64 | 27,16 | 382 | 1507,8 | 39,98 | 441 | 1737,85 | 31,04 | 500 | 1989,02 | 24,75 | 559 | 2310,15 | 41,24 |
| 324 | 1284,62 | 28,22 | 383 | 1508,77 | 23,17 | 442 | 1746,95 | 19,75 | 501 | 1992,02 | 22,05 | 560 | 2315,22 | 19,45 |
| 325 | 1284,69 | 21,88 | 384 | 1509,59 | 37,07 | 443 | 1749,88 | 30,61 | 502 | 1993,02 | 27,07 | 561 | 2318,29 | 26,28 |
| 326 | 1287,65 | 21,85 | 385 | 1510,72 | 28,39 | 444 | 1761,91 | 21,61 | 503 | 1993,96 | 32,2 | 562 | 2319,19 | 33,87 |
| 327 | 1296,65 | 19,37 | 386 | 1510,75 | 20,11 | 445 | 1764,86 | 29,97 | 504 | 1997,99 | 25,13 | 563 | 2320,79 | 22,48 |
| 328 | 1300,65 | 23,07 | 387 | 1522,78 | 22,81 | 446 | 1767,07 | 24,12 | 505 | 2004,02 | 24,63 | 564 | 2322,17 | 22,28 |
| 329 | 1304,66 | 21,85 | 388 | 1523,89 | 29,75 | 447 | 1768,9 | 20,78 | 506 | 2006,93 | 33 | 565 | 2323,14 | 22,39 |
| 330 | 1309,74 | 21,97 | 389 | 1524,72 | 20,02 | 448 | 1769,78 | 28,25 | 507 | 2008,03 | 22,13 | 566 | 2328,06 | 20,83 |
| 331 | 1310,64 | 27,14 | 390 | 1535,75 | 22,03 | 449 | 1773,87 | 34,89 | 508 | 2021,06 | 21,98 | 567 | 2332,08 | 50,2 |
| 332 | 1321,63 | 37,98 | 391 | 1540,81 | 39,97 | 450 | 1782,91 | 25,93 | 509 | 2022,97 | 33,39 | 568 | 2335,75 | 26,61 |
| 333 | 1321,65 | 28,4 | 392 | 1549,76 | 39,5 | 451 | 1783,86 | 39,94 | 510 | 2023,99 | 21,47 | 569 | 2339,08 | 34,01 |
| 334 | 1328,64 | 26,87 | 393 | 1552,57 | 37,35 | 452 | 1784,88 | 40,04 | 511 | 2026,1 | 25,66 | 570 | 2339,17 | 22,63 |
| 335 | 1338,66 | 23,99 | 394 | 1556,8 | 40,03 | 453 | 1786,66 | 38,34 | 512 | 2031 | 21,8 | 571 | 2343,49 | 20,08 |
| 336 | 1340,65 | 28,33 | 395 | 1560,69 | 22,08 | 454 | 1788,89 | 30,26 | 513 | 2035,07 | 40,19 | 572 | 2361,19 | 20,78 |
| 337 | 1349,48 | 36,47 | 396 | 1562,76 | 22,48 | 455 | 1794,84 | 30,49 | 514 | 2040,02 | 32,87 | 573 | 2367,48 | 34,22 |
| 338 | 1349,77 | 20,22 | 397 | 1563,77 | 29,56 | 456 | 1796,85 | 30,86 | 515 | 2042,14 | 25,21 | 574 | 2371,18 | 22,77 |
| 339 | 1350,65 | 21,56 | 398 | 1564,78 | 28,53 | 457 | 1798,79 | 37,33 | 516 | 2057,04 | 24,85 | 575 | 2377,19 | 20,79 |
| 340 | 1352,62 | 29,68 | 399 | 1567,77 | 20,2 | 458 | 1807,91 | 20,7 | 517 | 2065 | 24,48 | 576 | 2380,16 | 36,49 |
| 341 | 1352,83 | 24,47 | 400 | 1576,74 | 46,02 | 459 | 1813,78 | 31,79 | 518 | 2076,61 | 32,44 | 577 | 2389,33 | 22,4 |
| 342 | 1353,65 | 21,48 | 401 | 1577,75 | 40,06 | 460 | 1818,9 | 31,1 | 519 | 2078,05 | 22,47 | 578 | 2392,8 | 35,28 |
| 343 | 1358,44 | 36,46 | 402 | 1579,75 | 20,05 | 461 | 1821,88 | 30,43 | 520 | 2081,03 | 20,17 | 579 | 2394 | 23,93 |
| 344 | 1361,69 | 21,95 | 403 | 1579,78 | 29,83 | 462 | 1823,99 | 24,61 | 521 | 2087,96 | 19,46 | 580 | 2405,6 | 22,19 |
| 345 | 1362,72 | 22,13 | 404 | 1609,81 | 30,25 | 463 | 1825,87 | 31,81 | 522 | 2088,04 | 32,92 | 581 | 2410,16 | 23,45 |
| 346 | 1366,68 | 27,48 | 405 | 1610,91 | 30,12 | 464 | 1829,03 | 21,24 | 523 | 2088,95 | 23,68 | 582 | 2414,26 | 19,58 |
| 347 | 1368,68 | 21,7 | 406 | 1611,83 | 40,12 | 465 | 1834,9 | 31,15 | 524 | 2090,04 | 39,48 | 583 | 2430,19 | 28,28 |
| 348 | 1375,67 | 23,28 | 407 | 1611,89 | 23,27 | 466 | 1835,92 | 24,04 | 525 | 2095,53 | 24,83 | 584 | 2430,72 | 35,39 |
| 349 | 1378,67 | 28,85 | 408 | 1613,89 | 23,98 | 467 | 1838,89 | 27,03 | 526 | 2100,12 | 19,6 | 585 | 2436,15 | 22,8 |
| 350 | 1383,69 | 39,02 | 409 | 1619,78 | 22,6 | 468 | 1846,93 | 32,08 | 527 | 2110 | 24,11 | 586 | 2437,22 | 22,86 |
| 351 | 1390,5 | 37,05 | 410 | 1620,74 | 29,58 | 469 | 1852,96 | 25,14 | 528 | 2114,07 | 31,61 | 587 | 2445,8 | 28,15 |
| 352 | 1392,68 | 21,75 | 411 | 1620,79 | 22,71 | 470 | 1858,91 | 24,21 | 529 | 2118,04 | 27,72 | 588 | 2451,59 | 24,02 |
| 353 | 1395,71 | 22,59 | 412 | 1624,61 | 37,73 | 471 | 1859,9 | 24,38 | 530 | 2129,06 | 26,97 | 589 | 2471,25 | 34,76 |
| 354 | 1396,5 | 37,63 | 413 | 1631,78 | 47,02 | 472 | 1860,42 | 33,82 | 531 | 2134,04 | 27,78 | 590 | 2478,22 | 22,45 |
| 591 | 2479,15 | 48,79 | 650 | 2989,4 | 24,42 | 709 | 3333,28 | 19,42 | 768 | 4083,75 | 31,32 | 827 | 8853,9 | 21,1 |
| 592 | 2485,16 | 34,39 | 651 | 2999,23 | 22,22 | 710 | 3333,8 | 23,85 | 769 | 4097,98 | 24,6 | 828 | 8917,52 | 22,55 |
| 593 | 2501,21 | 34,37 | 652 | 3002,47 | 23,95 | 711 | 3334,27 | 31 | 770 | 4113,77 | 24,46 | 829 | 10199,94 | 21,11 |
| 594 | 2502,73 | 22,84 | 653 | 3011,46 | 29,72 | 712 | 3336,48 | 39,43 | 771 | 4122,1 | 24,06 | 830 | 10753,72 | 19,71 |
| 595 | 2507,21 | 22,78 | 654 | 3013,27 | 22,28 | 713 | 3341,91 | 21,05 | 772 | 4124,05 | 21,49 | 831 | 10770,19 | 19,64 |
| 596 | 2518,4 | 22,8 | 655 | 3017,95 | 49,05 | 714 | 3356,38 | 25,56 | 773 | 4143,81 | 26,55 | 832 | 11041,95 | 21,98 |
| 597 | 2525,21 | 27,72 | 656 | 3023,33 | 24,55 | 715 | 3359,67 | 31,88 | 774 | 4196,47 | 20,96 | 833 | 11738,36 | 19,6 |
| 598 | 2534,56 | 22,81 | 657 | 3025,69 | 29,85 | 716 | 3363,69 | 30,21 | 775 | 4215,52 | 26,95 | 834 | 11967,98 | 20,48 |
| 599 | 2548,38 | 35,1 | 658 | 3035 | 42,23 | 717 | 3375,69 | 31,9 | 776 | 4217,98 | 26 | 835 | 14111,34 | 21,97 |
| 600 | 2551,32 | 34,75 | 659 | 3035,17 | 47,18 | 718 | 3386,16 | 22,7 | 777 | 4229,11 | 29,06 | 836 | 3266,45 | 22,73 |
| 601 | 2553,52 | 34,27 | 660 | 3038,11 | 22,42 | 719 | 3400,07 | 42,03 | 778 | 4278,77 | 23,34 | | | |
| 602 | 2557,15 | 28,17 | 661 | 3041,47 | 29,95 | 720 | 3416,82 | 36,78 | 779 | 4306 | 25,13 | | | |
| 603 | 2559,25 | 19,44 | 662 | 3044,98 | 34,34 | 721 | 3435,94 | 22,7 | 780 | 4322,14 | 25,18 | | | |
| 604 | 2565,18 | 23,74 | 663 | 3047,64 | 26,68 | 722 | 3457,46 | 35,83 | 781 | 4339 | 25,4 | | | |
| 605 | 2567,28 | 28,17 | 664 | 3056,94 | 45,28 | 723 | 3457,73 | 31,51 | 782 | 4352,98 | 20,17 | | | |
| 606 | 2570,28 | 42,66 | 665 | 3064,36 | 20,55 | 724 | 3472,36 | 21,75 | 783 | 4363,14 | 23,05 | | | |
| 607 | 2574,08 | 32,92 | 666 | 3076,33 | 19,64 | 725 | 3510,65 | 40,23 | 784 | 4369,07 | 20,21 | | | |
| 608 | 2580,19 | 23 | 667 | 3080,99 | 29,92 | 726 | 3521,07 | 30,74 | 785 | 4378,88 | 23,14 | | | |
| 609 | 2587,39 | 21,17 | 668 | 3091,41 | 28,33 | 727 | 3530,63 | 26,11 | 786 | 4381,08 | 34,13 | | | |
| 610 | 2599,26 | 28,18 | 669 | 3092,45 | 30,97 | 728 | 3531,46 | 30,16 | 787 | 4395,06 | 23,09 | | | |
| 611 | 2607,6 | 34,4 | 670 | 3092,51 | 36,23 | 729 | 3546,82 | 26,7 | 788 | 4415,72 | 22,53 | | | |
| 612 | 2616 | 28,34 | 671 | 3098,49 | 30,07 | 730 | 3559,71 | 24,89 | 789 | 4426,27 | 20,09 | | | |
| 613 | 2627,08 | 23,44 | 672 | 3112,42 | 22,44 | 731 | 3571,61 | 31,03 | 790 | 4436,04 | 26,25 | | | |
| 614 | 2632,18 | 24,96 | 673 | 3113,81 | 20,39 | 732 | 3582,7 | 19,47 | 791 | 4441,22 | 22,43 | | | |
| 615 | 2639,41 | 20,98 | 674 | 3114,66 | 30,3 | 733 | 3583,5 | 41,45 | 792 | 4483,33 | 25,4 | | | |
| 616 | 2668,46 | 42,02 | 675 | 3121,31 | 30,23 | 734 | 3590,72 | 29,01 | 793 | 4491,91 | 26,23 | | | |
| 617 | 2682,22 | 22,49 | 676 | 3129,86 | 35,93 | 735 | 3596,51 | 21,57 | 794 | 4538,61 | 26,35 | | | |
| 618 | 2702,1 | 38,01 | 677 | 3136,55 | 24,5 | 736 | 3603,92 | 20,21 | 795 | 4548,93 | 26,45 | | | |
| 619 | 2710,58 | 35,15 | 678 | 3139,44 | 29,48 | 737 | 3633,02 | 33,15 | 796 | 4550,95 | 23,31 | | | |
| 620 | 2723,92 | 23,81 | 679 | 3143,51 | 32,87 | 738 | 3633,71 | 27,02 | 797 | 4567,27 | 23,29 | | | |
| 621 | 2726,32 | 28,87 | 680 | 3145,49 | 38,8 | 739 | 3635,47 | 31,87 | 798 | 4583,7 | 35,76 | | | |
| 622 | 2726,37 | 42,94 | 681 | 3152,23 | 24,54 | 740 | 3669,71 | 24,13 | 799 | 4619,43 | 25,07 | | | |
| 623 | 2733,84 | 34,18 | 682 | 3158,6 | 29,69 | 741 | 3686,04 | 22,21 | 800 | 4637,97 | 25,75 | | | |
| 624 | 2742,36 | 42,12 | 683 | 3168,38 | 24,69 | 742 | 3696,88 | 26,94 | 801 | 4805,62 | 26,47 | | | |
| 625 | 2748,87 | 36,35 | 684 | 3178,26 | 30,25 | 743 | 3702,45 | 32,45 | 802 | 4817,33 | 23,86 | | | |
| 626 | 2754,36 | 29,64 | 685 | 3184,67 | 24,91 | 744 | 3703,85 | 22,51 | 803 | 4833,29 | 23,89 | | | |
| 627 | 2761,4 | 21,49 | 686 | 3193,27 | 22,63 | 745 | 3706,68 | 22 | 804 | 4849,07 | 23,86 | | | |
| 628 | 2777,61 | 21,54 | 687 | 3195,2 | 20,64 | 746 | 3710,86 | 19,6 | 805 | 4863,23 | 26,67 | | | |
| 629 | 2796,02 | 21 | 688 | 3202,2 | 30,52 | 747 | 3737,71 | 37,15 | 806 | 4864,81 | 23,88 | | | |
| 630 | 2810,46 | 36,66 | 689 | 3204,32 | 35,31 | 748 | 3738,58 | 24,74 | 807 | 4933,46 | 20,58 | | | |
| 631 | 2816,5 | 28,41 | 690 | 3219,43 | 35,05 | 749 | 3765,51 | 20,21 | 808 | 4976,46 | 20,62 | | | |
| 632 | 2821,19 | 23,73 | 691 | 3223,42 | 24,74 | 750 | 3774,84 | 22,9 | 809 | 5027,18 | 26,53 | | | |
| 633 | 2837,91 | 23,99 | 692 | 3240,47 | 25,2 | 751 | 3775,25 | 27 | 810 | 5039,24 | 25,56 | | | |
| 634 | 2839,42 | 24,17 | 693 | 3249,16 | 22,61 | 752 | 3775,9 | 25,28 | 811 | 5043,12 | 26,54 | | | |
| 635 | 2853,32 | 23,8 | 694 | 3258,51 | 22,89 | 753 | 3790,98 | 32,88 | 812 | 5090,45 | 20,1 | | | |
| 636 | 2861,32 | 50,73 | 695 | 3260,4 | 41,55 | 754 | 3816,45 | 21,94 | 813 | 5160,13 | 21,74 | | | |
| 637 | 2887,42 | 35,66 | 696 | 3261,35 | 22,17 | 755 | 3839,91 | 19,7 | 814 | 5229,59 | 25,1 | | | |
| 638 | 2889,19 | 24,09 | 697 | 3265,33 | 36,02 | 756 | 3864,66 | 33,71 | 815 | 5276,59 | 20,04 | | | |
| 639 | 2889,65 | 28,73 | 698 | 3271,39 | 30,75 | 757 | 3906,75 | 24,13 | 816 | 5495,5 | 19,52 | | | |
| 640 | 2903,72 | 35,61 | 699 | 3276,25 | 33,59 | 758 | 3935,56 | 34,14 | 817 | 5574,44 | 23,22 | | | |
| 641 | 2911 | 21,68 | 700 | 3282,79 | 30,09 | 759 | 3948,83 | 45,72 | 818 | 6186,07 | 24,91 | | | |
| 642 | 2914,57 | 24,28 | 701 | 3287,39 | 30,84 | 760 | 3968,68 | 21,11 | 819 | 6237,1 | 21,07 | | | |
| 643 | 2917,41 | 29,03 | 702 | 3290,39 | 24,1 | 761 | 4002,73 | 20,66 | 820 | 6491,14 | 19,44 | | | |
| 644 | 2924,58 | 22,97 | 703 | 3295,51 | 25,38 | 762 | 4008,95 | 23,57 | 821 | 6650,84 | 25,52 | | | |
| 645 | 2926,2 | 22,16 | 704 | 3298,48 | 36,06 | 763 | 4025,43 | 33,23 | 822 | 7907,23 | 19,59 | | | |
| 646 | 2932,36 | 34,11 | 705 | 3310,26 | 36,5 | 764 | 4042,93 | 33,13 | 823 | 7958,72 | 34,38 | | | |
| 647 | 2942,4 | 22,22 | 706 | 3314,41 | 20,12 | 765 | 4044,98 | 26,39 | 824 | 8289,54 | 19,54 | | | |
| 648 | 2977,61 | 29,14 | 707 | 3318,68 | 30,98 | 766 | 4069,65 | 25,2 | 825 | 8445,41 | 19,52 | | | |
| 649 | 2982,9 | 22,24 | 708 | 3325,9 | 54,11 | 767 | 4081,58 | 24,53 | 826 | 8837,64 | 21,06 | | | |

2. Verfahren nach Anspruch 1, **dadurch gekennzeichnet, dass** eine Auswertung der bestimmten An- oder Abwesenheit der Marker 1-149 anhand folgender Referenzwerte erfolgt:
| Nummer | Vorkommen Gruppe BC | Vorkommen Kontrolle | Nummer | Vorkommen Gruppe BC | Vorkommen Kontrolle | Nummer | Vorkommen Gruppe BC | Vorkommen Kontrolle |
|---|---|---|---|---|---|---|---|---|
| 1 | 0,86 | 0,13 | 60 | 0 | 0,5 | 119 | 0,29 | 0,88 |
| 2 | 0,71 | 0 | 61 | 0 | 0,5 | 120 | 0,29 | 0,88 |
| 3 | 0,73 | 0,03 | 62 | 0 | 0,5 | 121 | 0,36 | 0,95 |
| 4 | 0,71 | 0,07 | 63 | 0 | 0,5 | 122 | 0 | 0,6 |
| 5 | 0,64 | 0,03 | 64 | 0 | 0,5 | 123 | 0 | 0,6 |
| 6 | 0,64 | 0,03 | 65 | 0 | 0,5 | 124 | 0 | 0,6 |
| 7 | 0,71 | 0,13 | 66 | 0,29 | 0,8 | 125 | 0 | 0,6 |
| 8 | 0,71 | 0,13 | 67 | 0,14 | 0,65 | 126 | 0,29 | 0,9 |
| 9 | 0,73 | 0,16 | 68 | 0,14 | 0,65 | 127 | 0,14 | 0,75 |
| 10 | 0,67 | 0,09 | 69 | 0,14 | 0,65 | 128 | 0,29 | 0,9 |
| 11 | 0,57 | 0 | 70 | 0,29 | 0,8 | 129 | 0,18 | 0,8 |
| 12 | 0,92 | 0,35 | 71 | 0,29 | 0,8 | 130 | 0,13 | 0,75 |
| 13 | 0,57 | 0 | 72 | 0,14 | 0,65 | 131 | 0,31 | 0,93 |
| 14 | 0,57 | 0 | 73 | 0,14 | 0,65 | 132 | 0,14 | 0,78 |
| 15 | 0,57 | 0,03 | 74 | 0,29 | 0,8 | 133 | 0 | 0,63 |
| 16 | 0,57 | 0,03 | 75 | 0,18 | 0,7 | 134 | 0,28 | 0,93 |
| 17 | 0,57 | 0,03 | 76 | 0,33 | 0,85 | 135 | 0 | 0,65 |
| 18 | 0,57 | 0,03 | 77 | 0,08 | 0,6 | 136 | 0,18 | 0,82 |
| 19 | 0,57 | 0,03 | 78 | 0,43 | 0,95 | 137 | 0,14 | 0,8 |
| 20 | 0,67 | 0,13 | 79 | 0,43 | 0,95 | 138 | 0,14 | 0,8 |
| 21 | 0,78 | 0,25 | 80 | 0,31 | 0,82 | 139 | 0 | 0,68 |
| 22 | 0,69 | 0,16 | 81 | 0,2 | 0,72 | 140 | 0 | 0,68 |
| 23 | 0,78 | 0,25 | 82 | 0,14 | 0,68 | 141 | 0 | 0,7 |
| 24 | 0,69 | 0,17 | 83 | 0,14 | 0,68 | 142 | 0 | 0,7 |
| 25 | 0,56 | 0,03 | 84 | 0,14 | 0,68 | 143 | 0 | 0,75 |
| 26 | 0,57 | 0,05 | 85 | 0,14 | 0,68 | 144 | 0 | 0,75 |
| 27 | 0,67 | 0,16 | 86 | 0 | 0,53 | 145 | 0,14 | 0,95 |
| 28 | 0,6 | 0,09 | 87 | 0 | 0,53 | 146 | 0,15 | 0,55 |
| 29 | 0,51 | 0 | 88 | 0,29 | 0,82 | 147 | 0,15 | 0,6 |
| 30 | 0,86 | 0,35 | 89 | 0,29 | 0,82 | 148 | 0,15 | 0,57 |
| 31 | 0,91 | 0,41 | 90 | 0,29 | 0,82 | 149 | 0,15 | 0,56 |
| 32 | 0,71 | 0,22 | 91 | 0,29 | 0,82 | | | |
| 33 | 0,51 | 0,03 | 92 | 0,29 | 0,82 | | | |
| 34 | 0,58 | 0,09 | 93 | 0,29 | 0,82 | | | |
| 35 | 0,89 | 0,41 | 94 | 0,29 | 0,82 | | | |
| 36 | 0,51 | 0,03 | 95 | 0,26 | 0,8 | | | |
| 37 | 0,73 | 0,25 | 96 | 0,43 | 0,97 | | | |
| 38 | 0,51 | 0,03 | 97 | 0,43 | 0,97 | | | |
| 39 | 0,62 | 0,16 | 98 | 0,43 | 0,97 | | | |
| 40 | 0,69 | 0,22 | 99 | 0,29 | 0,85 | | | |
| 41 | 0,58 | 0,13 | 100 | 0,14 | 0,7 | | | |
| 42 | 0,58 | 0,13 | 101 | 0,29 | 0,85 | | | |
| 43 | 0,73 | 0,38 | 102 | 0,14 | 0,7 | | | |
| 44 | 0,8 | 0,5 | 103 | 0,29 | 0,85 | | | |
| 45 | 0,42 | 0,72 | 104 | 0,29 | 0,85 | | | |
| 46 | 0,51 | 0,81 | 105 | 0,29 | 0,85 | | | |
| 47 | 0,44 | 0,75 | 106 | 0,26 | 0,82 | | | |
| 48 | 0,62 | 0,95 | 107 | 0 | 0,57 | | | |
| 49 | 0,57 | 0,9 | 108 | 0,26 | 0,82 | | | |
| 50 | 0,57 | 0,93 | 109 | 0 | 0,57 | | | |
| 51 | 0,29 | 0,65 | 110 | 0 | 0,57 | | | |
| 52 | 0,29 | 0,68 | 111 | 0 | 0,57 | | | |
| 53 | 0,42 | 0,81 | 112 | 0 | 0,57 | | | |
| 54 | 0,57 | 0,97 | 113 | 0,43 | 1 | | | |
| 55 | 0,14 | 0,55 | 114 | 0,24 | 0,81 | | | |
| 56 | 0,31 | 0,78 | 115 | 0,14 | 0,72 | | | |
| 57 | 0,33 | 0,81 | 116 | 0,14 | 0,72 | | | |
| 58 | 0 | 0,5 | 117 | 0,14 | 0,72 | | | |
| 59 | 0 | 0,5 | 118 | 0,29 | 0,88 | | | |

3. Verfahren nach Anspruch 1, **dadurch gekennzeichnet, dass** eine Auswertung der Amplitude der Marker 150-185 anhand folgender Referenzwerte erfolgt:
| Nummer | Mittlere Amplitude Gruppe BC [ppm] | Mittlere Amplitude Kontrollen [ppm] | Nummer | Mittlere Amplitude Gruppe BC [ppm] | Mittlere Amplitude Kontrollen [ppm] |
|---|---|---|---|---|---|
| 150 | 512 | 234 | 168 | 2602 | 981 |
| 151 | 4341 | 11833 | 169 | 321 | 745 |
| 152 | 320 | 107 | 170 | 106 | 308 |
| 153 | 4943 | 19582 | 171 | 596 | 1337 |
| 154 | 831 | 404 | 172 | 4128 | 10960 |
| 155 | 1615 | 4183 | 173 | 761 | 1961 |
| 156 | 882 | 2816 | 174 | 200 | 428 |
| 157 | 532 | 573 | 175 | 2150 | 4372 |
| 158 | 922 | 2703 | 176 | 995 | 2026 |
| 159 | 3070 | 7231 | 177 | 215 | 654 |
| 160 | 1863 | 3755 | 178 | 215 | 449 |
| 161 | 2322 | 5187 | 179 | 281 | 574 |
| 162 | 6370 | 1431 | 180 | 691 | 1578 |
| 163 | 1394 | 580 | 181 | 1860 | 4502 |
| 164 | 1039 | 428 | 182 | 1078 | 6650 |
| 165 | 225 | 542 | 183 | 406 | 846 |
| 166 | 769 | 1578 | 184 | 342 | 1360 |
| 167 | 42329 | 112162 | 185 | 1425 | 10166 |

4. Verfahren nach Anspruch 1, **dadurch gekennzeichnet, dass** eine Auswertung der Amplitude der Marker 186-836 anhand folgender Referenzwerte erfolgt
| Nummer | Mittlere Amplitude Gruppe BC | Mittlere Amplitude Kontrollen | Nummer | Mittlere Amplitude Gruppe BC | Mittlere Amplitude Kontrollen | Nummer | Mittlere Amplitude Gruppe BC | Mittlere Amplitude Kontrollen | Nummer | Mittlere Amplitude Gruppe BC | Mittlere Amplitude Kontrollen |
|---|---|---|---|---|---|---|---|---|---|---|---|
| 186 | 119,63 | 80,79 | 245 | 224,51 | 141,51 | 304 | 173,39 | 55,41 | 363 | 430 | 232,16 |
| 187 | 77,4 | 56,61 | 246 | 183,13 | 124,78 | 305 | 148,48 | 73,15 | 364 | 550,02 | 691,79 |
| 188 | 44,83 | 19,49 | 247 | 106,82 | 88,51 | 306 | 156,84 | 179,86 | 365 | 1968,51 | 197,72 |
| 189 | 75,44 | 45,2 | 248 | 3078,26 | 140,75 | 307 | 469,03 | 595,19 | 366 | 350,92 | 381,91 |
| 190 | 118,21 | 235,42 | 249 | 3190,15 | 3178,22 | 308 | 133,11 | 102,43 | 367 | 706,87 | 835,85 |
| 191 | 74,62 | 33,56 | 250 | 368,81 | 72,76 | 309 | 792,94 | 497,12 | 368 | 2384,33 | 175,16 |
| 192 | 374,91 | 44,29 | 251 | 256,09 | 245,38 | 310 | 1537,92 | 2939,46 | 369 | 3854,98 | 5258,01 |
| 193 | 157,08 | 123,78 | 252 | 130,05 | 134,05 | 311 | 1580,14 | 1795,44 | 370 | 14948,23 | 21376,25 |
| 194 | 453,57 | 10,31 | 253 | 448,49 | 239,5 | 312 | 193,48 | 190,55 | 371 | 728,16 | 250,02 |
| 195 | 88 | 45,2 | 254 | 232,48 | 315,01 | 313 | 794,93 | 201,72 | 372 | 552,5 | 144,36 |
| 196 | 90,46 | 720,98 | 255 | 205,39 | 158,14 | 314 | 928,42 | 699,24 | 373 | 210,05 | 246,6 |
| 197 | 111,95 | 85,68 | 256 | 404,85 | 342,62 | 315 | 143,26 | 115,01 | 374 | 347,41 | 249,22 |
| 198 | 410,43 | 469,76 | 257 | 249,07 | 176,66 | 316 | 303,03 | 129,16 | 375 | 207,87 | 315,21 |
| 199 | 427,05 | 319,41 | 258 | 94,05 | 45,1 | 317 | 1342,16 | 932,91 | 376 | 357,91 | 878,46 |
| 200 | 83,12 | 93,94 | 259 | 175,28 | 119,61 | 318 | 1790,3 | 3379,16 | 377 | 274,83 | 200,69 |
| 201 | 102,07 | 57,89 | 260 | 202,45 | 124,75 | 319 | 746,14 | 204,66 | 378 | 852,25 | 552,92 |
| 202 | 238,89 | 119,03 | 261 | 853,59 | 1140,19 | 320 | 706,29 | 396,08 | 379 | 427,78 | 628,41 |
| 203 | 111,31 | 90,37 | 262 | 1327,35 | 1993,54 | 321 | 609,39 | 342,54 | 380 | 344,12 | 480,68 |
| 204 | 195,66 | 197,97 | 263 | 356,82 | 284,59 | 322 | 201,07 | 279,53 | 381 | 10371,52 | 253,26 |
| 205 | 223,5 | 223,37 | 264 | 265,05 | 150,92 | 323 | 440,59 | 252,99 | 382 | 1974,82 | 2562,08 |
| 206 | 149,65 | 149,75 | 265 | 558,75 | 451,29 | 324 | 1500,12 | 166,99 | 383 | 869,39 | 408,55 |
| 207 | 159,86 | 56,01 | 266 | 221,77 | 143,72 | 325 | 231,13 | 194,08 | 384 | 72,76 | 94,63 |
| 208 | 353,83 | 70,68 | 267 | 1128,53 | 72,92 | 326 | 690,08 | 362,46 | 385 | 441,96 | 297,83 |
| 209 | 79,65 | 40,47 | 268 | 331,84 | 200,42 | 327 | 239,27 | 204,76 | 386 | 401,89 | 356,62 |
| 210 | 669,1 | 617,85 | 269 | 505,65 | 421,74 | 328 | 312,93 | 156,8 | 387 | 1259,94 | 1584,04 |
| 211 | 153,61 | 135,8 | 270 | 261,75 | 225,98 | 329 | 325,22 | 238 | 388 | 1178,53 | 2616,54 |
| 212 | 163,45 | 287,56 | 271 | 169,57 | 130,21 | 330 | 8160,78 | 334,35 | 389 | 358,67 | 507,39 |
| 213 | 1141,91 | 27,72 | 272 | 266,62 | 223,68 | 331 | 271 | 198,75 | 390 | 482,45 | 537,75 |
| 214 | 216,39 | 127,03 | 273 | 1362,12 | 1707,78 | 332 | 543,53 | 962,52 | 391 | 1542,53 | 2790,5 |
| 215 | 833,58 | 39,12 | 274 | 294,36 | 402,59 | 333 | 10101,83 | 12695,31 | 392 | 503,57 | 330,15 |
| 216 | 139 | 88,24 | 275 | 221,55 | 166,84 | 334 | 459,58 | 129,16 | 393 | 2430,83 | 3353,77 |
| 217 | 397,21 | 292,45 | 276 | 335,58 | 44,14 | 335 | 648,02 | 415,43 | 394 | 523,9 | 843,44 |
| 218 | 126,14 | 93,76 | 277 | 259,95 | 223,47 | 336 | 163,79 | 121,63 | 395 | 478,99 | 596,56 |
| 219 | 421,86 | 309,18 | 278 | 768,74 | 684,34 | 337 | 205,35 | 258,72 | 396 | 1207,17 7 | 1548,95 |
| 220 | 136,07 | 221,65 | 279 | 251,7 | 214,42 | 338 | 4607,6 | 550,93 | 397 | 248,65 | 483,71 |
| 221 | 152,19 | 120,89 | 280 | 169,01 | 89,8 | 339 | 112,46 | 181,81 | 398 | 166,63 | 140,29 |
| 222 | 219,55 | 145,69 | 281 | 206,44 | 145,14 | 340 | 1168,83 | 221,78 | 399 | 1049,78 | 696,02 |
| 223 | 125,21 | 31,02 | 282 | 3791,23 | 83,4 | 341 | 407,83 | 425,82 | 400 | 3862,62 | 4198,48 |
| 224 | 84,53 | 308,11 | 283 | 147,66 | 91,52 | 342 | 429,65 | 329,69 | 401 | 733,5 | 999,31 |
| 225 | 83,05 | 172,95 | 284 | 257,97 | 474,54 | 343 | 1760,83 | 2575,03 | 402 | 5193,79 | 7951,13 |
| 226 | 1842,39 | 1141,14 | 285 | 2312,84 | 72,28 | 344 | 514,01 | 309,71 | 403 | 2139,76 | 2730,17 |
| 227 | 1324,02 | 1031,48 | 286 | 2355,55 | 150,4 | 345 | 2515,14 | 115,68 | 404 | 916,36 | 816,75 |
| 228 | 350,65 | 89,47 | 287 | 1856,18 | 0 | 346 | 565,18 | 631,08 | 405 | 1684,49 | 1580,08 |
| 229 | 170,43 | 64,39 | 288 | 22744,05 | 27845,62 | 347 | 348,79 | 312,62 | 406 | 237,78 | 343,53 |
| 230 | 190,08 | 78,16 | 289 | 579,96 | 1024,9 | 348 | 184,84 | 126,88 | 407 | 2026,66 | 464,44 |
| 231 | 489,23 | 26,8 | 290 | 181 | 79,4 | 349 | 1992,63 | 2743,26 | 408 | 196,63 | 264,47 |
| 232 | 1996,47 | 72,63 | 291 | 449,65 | 287,08 | 350 | 4620,61 | 341,71 | 409 | 304,49 | 439,85 |
| 233 | 188,24 | 95,18 | 292 | 8039,6 | 6153,2 | 351 | 21183,33 | 33275,83 | 410 | 337,51 | 477,51 |
| 234 | 161,17 | 119,99 | 293 | 400,81 | 327,89 | 352 | 3541,13 | 4832,9 | 411 | 255,82 | 416,12 |
| 235 | 103,9 | 97,75 | 294 | 310,29 | 151 | 353 | 267,96 | 178,61 | 412 | 1221,53 | 1619,64 |
| 236 | 480,36 | 237,07 | 295 | 1031,54 | 860,46 | 354 | 260,72 | 600,85 | 413 | 9331,13 | 14100,68 |
| 237 | 5707,2 | 7336,84 | 296 | 633,16 | 564,48 | 355 | 598,13 | 660,69 | 414 | 197,33 | 300,63 |
| 238 | 379,06 | 56,43 | 297 | 218,71 | 298,42 | 356 | 318,88 | 381,49 | 415 | 212 | 165,73 |
| 239 | 308,98 | 301,08 | 298 | 687,69 | 58,53 | 357 | 784,39 | 610,28 | 416 | 1346,36 | 1460,35 |
| 240 | 462,23 | 78,2 | 299 | 462,1 | 597,81 | 358 | 394,52 | 233,81 | 417 | 820,34 | 1192,4 |
| 241 | 194,93 | 151,59 | 300 | 1581,02 | 1215,02 | 359 | 2575,84 | 1071,44 | 418 | 1548,36 | 1967,46 |
| 242 | 5055,93 | 4175,12 | 301 | 2630,29 | 3679,53 | 360 | 2460,15 | 3145,77 | 419 | 1218,82 | 1006,41 |
| 243 | 366,14 | 189,83 | 302 | 979,29 | 1913,27 | 361 | 1463,89 | 405,51 | 420 | 218,44 | 251,93 |
| 244 | 367,44 | 170,6 | 303 | 243,92 | 96,75 | 362 | 330,7 | 296,81 | 421 | 667,59 | 467,84 |
| 422 | 630,62 | 817,36 | 481 | 16250,01 | 1560,5 | 540 | 116,27 | 110,6 | 599 | 411,12 | 893,15 |
| 423 | 1128,79 | 1580,15 | 482 | 945,07 | 559,31 | 541 | 286,2 | 368,78 | 600 | 297,54 | 266,17 |
| 424 | 584,6 | 641,55 | 483 | 1339,21 | 1514,58 | 542 | 355,35 | 522,74 | 601 | 1122,23 | 1242,63 |
| 425 | 923,66 | 1154,89 | 484 | 358,02 | 192,21 | 543 | 3635,8 | 755,11 | 602 | 140,12 | 176,5 |
| 426 | 245,2 | 209,04 | 485 | 232,09 | 187,49 | 544 | 282,24 | 163,79 | 603 | 2821 | 1369,9 |
| 427 | 208,86 | 122,78 | 486 | 684,31 | 479,07 | 545 | 258,35 | 676,03 | 604 | 894,97 | 169,59 |
| 428 | 2141,81 | 2794,73 | 487 | 728,45 | 407,72 | 546 | 336,29 | 179,19 | 605 | 342,43 | 207,05 |
| 429 | 2673,32 | 3482,39 | 488 | 196,89 | 476,3 | 547 | 411,08 | 341,22 | 606 | 5208,16 | 8436,04 |
| 430 | 194,01 | 349,03 | 489 | 633,98 | 149,83 | 548 | 288,6 | 126,04 | 607 | 109,36 | 126,02 |
| 431 | 244,32 | 190,7 | 490 | 91,61 | 147,08 | 549 | 433,38 | 207,91 | 608 | 5005,47 | 886,26 |
| 432 | 171,13 | 269,09 | 491 | 502,32 | 282,04 | 550 | 468,04 | 764,16 | 609 | 385,8 | 404,35 |
| 433 | 389,06 | 220,01 | 492 | 441,16 | 507,47 | 551 | 514,82 | 290,72 | 610 | 384,35 | 2017,29 |
| 434 | 950,01 | 1103,05 | 493 | 714,01 | 210,4 | 552 | 309,49 | 252,04 | 611 | 1447,69 | 90,16 |
| 435 | 737,26 | 1145,11 | 494 | 349,59 | 153,45 | 553 | 352,9 | 63,73 | 612 | 189,43 | 158,12 |
| 436 | 698,31 | 1006,16 | 495 | 269,05 | 450,84 | 554 | 832,83 | 559,35 | 613 | 886,48 | 201,67 |
| 437 | 1034,44 | 1328,66 | 496 | 403,2 | 515,62 | 555 | 213,46 | 131,69 | 614 | 171,69 | 342,56 |
| 438 | 1549,89 | 2565,32 | 497 | 513,8 | 133,24 | 556 | 2691,55 | 211,72 | 615 | 712,07 | 723,54 |
| 439 | 242,37 | 346,97 | 498 | 295,46 | 431,46 | 557 | 5649,48 | 1095,48 | 616 | 604,67 | 979,18 |
| 440 | 4225,33 | 7163,11 | 499 | 790,03 | 915,09 | 558 | 267,74 | 274,93 | 617 | 645,45 | 1001,2 |
| 441 | 2026,98 | 2749,89 | 500 | 411,21 | 61,58 | 559 | 197,06 | 351,22 | 618 | 367,72 | 477,68 |
| 442 | 1669,33 | 64,04 | 501 | 1600,21 | 707,59 | 560 | 322,71 | 181,52 | 619 | 188,76 | 457,6 |
| 443 | 453,3 | 412,54 | 502 | 185,12 | 169,93 | 561 | 1774,17 | 965,1 | 620 | 612,89 | 94,01 |
| 444 | 284,09 | 110,58 | 503 | 211,53 | 252,44 | 562 | 674,15 | 1019,16 | 621 | 225,78 | 223,31 |
| 445 | 452,02 | 464,12 | 504 | 202,48 | 131,9 | 563 | 514,89 | 202,92 | 622 | 3910,57 | 6523,87 |
| 446 | 10928,25 | 6009,48 | 505 | 106,45 | 139,9 | 564 | 280,12 | 231,27 | 623 | 258,4 | 256,33 |
| 447 | 248,66 | 244,14 | 506 | 172,8 | 123,01 | 565 | 612,61 | 484,55 | 624 | 693,23 | 913,29 |
| 448 | 544,89 | 495,75 | 507 | 2238,19 | 1347,32 | 566 | 305,8 | 281,04 | 625 | 92,22 | 168,82 |
| 449 | 91,29 | 220,69 | 508 | 4881,51 | 210,22 | 567 | 6126,15 | 6394,03 | 626 | 338,07 | 497,04 |
| 450 | 690,87 | 378,52 | 509 | 386,33 | 241,64 | 568 | 564,34 | 127,81 | 627 | 1743,21 | 858,44 |
| 451 | 372,26 | 469,99 | 510 | 389,85 | 197,63 | 569 | 895,57 | 671,47 | 628 | 1934,06 | 274,04 |
| 452 | 695,43 | 437,27 | 511 | 1044,25 | 1860,09 | 570 | 234,83 | 74,14 | 629 | 3254,92 | 22,36 |
| 453 | 197,46 | 344,87 | 512 | 911,8 | 213,78 | 571 | 918,01 | 306,21 | 630 | 173,04 | 158,48 |
| 454 | 636,99 | 851,23 | 513 | 165,39 | 235,62 | 572 | 490,15 | 215,39 | 631 | 375,54 | 200,28 |
| 455 | 109,04 | 132,39 | 514 | 366,71 | 78,89 | 573 | 340,36 | 561,18 | 632 | 317,73 | 93 |
| 456 | 114,34 | 159,23 | 515 | 3952,23 | 943,15 | 574 | 293,37 | 165,07 | 633 | 2739,3 | 1120,66 |
| 457 | 247,87 | 278,24 | 516 | 394,1 | 154,31 | 575 | 1457,87 | 1267 | 634 | 563,71 | 786,49 |
| 458 | 1565,61 | 2420,43 | 517 | 721,07 | 344,83 | 576 | 186,86 | 193,04 | 635 | 1199,43 | 277,21 |
| 459 | 2149,51 | 2722,27 | 518 | 866,53 | 120,51 | 577 | 15200,82 | 840,95 | 636 | 744,12 | 919,2 |
| 460 | 1761,85 | 1313,47 | 519 | 384,56 | 159,41 | 578 | 445 | 510,51 | 637 | 346,57 | 197,87 |
| 461 | 458,34 | 487,39 | 520 | 1491,26 | 589,3 | 579 | 503,8 | 219,86 | 638 | 1090,93 | 283,03 |
| 462 | 1518,75 | 1405,34 | 521 | 969,57 | 410,04 | 580 | 3363,04 | 542,25 | 639 | 290,46 | 140,43 |
| 463 | 339,47 | 310,42 | 522 | 1522,66 | 1334,42 | 581 | 399,54 | 283,96 | 640 | 111,85 | 66,25 |
| 464 | 9070,76 | 1838,11 | 523 | 145,86 | 141,48 | 582 | 1073,6 | 416,48 | 641 | 455,21 | 420,21 |
| 465 | 3861,14 | 2831,77 | 524 | 189,68 | 287,18 | 583 | 344,17 | 359,53 | 642 | 345,83 | 199,62 |
| 466 | 423,42 | 203,85 | 525 | 1514,05 | 111,26 | 584 | 679,11 | 618,79 | 643 | 1651,16 | 1011,11 |
| 467 | 283,34 | 170,89 | 526 | 270,67 | 55,86 | 585 | 657,73 | 217,1 | 644 | 4741,83 | 614,65 |
| 468 | 367,72 | 315,21 | 527 | 223,41 | 313,99 | 586 | 3920,85 | 58,99 | 645 | 697,29 | 334,7 |
| 469 | 1424,84 | 339,86 | 528 | 220,43 | 162,9 | 587 | 192,77 | 218,19 | 646 | 277,24 | 288,32 |
| 470 | 1243,61 | 366,74 | 529 | 775,4 | 654,44 | 588 | 417,28 | 103,5 | 647 | 4690,58 | 3358,31 |
| 471 | 519,88 | 332,05 | 530 | 115,96 | 136,71 | 589 | 331,42 | 405,5 | 648 | 240,79 | 294,91 |
| 472 | 457,11 | 528,19 | 531 | 2682,19 | 2753,18 | 590 | 2854 | 126,77 | 649 | 641,99 | 200,78 |
| 473 | 1906,66 | 2592,95 | 532 | 1004,59 | 725,68 | 591 | 809,25 | 1010,64 | 650 | 371,35 | 238,75 |
| 474 | 840,95 | 1804,57 | 533 | 24215,6 | 23635,89 | 592 | 262,72 | 539,84 | 651 | 2349,83 | 1291,14 |
| 475 | 285,04 | 113,11 | 534 | 1380,23 | 1722,54 | 593 | 146,88 | 411,88 | 652 | 193,38 | 143,66 |
| 476 | 232,38 | 117,03 | 535 | 635,5 | 466,98 | 594 | 8079,35 | 1105,07 | 653 | 1226,05 | 1935,03 |
| 477 | 4444,41 | 11160,44 | 536 | 590,85 | 530,1 | 595 | 488,35 | 132,06 | 654 | 5138,39 | 3725,55 |
| 478 | 698,61 | 390,57 | 537 | 205,17 | 252,33 | 596 | 3335 | 859,68 | 655 | 3230,3 | 7444,98 |
| 479 | 648,4 | 507,64 | 538 | 262,07 | 133,39 | 597 | 516,5 | 679,53 | 656 | 746,95 | 386,71 |
| 480 | 442,16 | 240,49 | 539 | 236,94 | 716,14 | 598 | 1181,54 | 168,71 | 657 | 239,61 | 201,28 |
| 658 | 464,77 | 869,33 | 717 | 869,93 | 987,76 | 776 | 5071,3 | 7395,44 | 835 | 900,1 | 189,35 |
| 659 | 210,32 | 980,59 | 718 | 35304,93 | 419,08 | 777 | 271,15 | 292,43 | 836 | 4098,86 | 2173,05 |
| 660 | 3851,92 | 329,08 | 719 | 295,28 | 457,92 | 778 | 502,9 | 356,61 | | | |
| 661 | 2485,76 | 2716,97 | 720 | 97,18 | 91,86 | 779 | 4550,74 | 733,37 | | | |
| 662 | 347,83 | 623,37 | 721 | 5238,18 | 742,94 | 780 | 811,84 | 153,69 | | | |
| 663 | 145,28 | 189,73 | 722 | 1105,15 | 2446,05 | 781 | 5268,58 | 6177,03 | | | |
| 664 | 479,37 | 1413,92 | 723 | 15994,58 | 18395,94 | 782 | 964,7 | 441,9 | | | |
| 665 | 690,96 | 407,33 | 724 | 25574,15 | 56,87 | 783 | 1288,41 | 1241,77 | | | |
| 666 | 440,32 | 770,9 | 725 | 223,22 | 334,29 | 784 | 761,9 | 106,33 | | | |
| 667 | 348,99 | 416,59 | 726 | 390,33 | 591,38 | 785 | 302,57 | 220,29 | | | |
| 668 | 321,33 | 612,28 | 727 | 874,08 | 1016,73 | 786 | 441,39 | 34,69 | | | |
| 669 | 330,69 | 333,64 | 728 | 129,08 | 109,04 | 787 | 4805,19 | 408,09 | | | |
| 670 | 465,29 | 768,38 | 729 | 287,17 | 386,91 | 788 | 724,85 | 750,37 | | | |
| 671 | 804,23 | 897,03 | 730 | 938,84 | 1689,36 | 789 | 2797,11 | 4381,9 | | | |
| 672 | 331,26 | 117,39 | 731 | 249,13 | 373,29 | 790 | 1660,28 | 627,22 | | | |
| 673 | 18864,79 | 34,43 | 732 | 2681,59 | 934,95 | 791 | 82,16 | 51,86 | | | |
| 674 | 222,74 | 256,29 | 733 | 518,6 | 570,92 | 792 | 1500,15 | 777,08 | | | |
| 675 | 777,58 | 927,95 | 734 | 304,49 | 410,25 | 793 | 131,83 | 184,79 | | | |
| 676 | 218,75 | 161,04 | 735 | 515,18 | 321,95 | 794 | 1950,66 | 1314,25 | | | |
| 677 | 675,28 | 307,55 | 736 | 719,9 | 389,6 | 795 | 445,08 | 140,52 | | | |
| 678 | 1427,9 | 1826,69 | 737 | 162,63 | 213,43 | 796 | 421,99 | 182,5 | | | |
| 679 | 1500,77 | 1728,96 | 738 | 202,87 | 327,64 | 797 | 426,27 | 390,57 | | | |
| 680 | 1179,77 | 1493,41 | 739 | 310,7 | 366,38 | 798 | 498,29 | 564,91 | | | |
| 681 | 1035,25 | 621,15 | 740 | 1057,06 | 967,69 | 799 | 483,58 | 288,48 | | | |
| 682 | 827,45 | 539,76 | 741 | 1305,72 | 1525,5 | 800 | 177,85 | 148,57 | | | |
| 683 | 928,3 | 504,81 | 742 | 406,27 | 251,43 | 801 | 1927,19 | 290,82 | | | |
| 684 | 379,72 | 200,01 | 743 | 209,98 | 332,04 | 802 | 1908,71 | 378,98 | | | |
| 685 | 454,16 | 274,58 | 744 | 779,02 | 507,03 | 803 | 1356,99 | 309,29 | | | |
| 686 | 1247,87 | 797,98 | 745 | 509,21 | 780,22 | 804 | 590,4 | 454,65 | | | |
| 687 | 21125,88 | 178,01 | 746 | 550,52 | 205,81 | 805 | 479,33 | 263,09 | | | |
| 688 | 131 | 156,14 | 747 | 303,67 | 178,71 | 806 | 1688,17 | 334,94 | | | |
| 689 | 86,47 | 120,23 | 748 | 391,6 | 345,85 | 807 | 2524,15 | 1193,98 | | | |
| 690 | 184,62 | 196,27 | 749 | 161,04 | 486,26 | 808 | 613,82 | 295,85 | | | |
| 691 | 326,21 | 74,96 | 750 | 1633,21 | 1883,97 | 809 | 1847,5 | 1489 | | | |
| 692 | 467,89 | 307,96 | 751 | 1948,88 | 819,34 | 810 | 551,98 | 311,34 | | | |
| 693 | 796,96 | 485,58 | 752 | 947,01 | 1410,71 | 811 | 908,48 | 388,74 | | | |
| 694 | 2248,11 | 1305,99 | 753 | 88,29 | 103,94 | 812 | 124,16 | 66,69 | | | |
| 695 | 492,82 | 227,88 | 754 | 748,98 | 398,24 | 813 | 1567,12 | 859,71 | | | |
| 696 | 443,67 | 725,02 | 755 | 5654,78 | 2358,14 | 814 | 537,13 | 331,57 | | | |
| 697 | 810,12 | 535,52 | 756 | 176,88 | 284,44 | 815 | 1081,92 | 743,86 | | | |
| 698 | 998,77 | 1170,01 | 757 | 641,35 | 134,74 | 816 | 1288,32 | 753,52 | | | |
| 699 | 192,96 | 140,39 | 758 | 193,83 | 250,67 | 817 | 1522,33 | 1216,08 | | | |
| 700 | 115 | 132,51 | 759 | 265,87 | 443,61 | 818 | 3743,12 | 844,55 | | | |
| 701 | 2241,77 | 2710,35 | 760 | 1611,2 | 2134,8 | 819 | 211,47 | 155,57 | | | |
| 702 | 770,19 | 988,21 | 761 | 895 | 953,08 | 820 | 232,71 | 324,57 | | | |
| 703 | 401,99 | 247,99 | 762 | 4307,33 | 1318,11 | 821 | 311,5 | 454,92 | | | |
| 704 | 360,05 | 552,79 | 763 | 425,51 | 320,2 | 822 | 433,81 | 689,88 | | | |
| 705 | 107,75 | 98,14 | 764 | 157,27 | 248,54 | 823 | 2674,9 | 1470,8 | | | |
| 706 | 998,83 | 1032,67 | 765 | 2159,53 | 4308,89 | 824 | 2022,24 | 1507,34 | | | |
| 707 | 164,07 | 209,94 | 766 | 601,24 | 540,73 | 825 | 4346,69 | 2618,94 | | | |
| 708 | 1627,4 | 1118,73 | 767 | 259,57 | 190,8 | 826 | 2344,92 | 1552,78 | | | |
| 709 | 763,49 | 250,07 | 768 | 268,04 | 788,11 | 827 | 898,51 | 575,57 | | | |
| 710 | 773,85 | 675,53 | 769 | 1124,91 | 947,92 | 828 | 901,65 | 505,99 | | | |
| 711 | 155,22 | 286,79 | 770 | 345,81 | 258,56 | 829 | 1946,95 | 9898,6 | | | |
| 712 | 1272,34 | 2512,12 | 771 | 4076,84 | 585,19 | 830 | 961,96 | 4337,06 | | | |
| 713 | 27791,32 | 1191,08 | 772 | 1545,75 | 627,71 | 831 | 1561,13 | 2095,65 | | | |
| 714 | 459,25 | 281,17 | 773 | 635,06 | 927,49 | 832 | 5309,3 | 1583,61 | | | |
| 715 | 941,28 | 1159 | 774 | 1015,92 | 550,41 | 833 | 532,67 | 313,23 | | | |
| 716 | 341,85 | 213,77 | 775 | 475,86 | 490,03 | 834 | 855,38 | 600,48 | | | |

5. Verfahren nach mindestens einem der Ansprüche 1 bis 4, wobei mindestens zehn oder 20 oder 100 oder alle Polypeptidmarker verwendet werden, wie sie in Anspruch 1 definiert sind.

6. Verfahren nach mindestens einem der Ansprüche 1 bis 5, wobei die Probe eines Individuums eine Urinprobe oder eine Blutprobe (Serum- oder Plasmaprobe) ist.

7. Verfahren nach mindestens einem der Ansprüche 1 bis 6, wobei Kapillarelektrophorese, HPLC, Gasphasenionenspektrometrie und/oder Massenspektrometrie zum Nachweis der An- oder Abwesenheit oder Amplitude des/der Polypeptidmarker verwendet wird.

8. Verfahren nach mindestens einem der Ansprüche 1 bis 7, wobei vor der Messung der Molekularmasse der Polypeptidmarker eine Kapillarelektrophorese durchgeführt wird, vorzugsweise wobei Massenspektrometrie zum Nachweis der An- oder Abwesenheit oder Amplitude des/der Polypeptidmarker verwendet wird.

9. Verfahren nach mindestens einem der Ansprüche 1 bis 9, wobei das Verfahren zur Diagnose von Blasenkrebs verwendet wird, oder wobei das Verfahren zur Bestimmung eines Tumorstadiums von Blasenkrebs verwendet wird, oder wobei das Verfahren zur gleichzeitigen Diagnose von Blasenkrebs und zur Bestimmung eines Tumorstadiums von Blasenkrebs verwendet wird.

10. Verwendung mindestens eines Polypeptidmarkers ausgewählt aus den Markern Nr. 1 bis 836, der durch die Werte gemäß Anspruch 1 für die Molekularmassen und die Migrationszeit charakterisiert ist zur Diagnose und/oder Bestimmung eines Tumorstadiums von Blasenkrebs.

11. Verfahren zur Diagnose von Blasenkrebs (BC) umfassend die Schritte
a) der Auftrennung einer Probe in mindestens drei, bevorzugt 10 Teilproben
b) Analyse von mindestens zwei Teilproben zur Bestimmung einer An- oder Abwesenheit oder Amplitude mindestens eines Polypeptidmarkers in der Probe, wobei der Polypeptidmarker ausgewählt ist aus den Markern 1 bis 836, die durch die Molekularmassen und Migrationszeit (CE-Zeit) gemäß Anspruch 1 charakterisiert sind, wobei bevorzugt mindestens 10 Teilproben gemessen werden.

12. Verfahren zur Bestimmung eines Tumorstadiums von Blasenkrebs umfassend die Schritte
a) der Auftrennung einer Probe in mindestens 3, bevorzugt 10 Teilproben
b) Analyse von mindestens zwei Teilproben zur Bestimmung einer An- oder Abwesenheit oder Amplitude mindestens eines Polypeptidmarkers in der Probe, wobei der Polypeptidmarker ausgewählt ist aus den Markern 1 836, die durch die Molekularmassen und Migrationszeit (CE-Zeit) gemäß Anspruch 1 charakterisiert sind, wobei bevorzugt mindestens 10 Teilproben gemessen werden.

13. Verfahren nach mindestens einem der Ansprüche 1 bis 12, **dadurch gekennzeichnet dass** die CE-Zeit bezogen ist auf eine 90 cm lange Glaskapillare mit einem inneren Durchmesser (ID) von 50 µm bei einer angelegten Spannung von 25 kV, wobei als Laufmittel 20% Acetonitril, 0,25 M Ameisensäure in Wasser verwendet wird.

14. Markerkombination, umfassend mindestens 10 Marker ausgewählt aus den Markern 1 bis 836 die durch die Molekularmassen und Migrationszeit (CE-Zeit) gemäß Anspruch 1 charakterisiert sind.

15. Polypeptid mit der Sequen: REVQSKIGYGRQIIS.


SEQUENCE LISTING

<110> mosaiques diagnostics and therapeutics AG

<120> Polypeptidmarker zur Diagnose von Blasenkrebs

<130> 061851wo

<140> PCT/EP2006/06574214:40 09.01.2007
<141> 2006-08-28

<150> EP05107840.0
<151> 2005-08-26

<160> 4

<170> PatentIn version 3.3

<210> 1
<211> 15
<212> PRT
<213> Artificial

<220>
<223> Synthetisches Peptid

<400> 1

Arg Glu Val Gln Ser Lys Ile Gly Tyr Gly Arg Gln Ile Ile Ser
1               5                   10                  15

<210>  2
<211>  24
<212>  PRT
<213>  Artificial

<220>
<223>  Synthetisches Peptid

<400>  2

Glu Leu Met Thr Gly Glu Leu Pro Tyr Ser His Ile Asn Asn Arg Asp
1               5                   10                  15

Gln Ile Ile Phe Met Val Gly Arg
            20

<210>  3
<211>  21
<212>  PRT
<213>  Artificial

<220>
<223>  Synthetisches Peptid

<400>  3

Thr Gly Ser Leu Pro Tyr Ser His Ile Gly Ser Arg Asp Gln Ile Ile
1               5                   10                  15

Phe Met Val Gly Arg
            20

<210> 4
<211> 18
<212> PRT
<213> Artificial

<220>
<223> Synthetisches Peptid

<400> 4

Gly Ile Val Leu Tyr Glu Leu Met Thr Gly Glu Leu Pro Tyr Ser His
1               5                   10                  15

Ile Asn